# EUROPÄISCHE PATENTSCHRIFT

(11) **EP 1 603 923 B1**
(45) Veröffentlichungstag und Bekanntmachung des Hinweises auf die Patenterteilung: **30.05.2007**
(21) Anmeldenummer: 04718627.5
(22) Anmeldetag: 09.03.2004
(51) Int. Cl.: C07F 1/12, C07F 5/00, C07F 15/00

(54) **METALLKOMPLEXE**
METAL COMPLEXES
COMPLEXES METALLIQUES

(30) Priorität: 11.03.2003 DE 10310887
(43) Veröffentlichungstag der Anmeldung: 14.12.2005
(73) Patentinhaber: Merck Patent GmbH, 64293 Darmstadt (DE)
(72) Erfinder: STÖSSEL, Philipp, 60487 Frankfurt am Main (DE); SPREITZER, Hubert, 68519 Viernheim (DE)
(86) Internationale Anmeldenummer: PCT/EP2004/002393
(87) Internationale Veröffentlichungsnummer: WO 2004/081017

(56) Entgegenhaltungen:
- DE-A- 10 116 962
- BODAR-HOUILLON F ET AL: "Synthesis and Luminescence Properties of a New Tripode Containing 2,2 ?-Bipyrazine Subunits: The tris-[6-methyl-2,2 ?-bipyrazine-2-yl)m ethyl]amine" TETRAHEDRON LETTERS, ELSEVIER SCIENCE PUBLISHERS, AMSTERDAM, NL, Bd. 36, Nr. 6, 6. Februar 1995 (1995-02-06), Seiten 865-868, XP004028691 ISSN: 0040-4039
- XXth International Conference on Organometallic Chemistry; Corfu, July 7-12, 2002, Oral Presentations; O109; C. Slugovc and E. Cremona "Facile Triple C-H Activation Reactions with Iridium- TpPh Complexes" XP002284929
- SLUGOVC C. ET AL: "Generation of Heteroatom-Substituted Carbene Complexes of Iridium by Double C-H Activation of Ether and Amine Substrates" ANGEW. CHEM. INT. ED. ENGL., Bd. 39, Nr. 12, 2000, Seiten 2158-2160, XP002284927
- SLUGOVC, C. ET AL.: "Investigation of the C-H Activation Potential of..." HELV. CHIM. ACTA, Bd. 84, 2001, Seiten 2868-2883, XP002284928
- HAN R ET AL: "THE TRISU3-(9-ANTHRYL)PYRAZOL-1-YLHYDROBORATO LIGAND, UTPANT: COMPOSITIONAL DISORDER BETWEEN A VACANCY AND A CHAIN OF THREE ATOMS" POLYHEDRON, PERGAMON PRESS, OXFORD, GB, Bd. 14, Nr. 3, 1995, Seiten 387-391, XP000617745 ISSN: 0277-5387
- RHEINGOLD A L ET AL: "HYDROTRIS(3-MESITYLPYRAZOL-1-YL)BORATE AND HYDROBIS(3-MESITYLPYRAZOL- 1-YL)(5-MESITYLPYRAZOL-1-YL)BORATE: SYMMETRIC AND ASYMMETRIC LIGANDS WITH ROTATIONALLY RESTRICTED ARYL SUBSTITUENTS" INORGANIC CHEMISTRY, AMERICAN CHEMICAL SOCIETY, EASTON, US, Bd. 32, Nr. 16, 4. August 1993 (1993-08-04), Seiten 3471-3477, XP000617663 ISSN: 0020-1669
- CLARAMUNT R M ET AL: "A multinuclear NMR study in the solid state and in solution of thallium(I) tris-(pyrazol-1-yl)borates (thallium scorpionates)" JOURNAL OF ORGANOMETALLIC CHEMISTRY, ELSEVIER-SEQUOIA S.A. LAUSANNE, CH, Bd. 689, Nr. 2, 15. Januar 2004 (2004-01-15), Seiten 463-470, XP004484032 ISSN: 0022-328X
- LOPEZ C ET AL: "An <1>H and <13>C NMR spectroscopic study of the structure of potassium and thallium salts of tris- and tetrakis-(pyrazol-1-yl) borates in solution. Some <13>C- <11>B and <13>C- <205>T1 residual coupling constants" JOURNAL OF ORGANOMETALLIC CHEMISTRY, ELSEVIER-SEQUOIA S.A. LAUSANNE, CH, Bd. 503, Nr. 2, 15. November 1995 (1995-11-15), Seiten 265-276, XP004023635 ISSN: 0022-328X
- PIGUET C ET AL: "Tridentate binding units as structural patterns for the design of nine-coordinate lanthanide building blocks with predetermined properties" JOURNAL OF ALLOYS AND COMPOUNDS, ELSEVIER SEQUOIA, LAUSANNE, CH, Bd. 303-304, Mai 2000 (2000-05), Seiten 94-103, XP004204340 ISSN: 0925-8388
- BRINGMANN G ET AL: "The directed synthesis of axially chiral ligands, reagents, catalysts, and natural products through the 'lactone methodology'" JOURNAL OF ORGANOMETALLIC CHEMISTRY, ELSEVIER-SEQUOIA S.A. LAUSANNE, CH, Bd. 661, Nr. 1-2, 1. November 2002 (2002-11-01), Seiten 49-65, XP004390945 ISSN: 0022-328X
- WOODGATE P D ET AL: "Synthesis of dioxazaborocines from N,N'-alkylbridged-bis(bis(2-hydrox ybenzyl)aminomethyl)amines" JOURNAL OF ORGANOMETALLIC CHEMISTRY, ELSEVIER-SEQUOIA S.A. LAUSANNE, CH, Bd. 595, Nr. 2, Februar 2000 (2000-02), Seiten 215-223, XP004187391 ISSN: 0022-328X
- ULRICH G ET AL: "Phloroglucinol based podands, versatile tripodal ligands" TETRAHEDRON LETTERS, ELSEVIER SCIENCE PUBLISHERS, AMSTERDAM, NL, Bd. 43, Nr. 49, 2. Dezember 2002 (2002-12-02), Seiten 8835-8837, XP004391846 ISSN: 0040-4039
- GADE L H: "Transition metal complexes with polydentate amido ligands: novel structural building blocks and chemical reagents" JOURNAL OF ORGANOMETALLIC CHEMISTRY, ELSEVIER-SEQUOIA S.A. LAUSANNE, CH, Bd. 661, Nr. 1-2, 1. November 2002 (2002-11-01), Seiten 85-94, XP004390947 ISSN: 0022-328X
- REEVES ZOE R ET AL: "Lanthanide complexes of a new sterically hindered potentially hexadentate podand ligand based on a tris(pyrazolyl)borate core;crystal structures, solution structures and luminescence properties" JOURNAL OF THE CHEMICAL SOCIETY, DALTON TRANSACTIONS, CHEMICAL SOCIETY. LETCHWORTH, GB, Nr. 3, 1999, Seiten 349-356, XP002167995 ISSN: 1472-7773
- ARMAROLI N. ET AL.: "Structure and Photophysical Properties of ...." INORG. CHEM., Bd. 38, 11. Dezember 1999 (1999-12-11), Seiten 5769-5776, XP001182118
- CARIS C ET AL: "Synthesis and NMR Study of Two Lipophilic Iron(III) Sequestering Agents Based on 8-Hydroxyquinoline; H-bonding and Conformational Changes" TETRAHEDRON, ELSEVIER SCIENCE PUBLISHERS, AMSTERDAM, NL, Bd. 52, Nr. 13, 1. März 1996 (1996-03-01), Seiten 4659-4672, XP004104286 ISSN: 0040-4020
- ARMAROLI ET AL: "Luniniscence Properties" CHEM. PHYS. LETT, Bd. 276, Nr. 5-6, 26. September 1997 (1997-09-26), Seiten 435-440, XP001182074

## Beschreibung

Metallorganische Verbindungen - speziell Verbindungen der d⁸-Metalle - werden in naher Zukunft als Wirkkomponenten (= Funktionsmaterialien) in einer Reihe von verschiedenartigen Anwendungen, die im weitesten Sinne der Elektronikindustrie zugerechnet werden können, Einsatz als funktionelle Komponenten finden.
Bei den auf organischen Komponenten basierenden Organischen-Elektrolumineszenz-Vorrichtungen (allg. Beschreibung des Aufbaus vgl.
US-A-4,539,507 und US-A-5,151,629) bzw. deren Einzelbauteilen, den Organischen-Lichtemittierenden-Dioden (OLEDs) ist die Markteinführung bereits erfolgt, wie die erhältlichen Auto-Radios mit "Organischem Display" der Firma Pioneer belegen. Weitere derartige Produkte stehen kurz vor der Einführung. Trotz allem sind hier noch deutliche Verbesserungen nötig, um diese Displays zu einer echten Konkurrenz zu den derzeit marktbeherrschenden Flüssigkristallanzeigen (LCD) zu machen bzw. diese zu überflügeln.

Eine Entwicklung hierzu, ist die Verbesserung von Elektronentransportmaterialien und blauen Singulettemittern auf Basis von Metall-Chelatkomplexen, wobei hier insbesondere Aluminium- und Lanthan-Chelat-Komplexe von Interesse sind.
Eine weitere Entwicklung, die sich in den letzten Jahren abzeichnet, ist der Einsatz von metallorganischen Komplexen, die Phosphoreszenz statt Fluoreszenz zeigen [M. A. Baldo, S. Lamansky, P. E. Burrows, M. E. Thompson, S. R. Forrest, Applied Physics Letters, **1999**, *75*, 4-6].
Aus theoretischen Spin-statistischen Gründen ist unter Verwendung metallorganischer Verbindungen als Phosphoreszenz-Emitter eine bis zu vierfache Energie- und Leistungseffizienz möglich. Ob sich diese neue Entwicklung durchsetzen wird, hängt stark davon ab, ob entsprechende Device-Kompositionen gefunden werden können, die diese Vorteile (Triplett-Emission = Phosphoreszenz gegenüber Singulett-Emission = Fluoreszenz) auch in den OLEDs umsetzen können. Als wesentliche Bedingungen für die praktische Anwendung sind hier insbesondere eine hohe operative Lebensdauer, eine hohe Stabilität gegenüber Temperaturbelastung und eine niedrige Einsatz- und Betriebsspannung, um mobile Applikationen zu ermöglichen, zu nennen.

In beiden Fällen muß der effiziente chemische Zugang zu den entsprechenden Chelat-Komplexen bzw. Organo-Metall-Verbindungen gegeben sein. Von besonderem Interesse ist dies jedoch, vor dem Hintergrund der Seltenheit der Metalle, bei Ruthenium-, Osmium-, Rhodium-, Iridium- und Gold-Verbindungen.

In der Literatur wurden bis jetzt zwei grundlegende Bautypen von OLEDs, die Fluoreszenz- bzw. Phosphoreszenzemitter als farbgebende Komponenten aufweisen, beschrieben:
Typ 1 hat üblicherweise den folgenden Schicht-Aufbau [am Bsp. einer OLED mit Phosphozeszenzemitter: M. E. Thompson et al., Proceedings of SPIE, 31.07 - 02.08.2000, San Diego, USA, Volume 4105, Seite 119 - 124]:
   1. Trägerplatte = Substrat (üblicherweise Glas oder Kunststoffolien).
   2. Transparente Anode (üblicherweise Indium-Zinn-Oxid, ITO).
   3. Lochtransport-Schicht (**H**ole-**T**ransport-**L**ayer; **HTL**): üblicherweise auf Basis von Triarylamin-Derivaten.
   4. Emissions-Schicht (**E**mitter-**L**ayer; **EL**): diese Schicht besteht entweder aus einem Fluoreszenzemitter bzw. Phosphoreszenzemitter oder einem Matrixmaterial, das mit dem Fluoreszenzemitter bzw. Phosphoreszenzemitter dotiert ist.
   5. Elektronentransport-Schicht (**E**lectron-**T**ransport-**L**ayer; **ETL**): meistens auf Basis von Tris(8-hydroxychinolinato)aluminium(III) (AIQ₃).
   6. Kathode: hier werden in der Regel Metalle, Metallkombinationen oder Metallegierungen mit niedriger Austrittsfunktion verwendet, so z. B. Al-Li.
Typ 2 hat üblicherweise den folgenden Schicht-Aufbau [am Bsp. einer OLED mit Phosphozeszenzemitter: T. Tsutsui et al. Jpn. J. Appl. Phys., **1999**, *38*, L 1502 - L 1504]:
   1. Trägerplatte = Substrat (üblicherweise Glas oder Kunststoffolien).
   2. Transparente Anode (üblicherweise Indium-Zinn-Oxid, ITO).
   3. Lochtransport-Schicht (Hole-Transport-Layer; **HTL**): üblicherweise auf Basis von Triarylamin-Derivaten.
   4. Matrix- und Emissions-Schicht (Emitter-Layer; **EL**): diese Schicht besteht aus einem Matrixmaterial, z.B. auf Basis von Triarylamin-Derivaten, das mit dem Fluoreszenzemitter bzw. Phosphoreszenzemitter dotiert ist.
   5. Elektronentransport-/Lochblockier-Schicht (Hole-Blocking-Layer, **HBL**): üblicherweise auf Basis von Stickstoff-Heterocyclen oder auf Basis von Metallkomplexen, wie z.B. Bis(2-methyl-8-hydroxychinolinato)-(4-phenyl-phenolato)aluminium(III) (B-AlQ₃).
   6. Elektronentransport-Schicht (**E**lektron-**T**ransport-**L**ayer; **ETL**): meistensauf Basis von Tris(8-hydroxychinolinato)aluminium(III) (AlQ₃).
   7. Kathode: hier werden in der Regel Metalle, Metallkombinationen oder Metallegierungen mit niedriger Austrittsfunktion verwendet, so z. B. Al.

Es ist auch möglich, das Licht aus einer dünnen transparenten Kathode auszukoppeln. Diese Vorrichtungen werden entsprechend (je nach Anwendung) strukturiert, kontaktiert und schließlich hermetisch eingesiegelt, da sich i. d. R. die Lebensdauer derartiger Vorrichtungen bei Anwesenheit von Wasser und/oder Luft drastisch verringert.

Die Kenndaten der oben beschriebenen OLEDs zeigen unter anderem folgende Schwachpunkte auf:
1. Die operative Lebensdauer ist in den meisten Fällen noch deutlich zu gering, was einer Einführung von OLEDs im Markt entgegen steht.
2. 2. Aus den Effizienz - Helligkeits - Kurven geht hervor, daß die Effizienz mit steigender Helligkeit häufig stark abnimmt. Dies bedeutet, daß die in der Praxis notwendigen großen Helligkeiten nur über eine hohe Leistungsaufnahme erreicht werden können. Große Leistungsaufnahmen setzen aber große Batterieleistungen portabler Geräte (Mobile-Phones, Lap-Tops etc.) voraus. Außerdem kann die große Leistungsaufnahme, die zum großen Teil in Wärme umgesetzt wird, zur thermischen Schädigung des Displays führen.

Bei der oben erläuterten OLED-Vorrichtung wurden bzw. werden die oben genannten Funktionsmaterialien intensiv optimiert.
Seit einiger Zeit werden (pseudo)oktaedrische Metallkomplexe im weitesten Sinne als ETL (z.B. AIQ₃, s.: C. W. Tang et al., Applied Phys. Lett. **1987**, *51*(12), 913), HBL (z.B. B-AlQ₃, s.: R. Kwong et al., Applied Physics Letters **2002**, *81*(1), 162), als Matrixmaterial in der EL (z.B. B-AlQ₃, s.: C. H. Chen et. al., Proceedings of SPIE-The International Society for Optical Engineering **1998**, *3421*(Display Technologies II), 78), als Singulett-Emitter (z.B. AlQ₃ und andere Komplexe, s.: S. Tokito et al., Synthetic Metals **2000**, *111 - 112,* 393) und als Triplett-Emitter (z.B. Ir(PPy)₃, s.: WO 00/70655; z.B.: Ir(TPy)₃ und Ir(BTPy)₃, s.: S. Okada et al., Proceedings of the SID, **2002**, *52.2*, 1360) eingesetzt. Neben den individuellen, für jedes Material spezifischen Schwachpunkten besitzen die bekannten Metallkomplexe generelle Schwachpunkte, die im folgenden kurz aufgezeigt werden:
1. Viele der bekannten Metallkomplexe, insbesondere solche, die Hauptgruppenmetalle wie Aluminium beinhalten, weisen eine zum Teil erhebliche Hydrolyseempfindlichkeit auf, die so weit gehen kann, daß der Metallkomplex schon nach kurzer Exposition an Luft merklich zersetzt wird. Andere dagegen, wie zum Beispiel das als Elektronentransportmaterial verwendete AlQ₃, neigen zur Anlagerung von Wasser.
   Die starke Hygroskopie dieser und ähnlicher Aluminiumkomplexe ist ein entscheidender praktischer Nachteil. AlQ₃, welches unter normalen Bedingungen synthetisiert und aufbewahrt wird, enthält neben den Hydroxychinolin-Liganden immer noch ein Molekül Wasser pro Komplex-Molekül [vgl. z. B.: H. Schmidbaur et al., Z. Naturforsch. **1991**, *46b*, 901-911]. Dieses ist extrem schwer zu entfernen. Für die Verwendung in OLEDs muß AlQ₃ deshalb in komplizierten, mehrstufigen Sublimations-Verfahren aufwendig gereinigt und im Anschluß daran unter Wasserausschluß in einer Schutzgasatmosphäre gelagert und gehandhabt werden. Weiterhin wurden große Schwankungen in der Qualität einzelner AlQ₃-Chargen, sowie eine schlechte Lagerstabilität festgestellt (s.: S. Karg, E-MRS Konferenz 30.5.00-2.6.00 Straßburg).
2. Viele der bekannten Metallkomplexe besitzen eine geringe thermische Stabilität. Diese führt bei einer Vakuumdeposition der Metallkomplexe zwangsläufig immer zur Freisetzung organischer Pyrolyseprodukte, die zum Teil schon in geringen Mengen die operative Lebensdauer der OLEDs erheblich verringern.
3. Nahezu alle in der Literatur aufgeführten Metallkomplexe, die in OLEDs bisher Verwendung fanden, sind homoleptische, (pseudo)oktaedrische Komplexe bestehend aus einem Zentralmetall koordiniert an drei zweizähnige Liganden. Komplexe diesen Bautyps können in zwei isomeren Formen, dem *meridionalen-* und dem *facialen-*Isomeren, auftreten. Häufig ist eines der beiden Isomeren thermodynamisch nur etwas bevorzugt. Dies führt dazu, daß unter bestimmten Bedingungen - beispielsweise einer bestimmten Sublimationstemperatur - das eine oder das andere Isomere oder gar Gemische beider anfallen. Dies ist nicht erwünscht, da sich die beiden Isomeren oft deutlich in ihren physikalischen Eigenschaften (Emissionsspektrum, Elektronen- und Lochleitungseigenschaften, etc.) unterscheiden und somit die Eigenschaften einer OLED schon bei geringen Änderungen des Herstellungsprozesses deutlich voneinander abweichen können. Ein Beispiel hierfür sind die deutlich verschiedenen Eigenschaften von *mer*-AlQ₃ und *fac*-AlQ₃, die grüne bzw. blaue Photolumineszenz zeigen (s.: M. Coelle, Chemical Communications, **2002**, *23*, 2908-2909).

Es bestand daher ein Bedarf an alternativen Verbindungen, die die oben genannten Schwachpunkte nicht aufweisen, den bekannten Metallkomplexen jedoch an Effizienz und Emissionsfarbe um nichts nachstehen.

Es wurde nun überraschend gefunden, daß Metallkomplexe polypodaler Liganden hervorragende Eigenschaften bei der Verwendung als ETL, als HBL, als Matrixmaterial in der EL, als Singulett-Emitter und auch als Triplett-Emitter aufzeigen, wobei die jeweilige, konkrete Funktion durch die geeignete Wahl des Metalls und des geeigneten zugehörigen Liganden bestimmt wird.
Diese Verbindungen sind Gegenstand der vorliegenden Erfindung. Die Verbindungen zeichnen sich durch folgende generelle Eigenschaften aus:
1. Die erfindungsgemäßen Verbindungen zeichnen sich - im Gegensatz zu vielen bekannten Metallkomplexen, die der teilweisen oder vollständigen pyrolytischen Zersetzung bei Sublimation unterliegen - durch eine große thermische Stabilität aus. Diese Stabilität führt bei Verwendung in entsprechenden Vorrichtungen zu einer deutlichen Erhöhung der operativen Lebensdauer.
2. Die erfindungsgemäßen Verbindungen weisen keine erkennbare Hydrolyse bzw. Hygroskopie auf. Lagerung für mehrere Tage bzw. Wochen unter Zutritt von Luft und Wasserdampf führt zu keinen Veränderungen der Substanzen. Die Anlagerung von Wasser an die Verbindungen konnte nicht nachgewiesen werden. Dies hat den Vorteil, daß die Substanzen unter einfacheren Bedingungen gereinigt, transportiert, gelagert und für den Einsatz vorbereitet werden können.
3. Die erfindungsgemäßen Verbindungen - eingesetzt als ETL-Material in Elektrolumineszenz-Vorrichtungen - führen zu hohen Effizienzen, die insbesondere unabhängig von den verwendeten Stromdichten sind. Damit werden auch bei hohen Stromdichten sehr gute Effizienen ermöglicht.
4. Die erfindungsgemäßen Verbindungen - eingesetzt als HBL-Material in Elektrolumineszenz-Vorrichtungen - führen zu hohen Effizienzen, die insbesondere unabhängig von den verwendeten Stromdichten sind. Damit werden auch bei hohen Stromdichten, d.h. hohen Helligkeiten, sehr gute Effizienen ermöglicht. Außerdem sind die erfindungsgemäßen Materialien stabil gegen Löcher, was z.B. bei anderen Metallkomplexen - z.B. AlQ₃ und analogen Verbindungen - nicht in ausreichendem Maße gegeben ist (s.: z. B. Z. Popovic et al., Proceedings of SPIE, **1999,** *3797*, 310-315).
5. Die erfindungsgemäßen Verbindungen - eingesetzt in Elektrolumineszenz-Vorrichtungen als EL-Material in reiner Form oder als Matrixmaterial in Kombination mit einem Dotanten - führen in diesen zu hohen Effizienzen, wobei sich die Elektrolumineszenz-Vorrichtungen durch steile Strom-Spannungs-Kurven und besonders durch lange operative Lebensdauer auszeichnen.
6. Die erfindungsgemäßen Verbindungen sind gut reproduizerbar in verlässlicher hoher Reinheit herstellbar und weisen keine Chargenschwankung auf.
7. Die erfindungsgemäßen Verbindungen weisen zum Teil eine exzellente Löslichkeit in organischen Lösungsmitteln auf. Damit lassen sich diese Materialien leichter reinigen und sind auch aus Lösung durch Beschichtungs- oder Drucktechniken verarbeitbar. Auch bei der üblichen Verarbeitung durch Verdampfen ist diese Eigenschaft von Vorteil, da so die Reinigung der Anlagen bzw. der eingesetzten Schattenmasken erheblich erleichtert wird.

F. Bodar-Houillon und A. Marsura (*Tetrahedron Lett.* **1995**, *36*, 865-868) beschreiben Europium- und Terbiumkomplexe mit tripodalen Bipyrazinliganden und deren Absorptions- und Emissionseigenschaften.

Z. R. Reeves *et al.* (*J. Chem. Soc*., *Dalton Trans.* **1999**, 349-355) beschreiben Lanthanidkomplexe mit tripodalen Pyridylpyrazolyl-Liganden und deren Lumineszenzeigenschaften.

N. Armaroli *et al.* (*Inorg. Chem.* **1999,** *38*, 5769-5776) beschreiben Lanthanidkomplexe mit tripodalen Pyridylpyrazolyl-Liganden und deren Absorptions- und Lumineszenzeigenschaften.

Die unten genauer ausgeführte Klasse der Chelat-Komplexe bzw. Organo-Metall-Verbindungen polypodaler Liganden und deren Verwendung als Funktionsmaterialien in elektro-optischen Komponenten ist neu und bisher in der Literatur nicht beschrieben worden, ihre effiziente Darstellung und Verfügbarkeit als Reinstoffe ist hierfür aber von großer Bedeutung.

Gegenstand der vorliegenden Erfindung sind somit Metallkomplexe der Struktur 1, 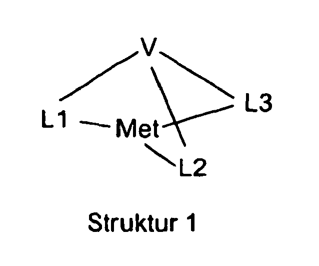 enthaltend mindestens ein Metall Met, koordiniert an einen polypodalen Liganden Lig gemäß Struktur 2, 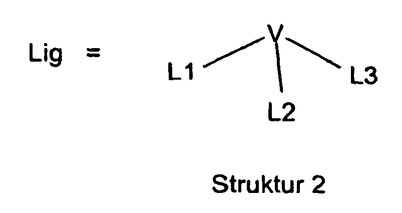 wobei V eine Verbrückungseinheit ist, dadurch gekennzeichnet, daß sie 1 bis 80 Atome enthält und die drei Teilliganden L1, L2 und L3, die gleich oder verschieden bei jedem Auftreten sein können, kovalent miteinander verbindet, und wobei die drei Teilliganden L1, L2 und L3 der Struktur 3 genügen, 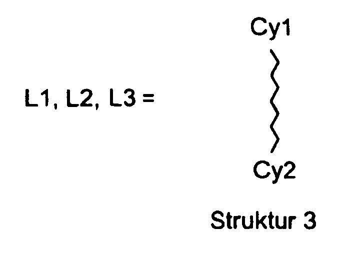 wobei Cy1 und Cy2, gleich oder verschieden bei jedem Auftreten, substituierten oder unsubstituierten, gesättigten, ungesättigten oder aromatischen Homo- oder Heterocyclen bzw. Homo- oder Heteroteilcyclen eines kondensierten Systems entsprechen, die jeweils über ein Ringatom oder über ein exocyclisch an den Homo- oder Heterocyclus gebundenes Atom ionisch, kovalent oder koordinativ an das Metall gebunden ist,
wobei die Verbindungen der Struktur 1 nicht geladen, d. h. nach außen elektrisch neutral, sind.

Die Verbrückungseinheit V weist 1 bis 80 Atome aus der III., IV. und/oder V. Hauptgruppe der Elemente des Periodensystems auf. Diese bilden das Grundgerüst der Verbrückungseinheit.

Das oben gewählte Symbol der Zick-Zack-Linie beschreibt hier nur allgemein die Verknüpfung von Cy1 mit Cy2. Eine nähere Ausführung der möglichen Verknüpfungen der Cyclen wird im folgenden ausgeführt.

Die Homo- oder Heterocyclen Cy1 und Cy2 können über eine Einfachbindung verknüpft sein. Außerdem können die Homo- oder Heteroteilcyclen Cy1 und Cy2, über eine gemeinsame Kante verknüpft sein. Weiterhin können sie, neben der Verknüpfung über eine Einfachbindung oder eine gemeinsame Kante, über Substituenten an den Homo- oder Heterocyclen Cy1 und Cy2 bzw. den Homo- oder Heteroteilcyclen miteinander verküpft sein und so ein polycyclisches, aromatisches oder aliphatisches Ringsystem aufspannen.

Die prinzipellen Verknüpfungsmöglichkeiten seien hier am Beispiel eines Benzolrings (Cy1) und eines Pyridins (Cy2) exemplarisch dargestellt (s.Abb.1), ohne die Vielfalt aller möglichen Verknüpfungen dadurch einschränken zu wollen.

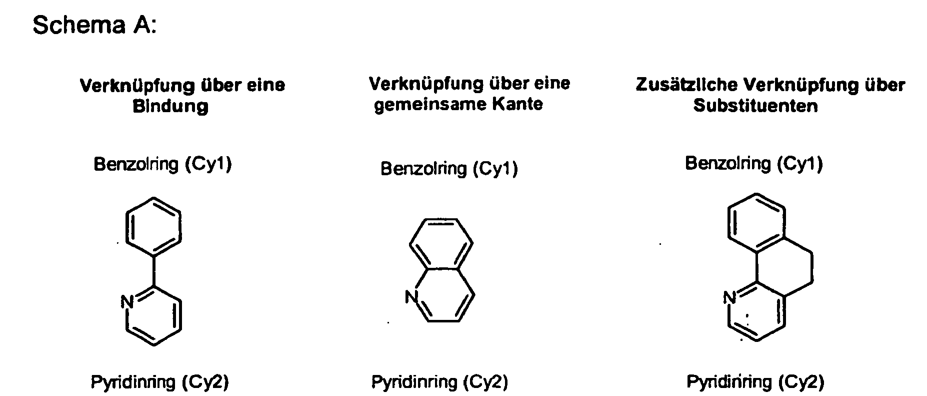

Dabei sind erfindungsgemäße Verbindungen der Struktur 1 dadurch gekennzeichnet, daß diese nicht geladen, d.h. nach außen hin elektrisch neutral, sind.

Bevorzugt sind erfindungsgemäße Verbindungen der Struktur 1, dadurch gekennzeichnet, daß mindestens einer der Teilliganden L1, L2 und L3, bevorzugt mindestens zwei der Teilliganden L1, L2 und L3 und besonders bevorzugt alle drei Teilliganden L1, L2 und L3 einfach negativ geladen sind.

Bevorzugt sind erfindungsgemäße Verbindungen der Struktur 1, dadurch gekennzeichnet, daß L1 = L2 = L3 ist.

Ebenfalls bevorzugt sind erfindungsgemäße Verbindungen der Struktur 1, dadurch gekennzeichnet, daß L1≠ L2 ist.

Weiterhin bevorzugt sind erfindungsgemäße Verbindungen der Struktur 1, dadurch gekennzeichnet, daß Cy1 ungleich Cy2 ist.

Bevorzugt sind erfindungsgemäße Verbindungen der Struktur 1, dadurch gekennzeichnet, daß die Verknüpfungseinheit V als verknüpfendes Atom ein Element der 3., 4. oder 5. Hauptgruppe oder einen 3- bis 6-gliedrigen Homo- oder Heterocyclus enthält.

Bevorzugt sind erfindungsgemäße Verbindungen der Struktur 1, dadurch gekennzeichnet, daß der polypodale Ligand Lig gemäß Struktur 4 eine *faciale* Koordination am Metall Met erzeugt.

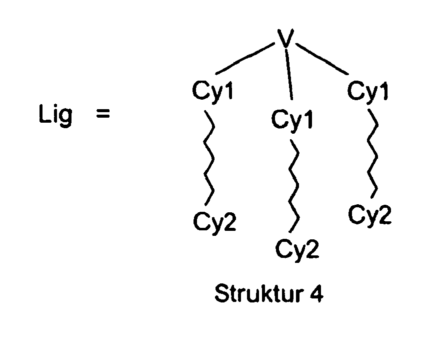

Ebenfalls bevorzugt sind erfindungsgemäße Verbindungen der Struktur 1, dadurch gekennzeichnet, daß der polypodale Ligand Lig gemäß Struktur 5 eine *meridionale* Koordination am Metall Met erzeugt.

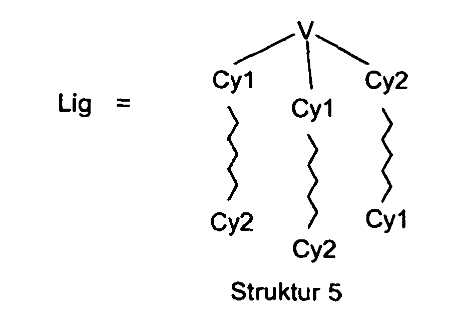

*Faciale* bzw. *meridionale* Koordination im Sinne dieser Anmeldung beschreibt die Umgebung des Metalls Met mit den sechs Donoratomen. Eine *faciale* Koordination liegt dann vor, wenn drei identische Donoratome eine Dreiecksfläche im (pseudo)oktaedrischen Koordinationspolyeder und drei identische, aber von den ersten verschiedene Donoratome, eine andere Dreiecksfläche im (pseudo)oktaedrischen Koordinationspolyeder besetzen. Analog wird unter einer *meridionalen* Koordination eine solche verstanden, bei der drei identische Donoratome den einen Meridian im (pseudo)okatedrischen Koordinationspolyeder und drei identische, aber von den ersten verschiedene Donoratome, den anderen Meridian im (pseudo)okatedrischen Koordinationspolyeder besetzen. Dies sei im folgenden anhand eines Beispiels einer Koordination von drei N-Donoratomen und drei C-Donoratomen gezeigt (s. Abb. 2). Da sich diese Beschreibung auf Donoratome bezieht, und nicht auf die Cyclen Cy1 und Cy2, die diese Donoratome bereitstellen, können die drei Cyclen Cy1 und die drei Cyclen Cy2 bei jedem Auftreten gleich oder verschieden sein und trotzdem einer *facialen* oder *meridionalen* Koordination im Sinne dieser Anmeldung entsprechen.

Als identische Donoratome werden solche verstanden, die aus den gleichen Elementen (z.B. Stickstoff) bestehen, unabhängig ob diese Elemente in unterschiedlichen Strukturen bzw. cyclischen Strukturen eingebaut sind.

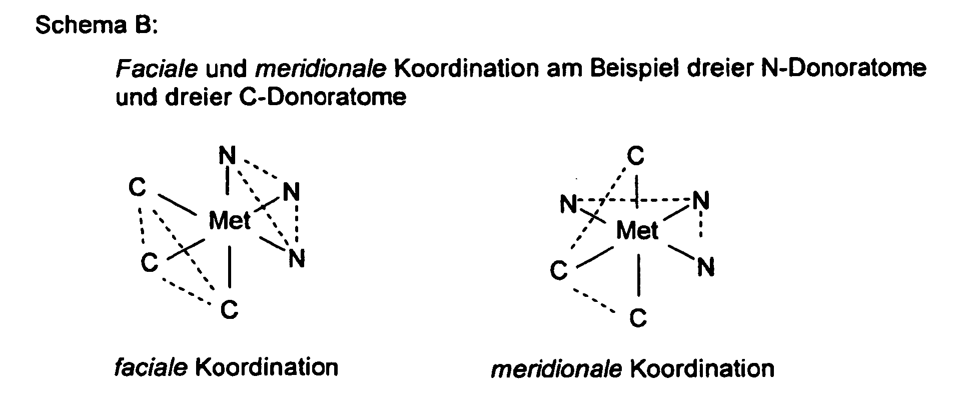

Insbesondere bevorzugt sind Metallkomplexe gemäß den Verbindungen (1) bis (8) mit *facialer* Koordinationsgeometrie am Metall gemäß Schema 1, 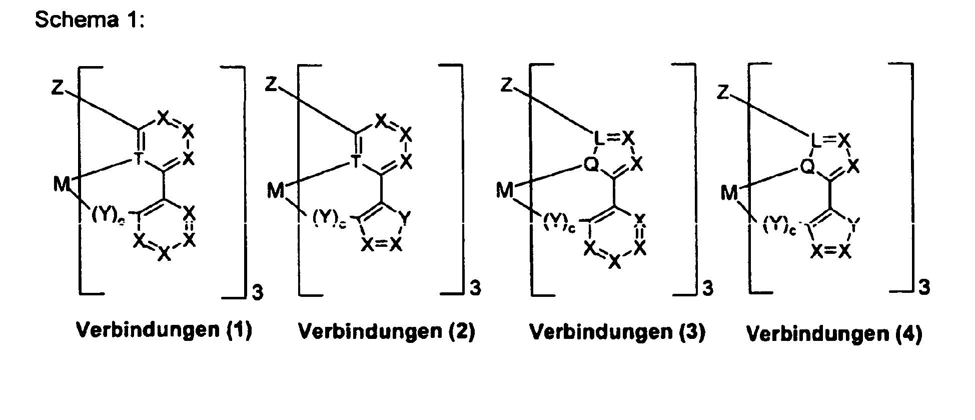 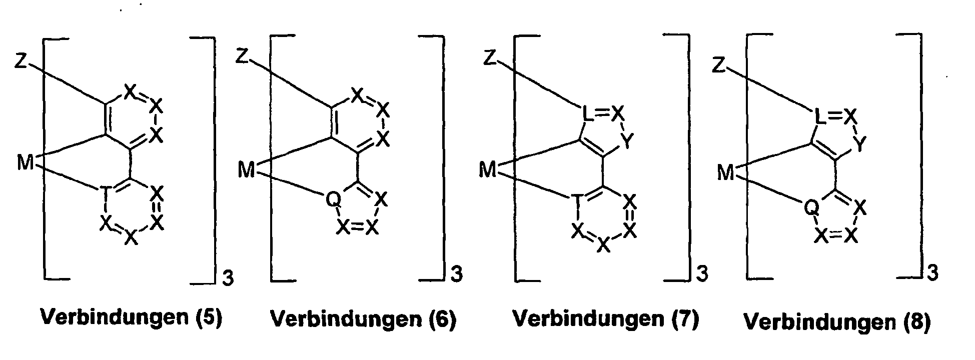 wobei die Symbole und Indizes folgende Bedeutung haben:
- M: Al, Ga, In, Tl, P, As, Sb, Bi, Sc, Y, La, V, Nd Ta, Cr, Mo, W, Fe, Ru, Os, Co, Rh, lr, Cu, Au, Pr, Nd, Sm, Eu, Gd, Tb, Dy, Ho, Er, Tm, Yb, Lu;
- L: ist gleich oder verschieden bei jedem Auftreten C, N, P;
- Q: ist gleich oder verschieden bei jedem Auftreten O, S, Se, Te, N;
- T: ist gleich oder verschieden bei jedem Auftreten N, P, C;
- X: ist gleich oder verschieden bei jedem Auftreten CR, N, P;
- Y: ist gleich oder verschieden bei jedem Auftreten NR¹, O, S, Se, Te, SO, SeO, TeO, SO₂, SeO₂, TeO₂;
- Z: B, BR, B(CR₂)₃, B(CR₂CR₂)₃, CR, COH, COR¹, CF, CCI, CBr, C-l, CNR¹₂, RC(CR₂)₃, RC(CR₂CR₂)₃, RC(SiR₂)₃, RC(SiR₂CR₂)₃, RC(CR₂SiR₂)₃, RC(SiR₂SiR₂)₃, cis,cis-1,3,5-Cyclohexyl, 1,3,5-(CR₂)₃C₆H₃, SiR, SiOH, SiOR¹, RSi(CR₂)₃, RSi(CR₂CR₂)₃, RSi(SiR₂)₃, RSi(SiR₂CR₂)₃, RSi(CR₂SiR₂)₃, RSi(SiR₂SiR₂)₃, N, N(CR₂)₃, N(C=O)₃, N(CR₂CR₂)₃, NO, P, As, Sb, Bi, PO, AsO, SbO, BiO, PSe, AsSe, SbSe, BiSe, PTe, AsTe, SbTe, BiTe;
- R: ist gleich oder verschieden bei jedem Auftreten H, F, Cl, Br, I, NO₂, CN, eine geradkettige oder verzweigte oder cyclische Alkyl- oder Alkoxygruppe mit 1 bis 20 C-Atomen, wobei ein oder mehrere nicht benachbarte CH₂-Gruppen durch -R¹C=CR¹-, -C≡C-, Si(R¹)₂, Ge(R¹)₂, Sn(R¹)₂, C=O, C=S, C=Se, C=NR¹, -O-, -S-, -NR¹- oder -CONR¹- ersetzt sein können und wobei ein oder mehrere H-Atome durch F ersetzt sein können, oder eine Aryl- oder Heteroarylgruppe mit 1 bis 14 C-Atomen, die durch einen oder mehrere, nicht aromatische Reste R substituiert sein kann, wobei mehrere Substituenten R, sowohl am selben Ring als auch an den beiden unterschiedlichen Ringen zusammen wiederum ein weiteres mono- oder polycyclisches, aliphatisches oder aromatisches Ringsystem aufspannen können;
- R¹: sind gleich oder verschieden bei jedem Auftreten H oder ein aliphatischer oder aromatischer Kohlenwasserstoffrest mit 1 bis 20 C-Atomen;
- c: ist gleich oder verschieden bei jedem Auftreten 0 oder 1.

Darüberhinaus sind ebenfalls bevorzugt die Verbindungen (9) bis (12) mit *meridionaler* Koordinationsgeometrie am Metall gemäß Schema 2, 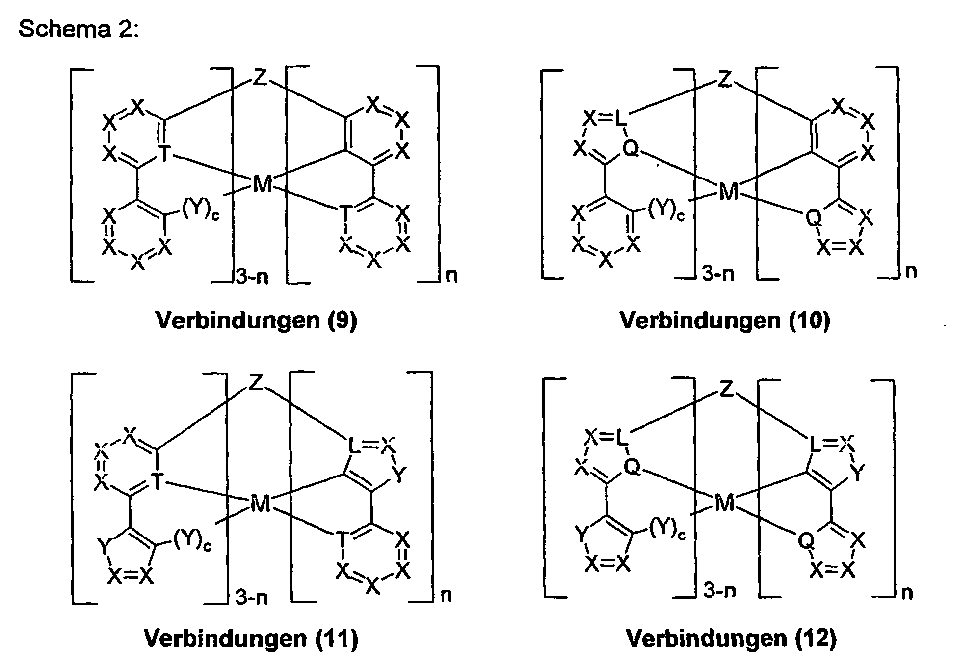 wobei die Symbole und Indizes M, L, Q, T, X, Y, Z, R, R¹ und c die unter Schema 1 angegebene Bedeutung haben, und wobei:
- n: 1 oder 2 ist.

Ein weiterer Gegenstand der Erfindung sind ebenfalls solche Verbindungen, die gleichzeitig Teilliganden vom Typ wie bei Verbindungen (1), (2), (3) und/oder (4) aufweisen, d.h. gemischte Ligandensysteme. Diese werden durch die Formeln (13) bis (30) - gemäß Schema 3 - beschrieben: 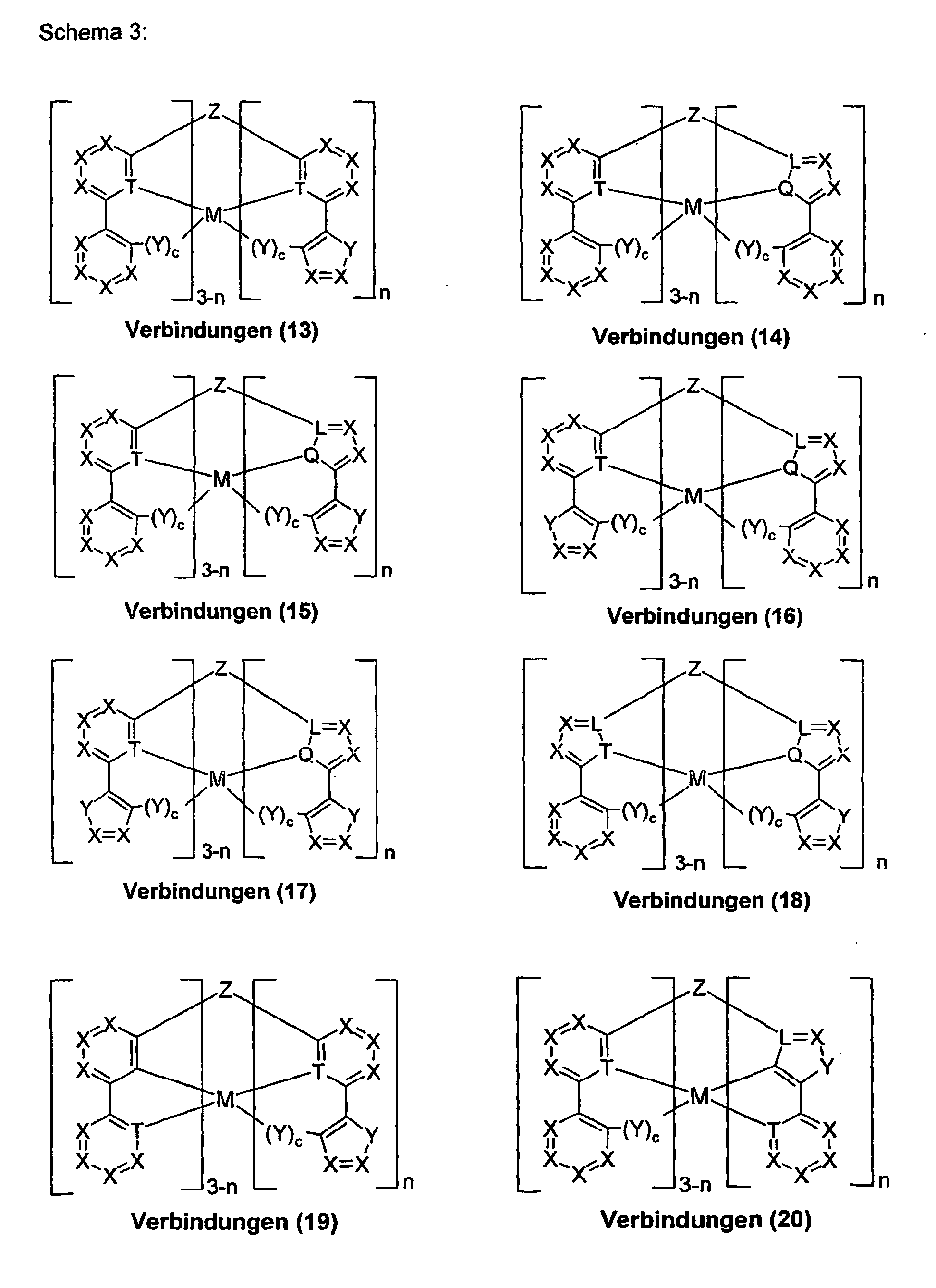 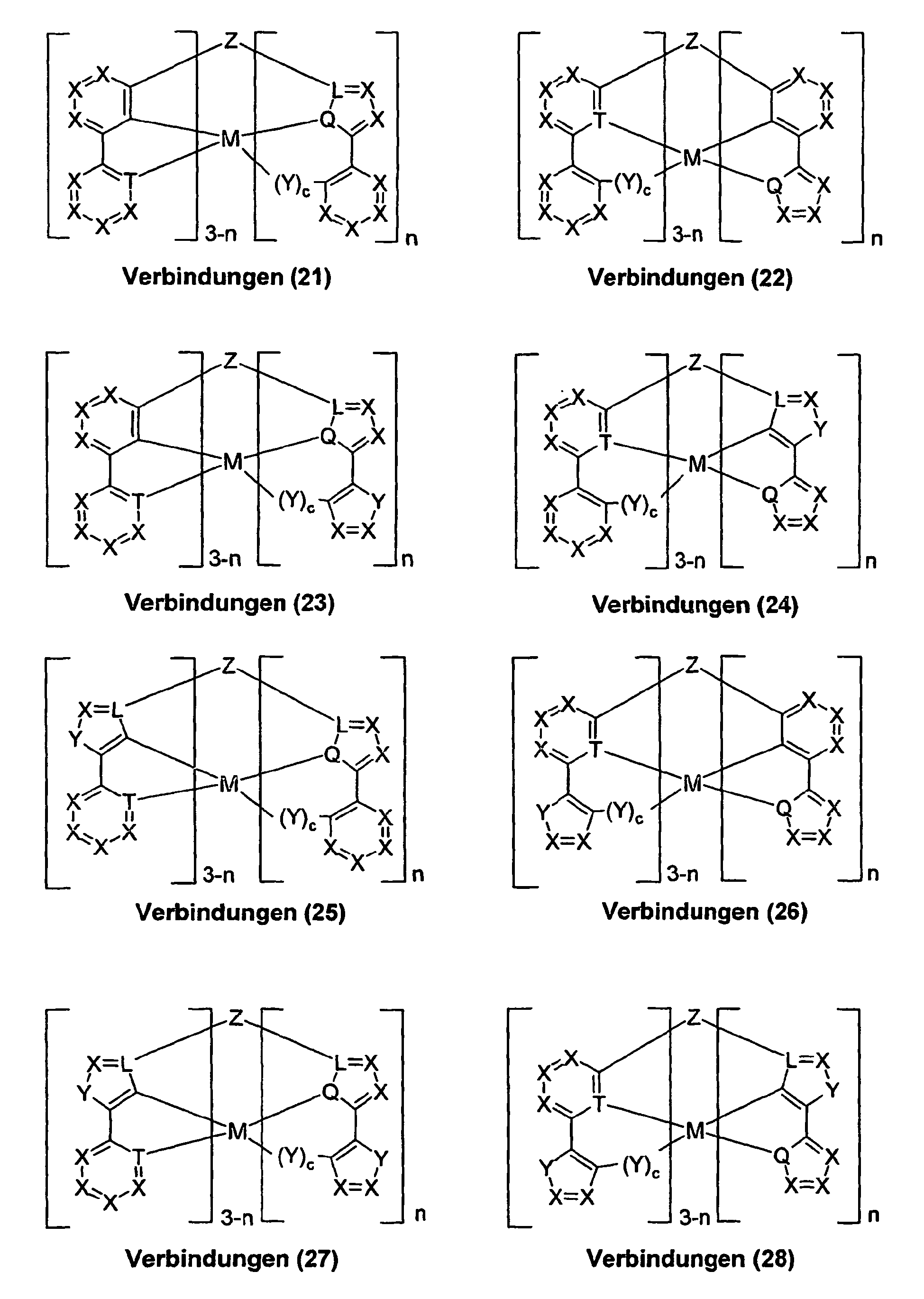 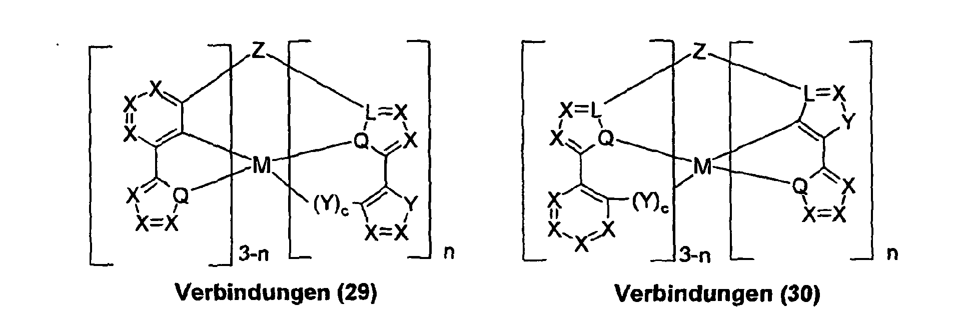 wobei die Symbole und Indizes M, L, Q, T, X, Y, Z, R, R¹, c und n die unter Schema 1 und 2 angegebene Bedeutung haben.

Ein weiterer Gegenstand der Erfindung sind ebenfalls die Verbindungen (31) bis (41), die kondensierte aromatische Ligandensysteme enthalten, gemäß Schema 4: 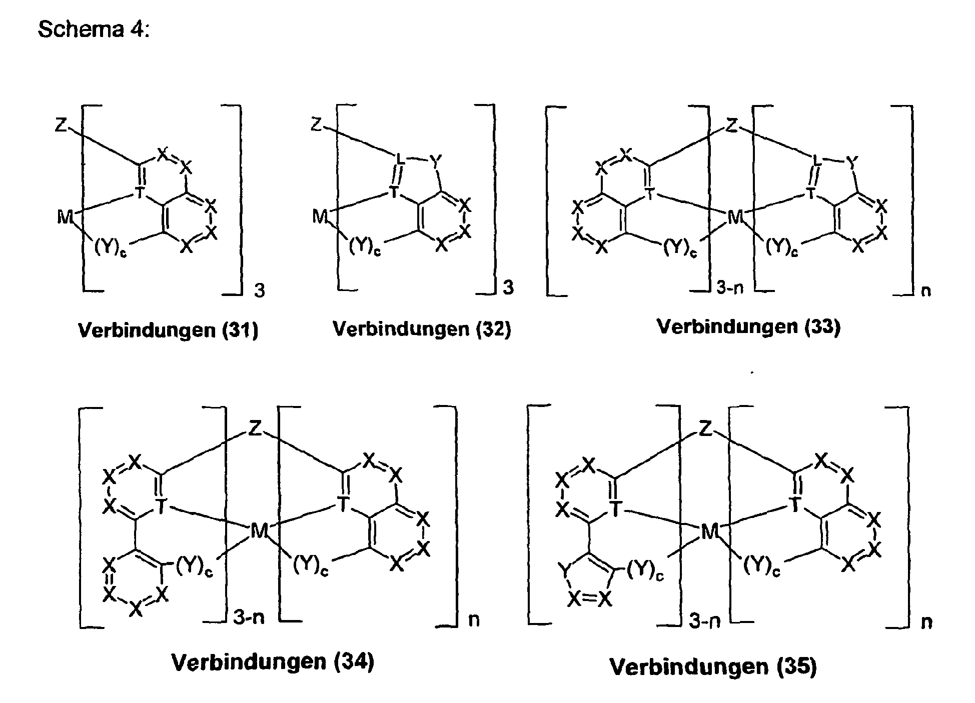 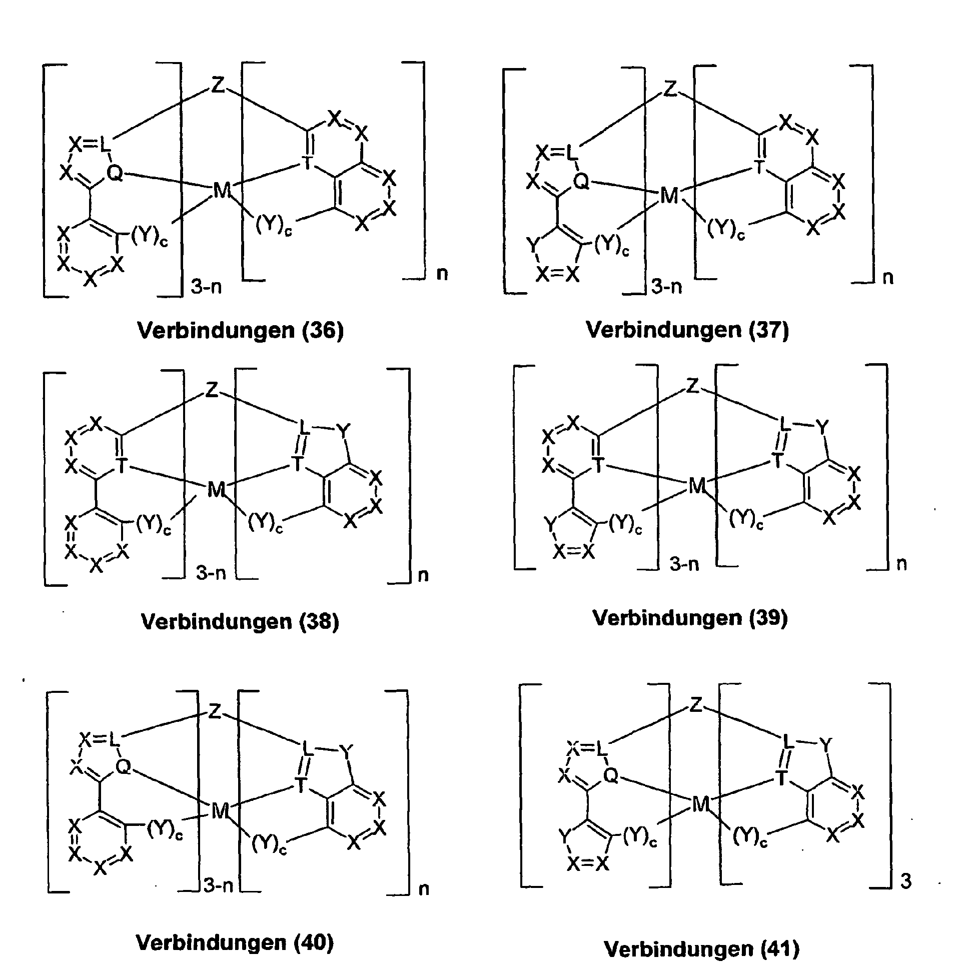 wobei die Symbole und Indizes M, L, Q, T, X, Y, Z, R, R¹, c und n die unter Schema 1 und 2 angegebene Bedeutung haben.

Bevorzugt sind erfindungsgemäße Verbindungen (1) bis (41), bei denen für das Symbol M = Al, Ga, In, Sc, Y, La, Ru, Os, Rh, Ir, Au gilt.

Bevorzugt sind ebenfalls erfindungsgemäße Verbindungen (1) bis (41), bei denen für das Symbol L = C, N gilt.

Bevorzugt sind ebenfalls erfindungsgemäße Verbindungen (1) bis (41), bei denen für das Symbol Q = O, S gilt.

Bevorzugt sind ebenfalls erfindungsgemäße Verbindungen (1) bis (41), bei denen für das Symbol T = N gilt.

Bevorzugt sind ebenfalls erfindungsgemäße Verbindungen (1) bis (41), bei denen für das Symbol X = CR, N gilt.

Bevorzugt sind ebenfalls erfindungsgemäße Verbindungen (1) bis (41), bei denen für das Symbol Z = B, CH, CR¹, COR¹, CF, CCI, CBr, SiR, N, P, PO, RC(CR₂)₃, RC(CR₂CR₂)₃, cis,cis-1,3,5-Cyclohexyl, RSi(CR₂)₃, RSi(CR₂CR₂)₃, N(CR₂)₃, N(C=O)₃, N(CR₂CR₂)₃ gilt.

Bevorzugt sind ebenfalls erfindungsgemäße Verbindungen (1) bis (41), bei denen für das Symbol Y = O, S gilt.

Bevorzugt sind ebenfalls erfindungsgemäße Verbindungen (1) bis (41), bei denen das Symbol R für H, F, Cl, Br, I, CN, eine geradkettige oder verzweigte oder cyclische Alkyl- oder Alkoxygruppe mit 1 bis 6 C-Atomen oder eine Aryl- oder Heteroarylgruppe mit 3 bis 8 C-Atomen steht, die durch einen oder mehrere, nicht aromatische Reste R substituiert sein kann, wobei mehrere Substituenten R, sowohl am selben Ring als auch an den beiden unterschiedlichen Ringen zusammen wiederum ein weiteres mono- oder polycyclisches, aliphatisches oder aromatisches Ringsystem aufspannen können.

Sofern in den Verbindungen (1) bis (41) durch die Reste R Ringsysteme aufgespannt werden, sind diese bevorzugt Benzol, 1- bzw. 2-Naphthalin, 1-, 2- bzw. 9-Anthracen, 2-, 3- bzw. 4-Pyridin, 2-, 4- bzw. 5-Pyrimidin, 2-Pyrazin, 3- bzw. 4-Pyridazin, 2-, 3-, 4-, 5-, 6-, 7- bzw. 8-Chinolin, 2- bzw. 3-Pyrrol, 3-, 4- bzw. 5-Pyrazol, 2-, 4- bzw. 5-Imidazol, 2- bzw. 3-Thiophen, 2- bzw. 3-Selenophen, 2- bzw. 3-Furan, 2-(1,3,4-Oxadiazol), Indol oder Carbazol.

Ebenfalls Gegenstand der vorliegenden Erfindung sind die polypodalen Liganden gemäß Verbindungen (42) bis (82), gemäß Schema 5: 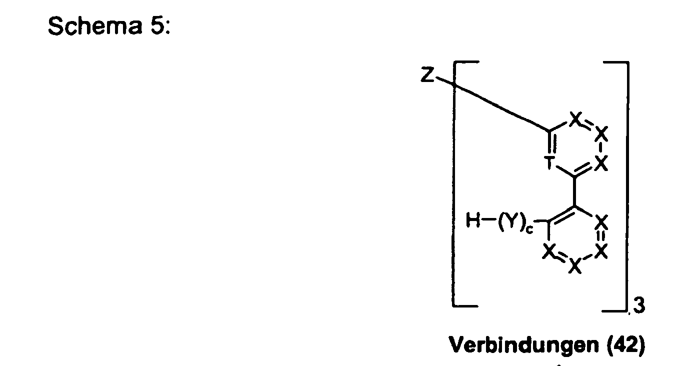 wobei die Symbole und Indizes T, Y, Z, R, R¹, c und n die unter Schema 1 und 2 angegebene Bedeutung haben und X, gleich oder verschieden bei jedem Auftreten, CR oder P ist; 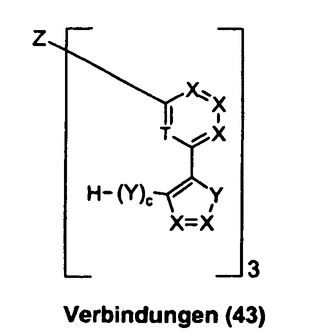 wobei die Symbole und Indizes T, X, Y, Z, R, R¹, c und n die unter Schema 1 und 2 angegebene Bedeutung haben; 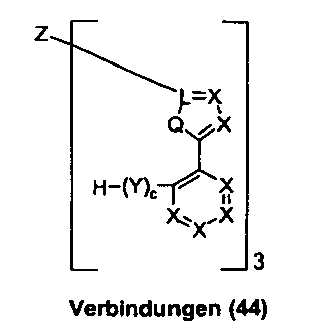 wobei die Symbole und Indizes Q, T, X, Y, Z, R, R¹, c und n die unter Schema 1 und 2 angegebene Bedeutung haben und L, gleich oder verschieden bei jedem Auftreten, C oder P ist; 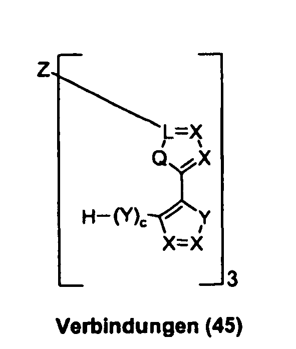 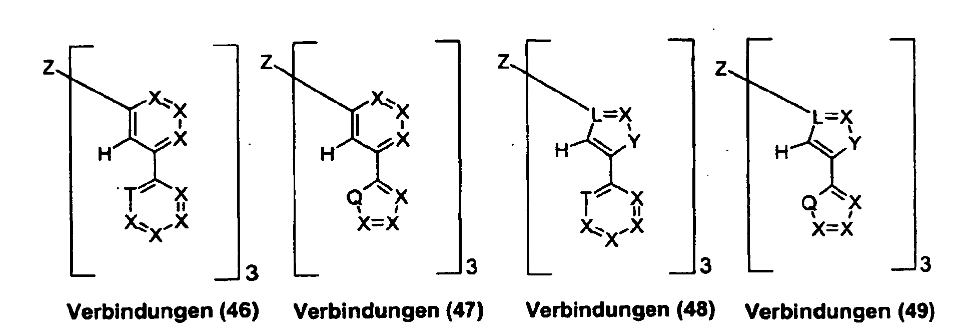 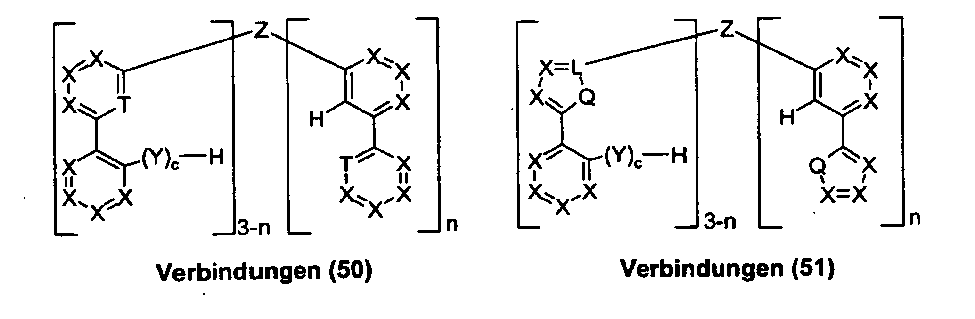 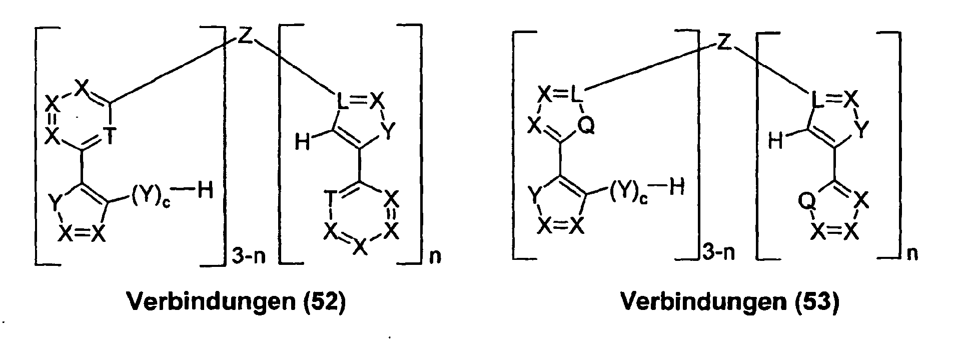 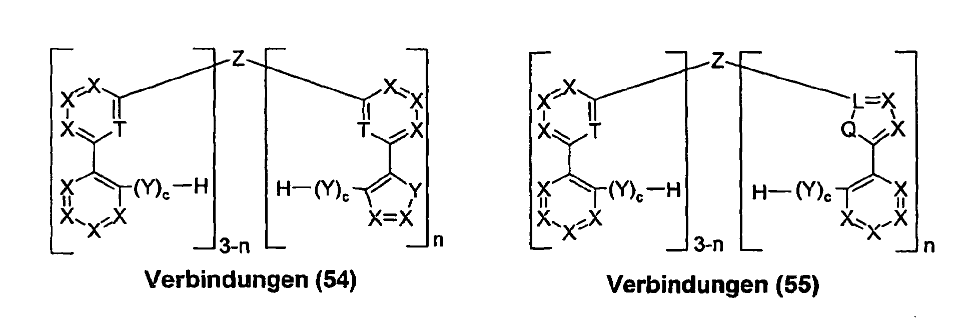 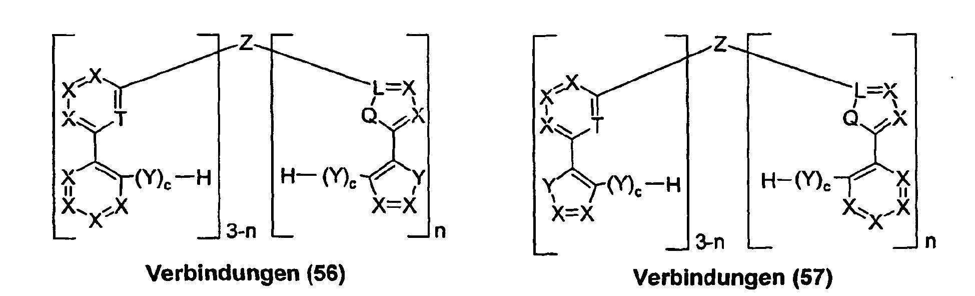 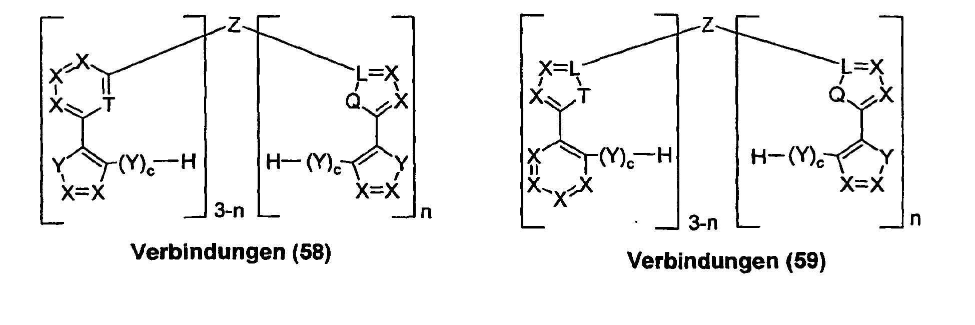 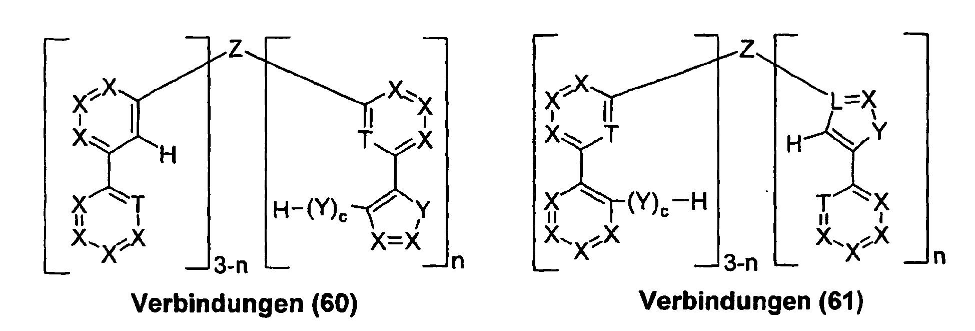 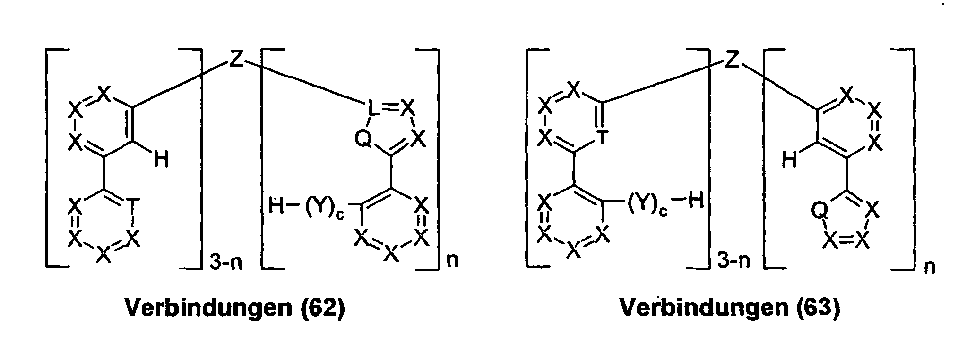 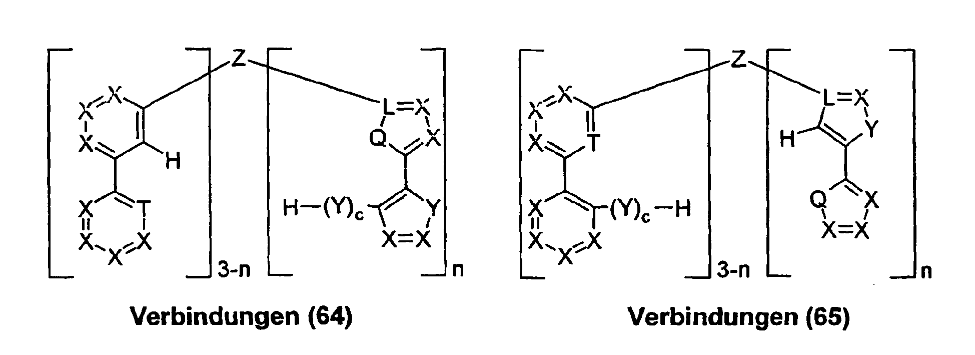 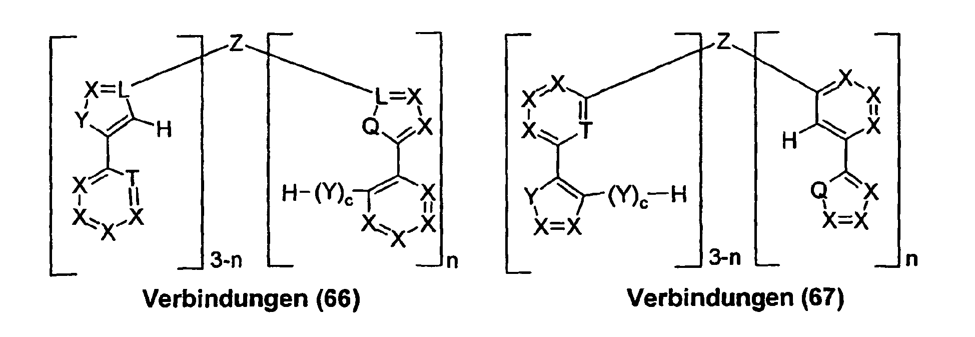 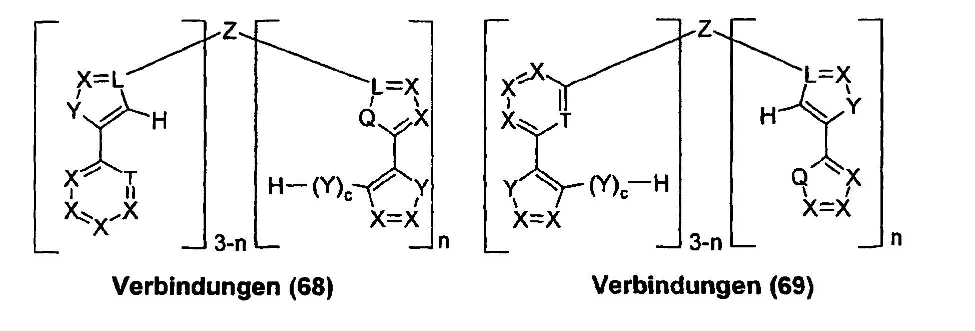 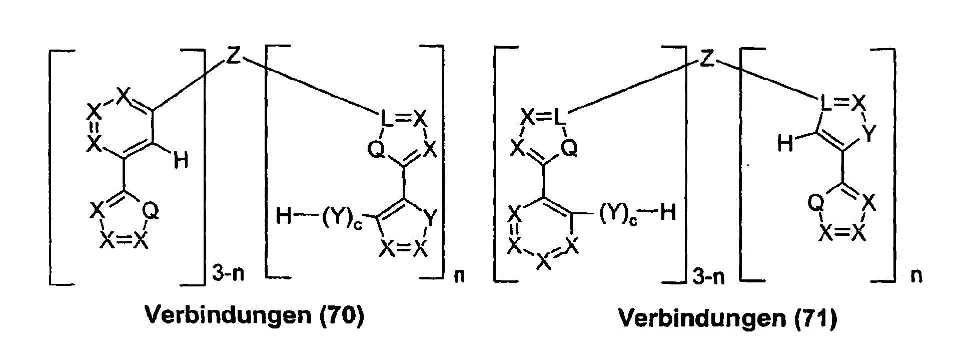 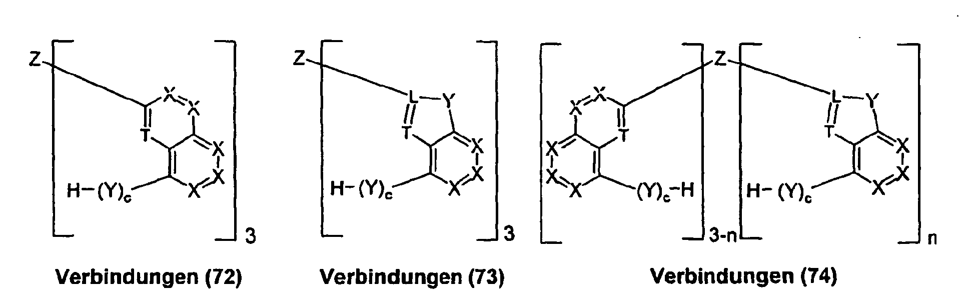 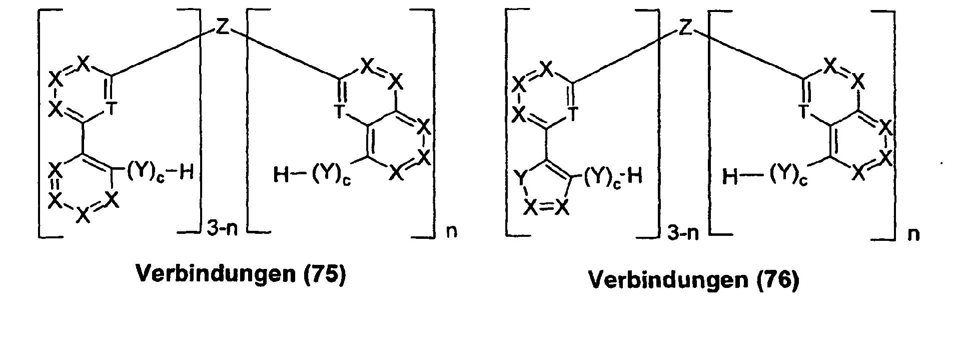 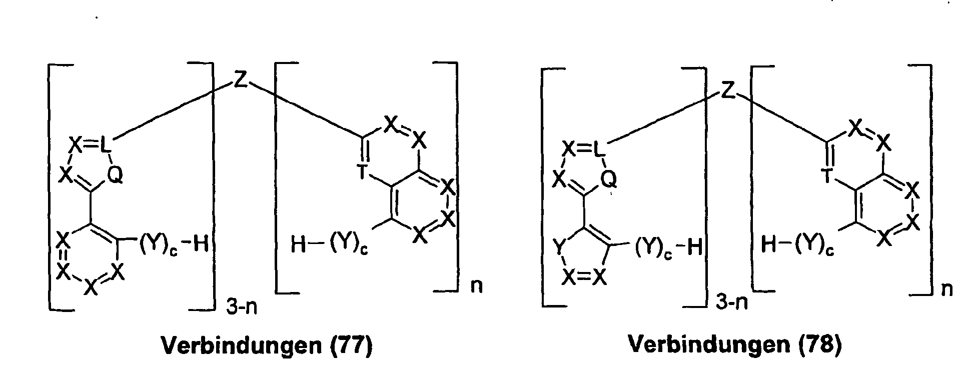 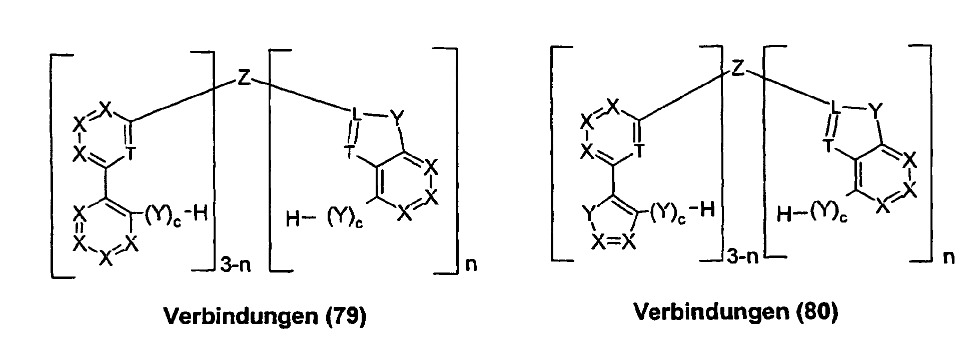 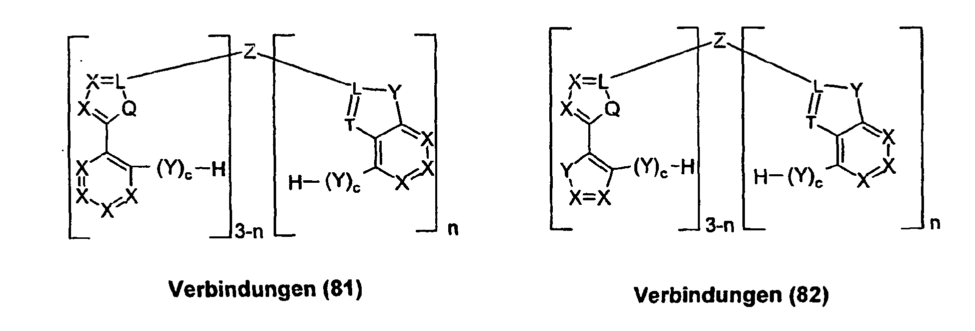 wobei die Symbole und Indizes Q, L, T, X, Y, Z, R, R¹, c, n die unter Schema 1 und 2 angegebene Bedeutung haben.

Die erfindungsgemäßen Verbindungen (1) bis (41) sind prinzipiell durch verschiedene Verfahren herstellbar, wobei sich jedoch das im folgenden beschriebene Verfahren als besonders gut geeignet herausgestellt hat.

Daher ist ein weiterer Gegenstand der vorliegenden Erfindung ein Verfahren zur Herstellung der Verbindungen (1) bis (41) durch Umsetzung der polypodalen Liganden gemäß Verbindungen (42) bis (82) mit Metallalkoholaten der Formel (83), mit Metallketoketonaten der Formel (84) und Metallhalogeniden der Formel (85), 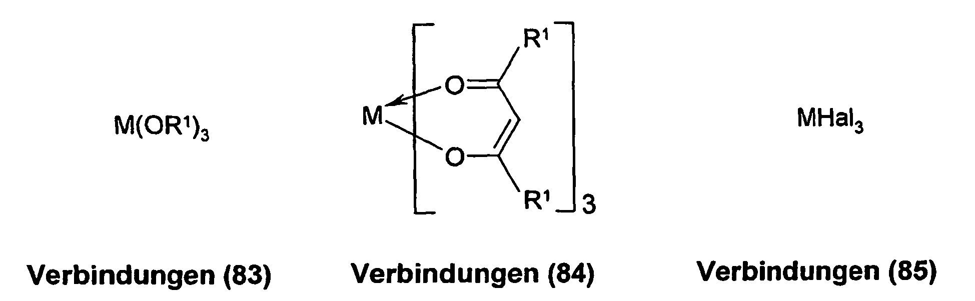 wobei das Symbol R¹ die unter Schema 1 angegebene Bedeutung hat und Hal = F, Cl, Br, l ist.

Durch diese Verfahren lassen sich die Komplexe leicht in hoher Reinheit, bevorzugt in einer Reinheit von > 99%, nach ¹H-NMR oder HPLC, erhalten.

Mit den hier erläuterten Synthesemethoden lassen sich unter anderem die im folgenden dargestellten Beispiele für Verbindungen (1) bis (41) herstellen.

| | | |
|---|---|---|
| 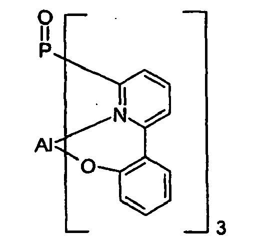 | 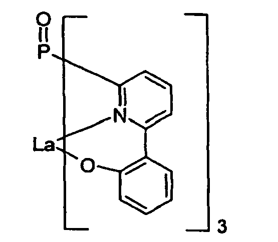 | 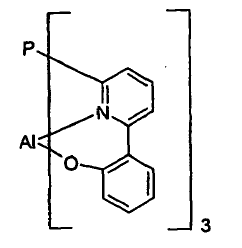 |
| Beispiel 1 | Beispiel 2 | Beispiel 3 |
| 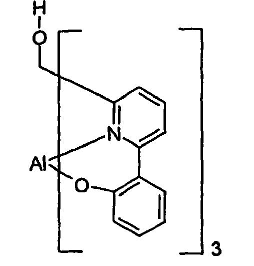 | 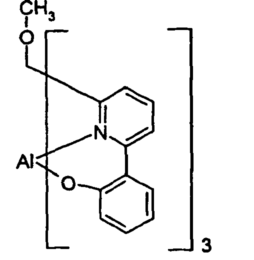 | 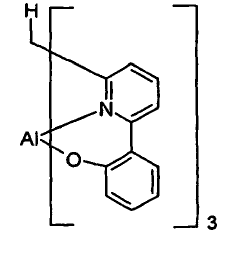 |
| Beispiel 4 | Beispiel 5 | Beispiel 6 |
| 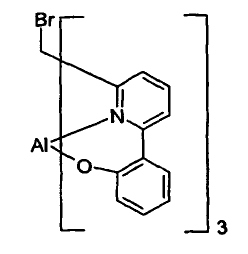 | 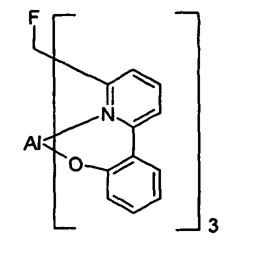 | 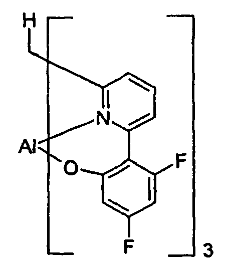 |
| Beispiel 7 | Beispiel 8 | Beispiel 9 |
| 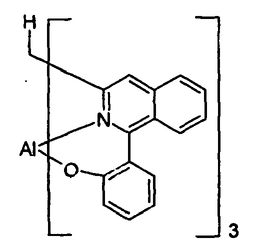 | 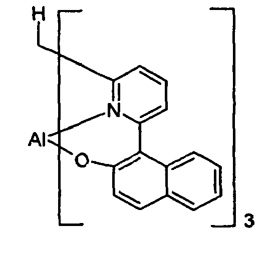 | 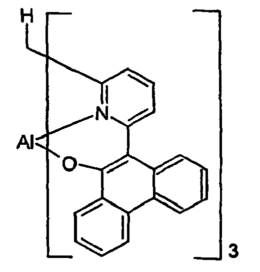 |
| Beispiel 10 | Beispiel 11 | Beispiel 12 |
| 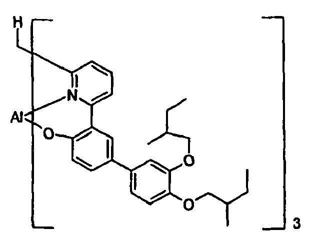 | 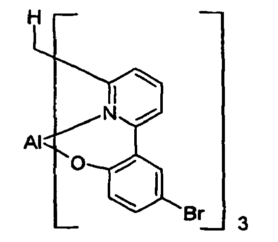 | 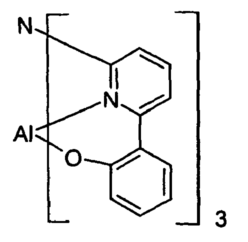 |
| Beispiel 13 | Beispiel 14 | Beispiel 15 |
| 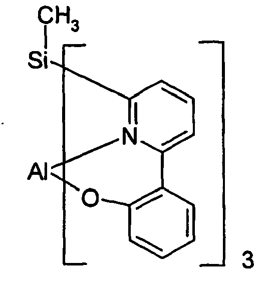 | 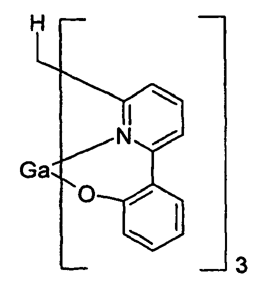 | 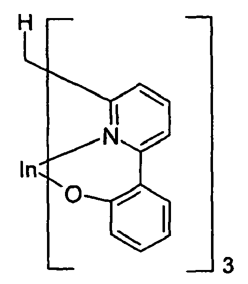 |
| Beispiel 16 | Beispiel 17 | Beispiel 18 |
| 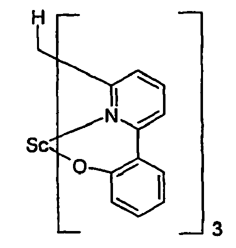 | 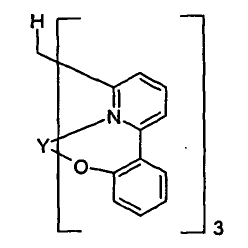 | 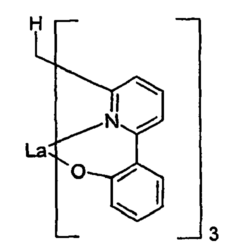 |
| Beispiel 19 | Beispiel 20 | Beispiel 21 |
| 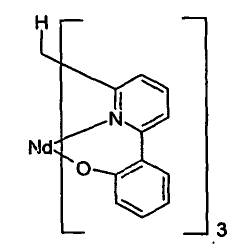 | 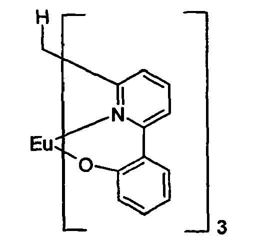 | 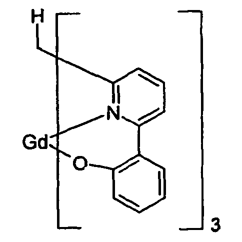 |
| Beispiel 22 | Beispiel 23 | Beispiel 24 |
| 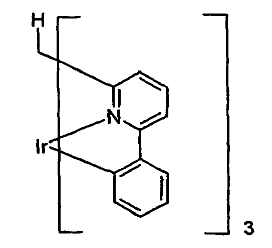 | 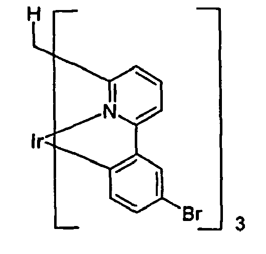 | 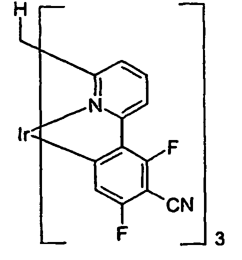 |
| Beispiel 25 | Beispiel 26 | Beispiel 27 |
| 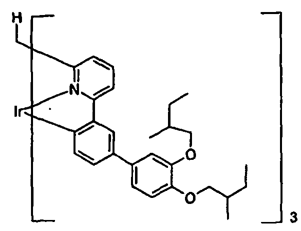 Beispiel 28 | 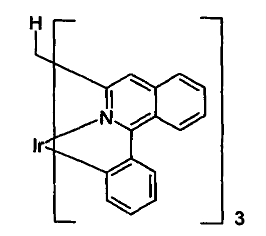 Beispiel 29 | 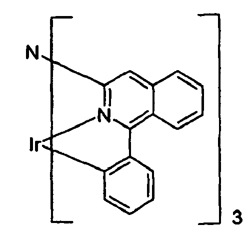 Beispiel 30 |
| 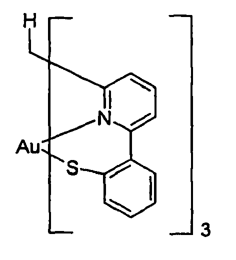 | 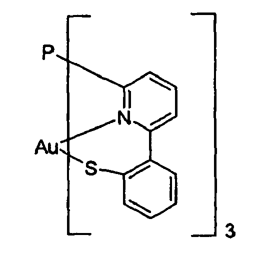 | 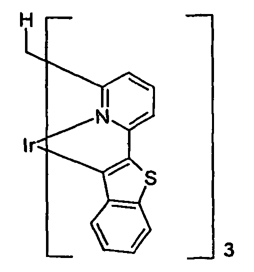 |
| Beispiel 31 | Beispiel 32 | Beispiel 33 |
| 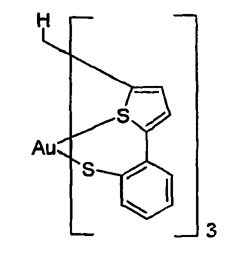 | 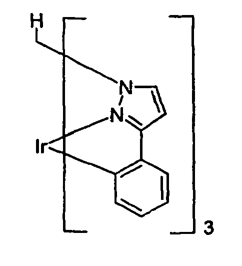 | 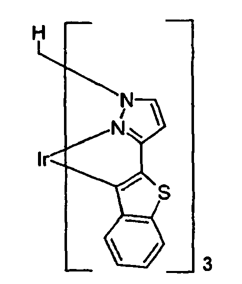 |
| Beispiel 34 | Beispiel 35 | Beispiel 36 |
| 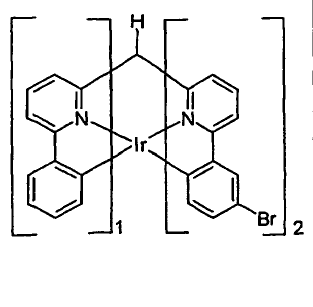 | 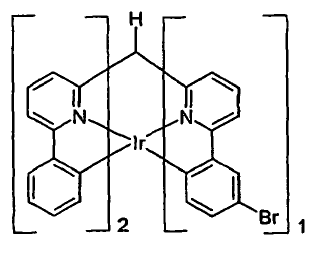 | 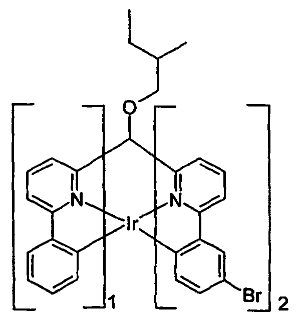 |
| Beispiel 37 | Beispiel 38 | Beispiel 39 |
| 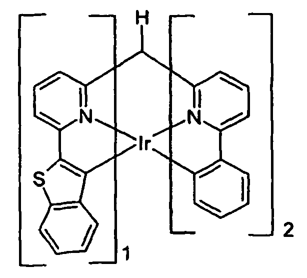 | 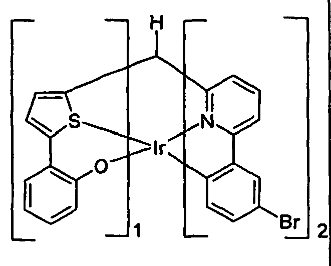 | 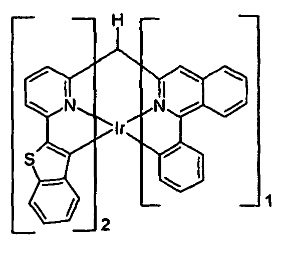 |
| Beispiel 40 | Beispiel 41 | Beispiel 42 |
| 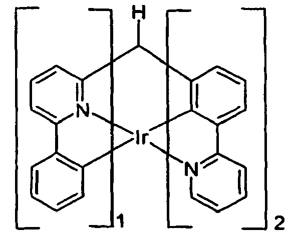 | 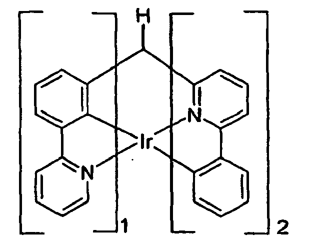 | 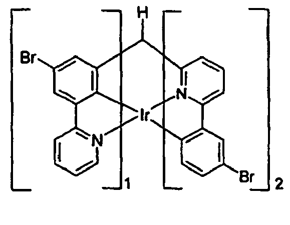 |
| Beispiel 43 | Beispiel 44 | Beispiel 45 |
| 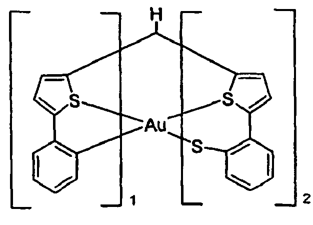 | 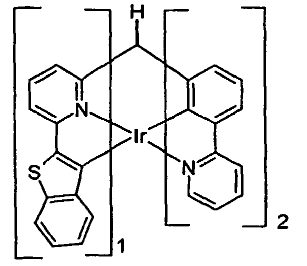 | 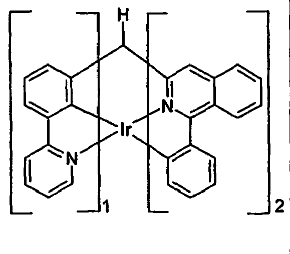 |
| Beispiel 46 | Beispiel 47 | Beispiel 48 |
| 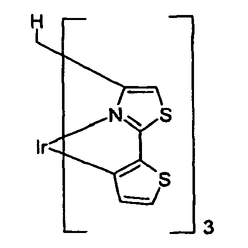 | 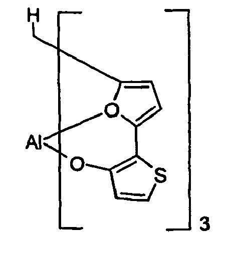 | 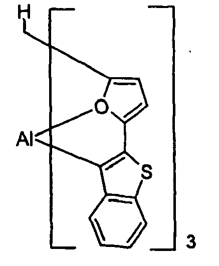 |
| Beispiel 49 | Beispiel 50 | Beispiel 51 |
| 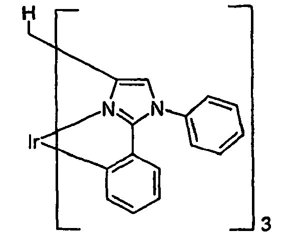 | 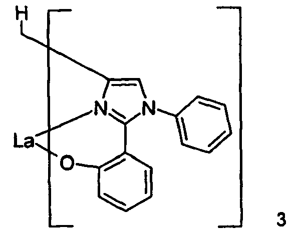 | 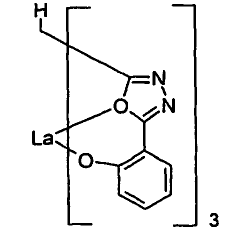 |
| Beispiel 52 | Beispiel 53 | Beispiel 54 |
| 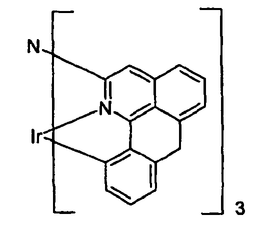 | 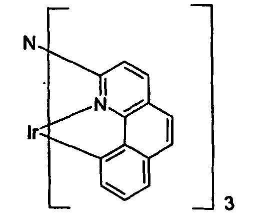 | 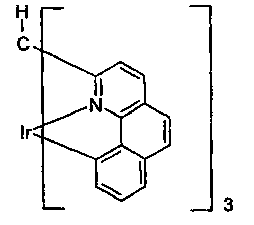 |
| Beispiel 55 | Beispiel 56 | Beispiel 57 |
| 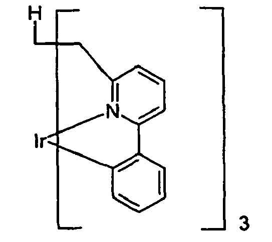 | 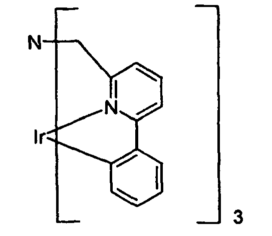 | 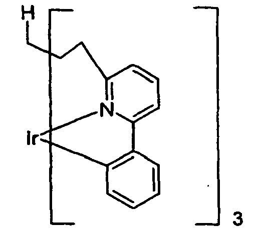 |
| Beispiel 58 | Beispiel 59 | Beispiel 60 |
| 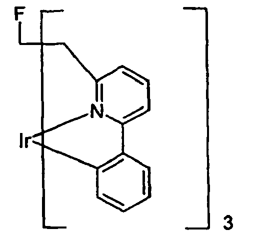 | 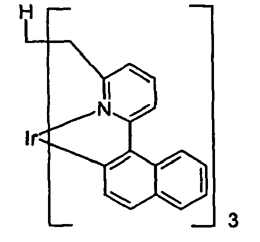 | 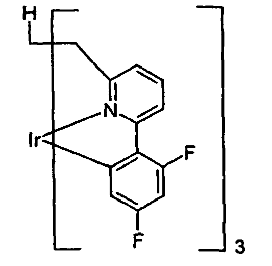 |
| Beispiel 61 | Beispiel 62 | Beispiel 63 |
| 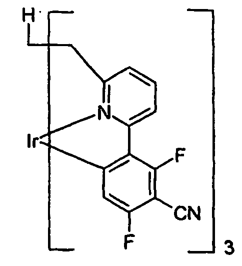 | 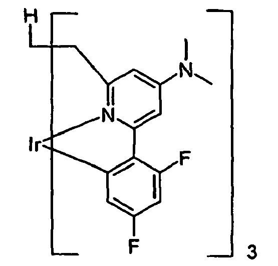 | 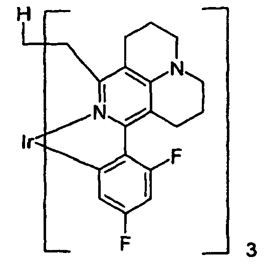 |
| Beispiel 64 | Beispiel 65 | Beispiel 66 |
| 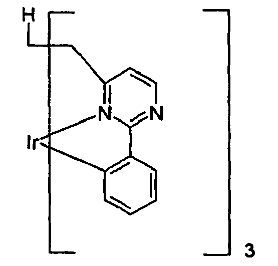 | 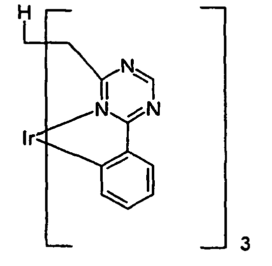 | 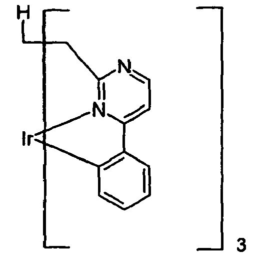 |
| Beispiel 67 | Beispiel 68 | Beispiel 69 |
| 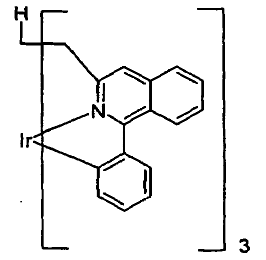 | 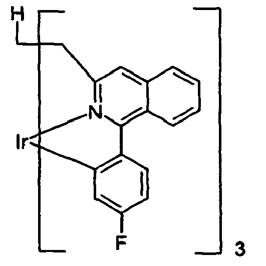 | 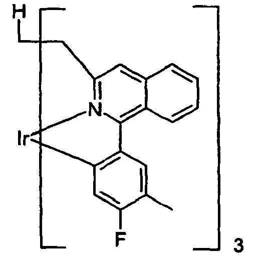 |
| Beispiel 70 | Beispiel 71 | Beispiel 72 |
| 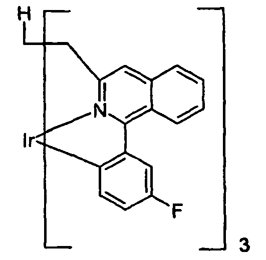 | 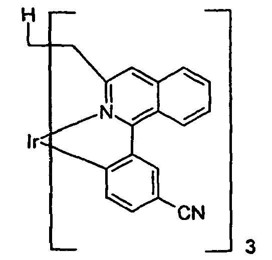 | 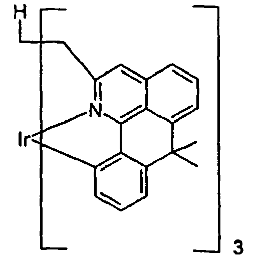 |
| Beispiel 73 | Beispiel 74 | Beispiel 75 |
| 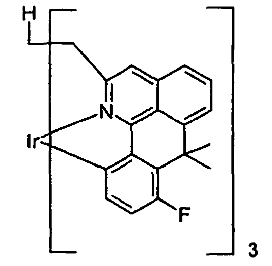 | 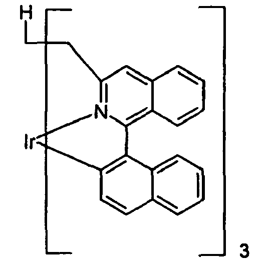 | 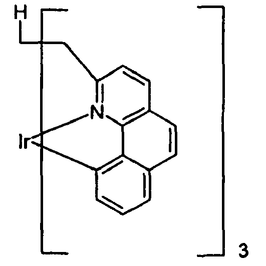 |
| Beispiel 76 | Beispiel 77 | Beispiel 78 |
| 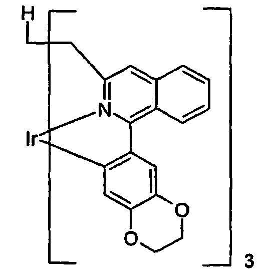 | 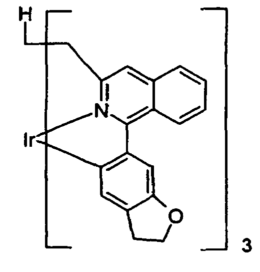 | 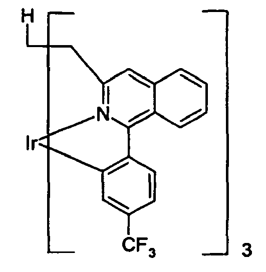 |
| Beispiel 79 | Beispiel 80 | Beispiel 81 |
| 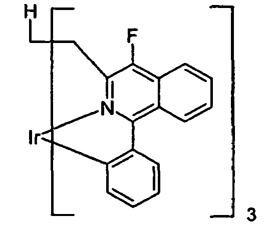 | 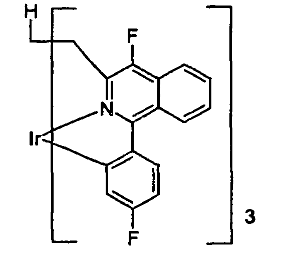 | 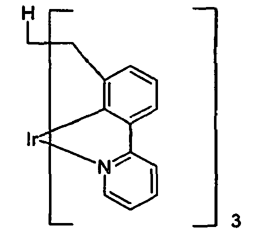 |
| Beispiel 82 | Beispiel 83 | Beispiel 84 |
| 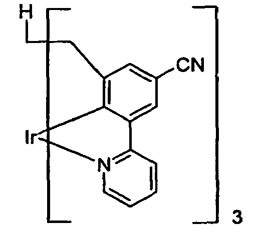 | 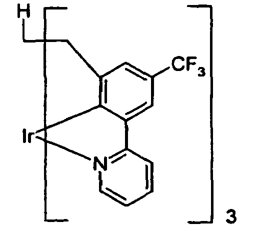 | 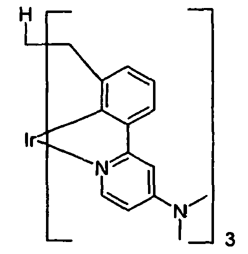 |
| Beispiel 85 | Beispiel 86 | Beispiel 87 |
| 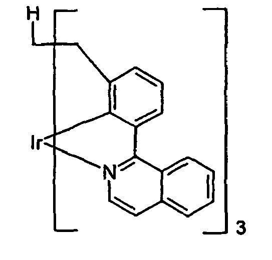 | 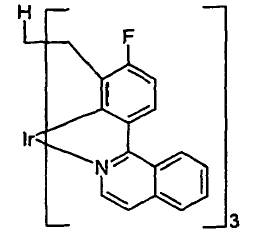 | 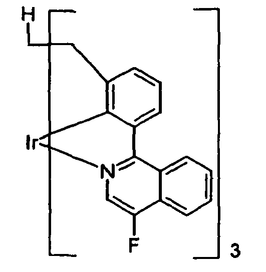 |
| Beispiel 88 | Beispiel 89 | Beispiel 90 |
| 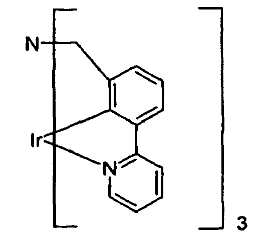 | 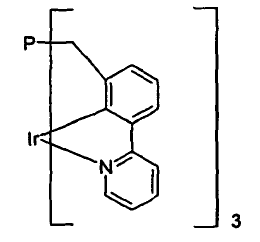 | 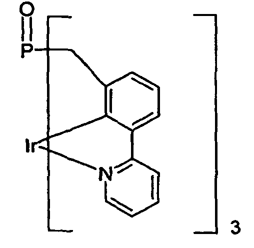 |
| Beispiel 91 | Beispiel 92 | Beispiel 93 |
| 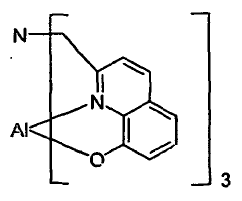 | 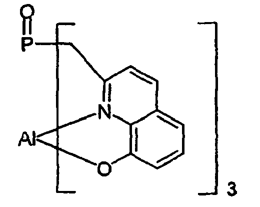 | 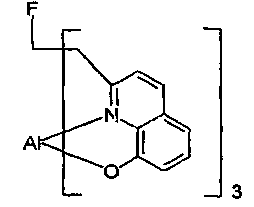 |
| Beispiel 94 | Beispiel 95 | Beispiel 96 |
| 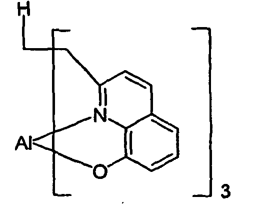 | 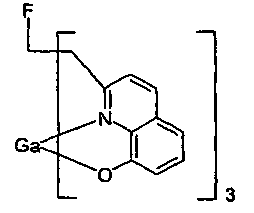 | 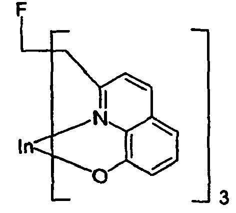 |
| Beispiel 97 | Beispiel 98 | Beispiel 99 |
| 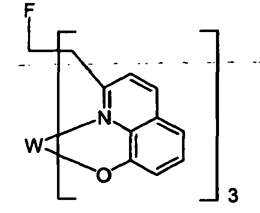 | 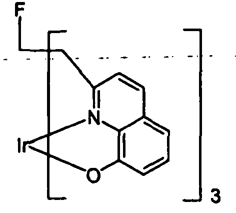 | 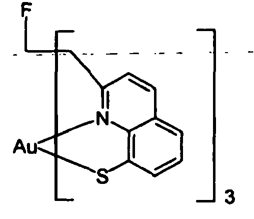 |
| Beispiel 100 | Beispiel 101 | Beispiel 102 |

Die oben beschriebenen erfindungsgemäßen Verbindungen - z.B. Verbindungen gemäß den Beispielen 7, 14, 26, 27, 37, 38, 39 und 41 - können beispielsweise als Co-Monomere zur Erzeugung entsprechender konjugierter, teilkonjugierter oder auch nicht-konjugierter Polymere oder auch als Kern von Dendrimeren - z.B. Verbindungen gemäß den Beispielen 14 und 26 - Verwendung finden. Die entsprechende Einpolymerisation erfolgt dabei bevorzugt über die Halogenfunktionalität.
So können sie u. a. in lösliche Polyfluorene (z. B. gemäß EP-A-842208 oder WO 00/22026), Poly-spirobifluorene (z. B. gemäß EP-A- 707020 oder EP-A-894107), Poly-para-phenylene (z. B. gemäß WO 92/18552), Poly-carbazole (z.B. gemäß den Anmeldungen DE 10304819.7 und DE 10328627.6), Polyvinylcarbazole oder auch Polythiophene (z. B. gemäß EP-A-1028136), oder auch Copolymere aus mehreren dieser Einheiten, einpolymerisiert werden.

Weiterer Gegenstand der Erfindung sind somit konjugierte, teilkonjugierte und nicht-konjugierte Polymere oder Dendrimere enthaltend eine oder mehrere Verbindungen der Formel (1) bis (41), wobei ein oder mehrere der oben definierten R eine Bindung zum Polymer oder Dendrimer darstellt.

Weiterhin können die erfindungsgemäßen Metallkomplexe natürlich auch weiter funktionalisiert werden und so zu *erweiterten Metallkomplexen* umgesetzt werden. Hier ist als Beispiel die Funktionalisierung mit Arylboronsäuren gem. SUZUKI oder mit Aminen gem. HARTWIG-BUCHWALD zu nennen.

Die oben beschriebenen erfindungsgemäßen Verbindungen, Polymere, Dendrimere oder wie oben beschrieben weiter funktionalisierten Verbindungen finden Verwendung als aktive Komponenten in elektronischen Bauteilen, wie z. B. Organischen Leuchtdioden (OLEDs), Organischen Integrierten Schaltungen (O-ICs), Organischen Feld-Effekt-Transistoren (OFETs), Organischen Dünnfilmtransistoren (OTFTs), Organischen Solarzellen (Q-SCs) oder auch Organische Laserdioden (O-Laser).

Aktive Komponenten sind beispielsweise Ladungsinjektions- oder Ladungstransportmaterialien, Ladungsblocklermaterialien und Emissionsmaterialien. Für diese Funktion zeigen die erfindungsgemäßen Verbindungen besonders gute Eigenschaften, wie einerseits vorne schon erläutert und andererseits im folgenden noch näher ausgeführt wird.

Gegenstand der Erfindung ist also weiterhin die Verwendung dieser Verbindungen in elektronischen Bauteilen.

Weiterhin Gegenstand der Erfindung sind elektronische Bauteile, wie z. B. Organische Integrierte Schaltungen (O-ICs), Organische Feld-Effekt-Transistoren (OFETs), Organische Dünnfilmtransistoren (OTFTs), Organische Solarzellen (O-SCs) oder auch Organische Laserdioden (O-Laser), insbesondere aber organische Leuchtdioden (OLEDs), die ein oder mehrere der erfindungsgemäßen Verbindungen, Polymere oder Dendrimere enthalten.

Die vorliegende Erfindung wird durch die folgenden Beispiele für Ladungstranport- bzw. Lochblockermaterialien näher erläutert, ohne sie darauf beschränken zu wollen. Der Fachmann kann aus den Schilderungen ohne erfinderisches Zutun weitere erfindungsgemäße Komplexe, wie beispielsweise Emissionsmaterialien, herstellen bzw. das erfindungsgemäße Verfahren anwenden.

### Beispiele:

### Synthese von homoleptischen Aluminium-, Eisen- und Lanthan-Chelatkomplexen mit hexapodalen Liganden:

Die nachfolgenden Synthesen wurden - sofern nicht anders angegeben - unter einer Schutzgasatmosphäre in getrockneten Lösungsmitteln durchgeführt. Die Edukte wurden von ALDRICH bzw. ABCR [2-Methoxybenzolboronsäure, 2-Brom-4-fluorphenol, 2-Brom-5-fluorphenol, Kaliumfluorid (sprühgetrocknet), Diethylaminoschwefeltrifluorid (DAST), Tri-*tert*-butylphosphin, Palladium(II)acetat, Pyridiniumhydrochlorid, Aluminium-tri-*iso*-propylat, 10 Gew.-%ige Lösung von Tris(2-methoxyethanolato)lanthan(III) in 2-Methoxyethanol] bezogen. Tris(2-brom-6-pyridyl)phosphin und Tris(2-brom-6-pyridyl)methanol wurden, wie in WO 98/22148 beschrieben, dargestellt.

### Beispiel 1: Tris(2-brom-6-pyridyl)phosphinoxid

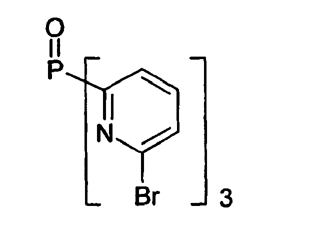

Eine zum Sieden erhitzte Suspension von 50.2 g (100.0 mmol) Tris(2-brom-6-pyridyl)phosphin in 500 ml Chloroform wurde unter intensivem Rühren tropfenweise mit einer Mischung aus 11 ml 35 Gew.%igem H₂O₂ und 50 ml Wasser versetzt, wodurch man eine klare Lösung erhielt. Nach 5 h Rühren unter Rückfluß ließ man die Lösung auf Raumtemperatur abkühlen. Die Lösung wurde mit 500 ml Wasser gewaschen, die organische Phase wurde abgetrennt und im Vakuum auf 50 ml eingeengt. Nach 2 h Stehen wurden die ausgeschiedenen Kristalle abfiltriert, dreimal mit 100 ml *n*-Hexan gewaschen und anschließend im Vakuum bei 70 °C getrocknet. Die Ausbeute bei einer Reinheit von 99.0% betrug 47,1 g (90.9 %).
¹H-NMR (CDCl₃): δ [ppm] = 8.14 (ddd, ³J_{HP} = 5.4 Hz, ³J_{HH} = 7.8 Hz, ⁴J_{HH} = 1.0 Hz, 3 H, H-3), 7.69 (ddd, ⁴J_{HP} = 4.6 Hz, ³J_{HH} = 7.9 Hz, ³J_{HH} = 7.9 Hz, 3 H, H-4), 7.61 (ddd, ⁵J_{HP} = 2.1 Hz, ³J_{HH} = 7.9 Hz, ⁴J_{HH} = 1.0 Hz, 3 H, H-5).
³¹P{¹H}-NMR (CDCl₃): δ [ppm] = 11.8 (s).

### Beispiel 2: Tris(6-(2-methoxyphenyl)-2-pyridyl)phosphinoxid

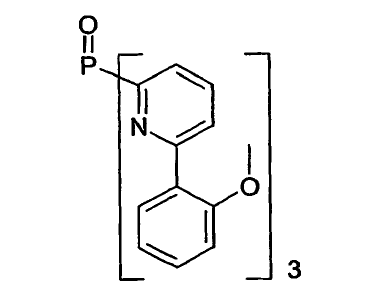

Eine gut gerührte Suspension von 38.8 g (75.0mmol) Tris(2-brom-6-pyridyl)phosphinoxid, 51.3 g (337.5 mmol) 2-Methoxybenzolboronsäure und 43.1 g (742.5 mmol) Kaliumfluorid in 750 ml wasserfreiem THF wurde mit 593 mg (2.93 mmol) Tri*-tert* butylphosphin und dann mit 505 mg (2.25 mmol) Palladium(II)acetat versetzt und anschließend 16 h unter Rückfluß erhitzt. Nach Abkühlen wurde die Reaktionsmischung mit 1500 ml Essigsäureethylester und 1000 ml Wasser versetzt. Die organische Phase wurde abgetrennt, zweimal mit 500 ml Wasser und einmal mit 500 ml ges. Natriumchloridlösung gewaschen und anschließend über Magnesiumsulfat getrocknet. Nach Einengen der organischen Phase im Vakuum (Enddruck 1 mbar, Temperatur 90 °C) verblieben 44.3 g (98.5 %) eines fahlgelben hochviskosen Öls, das ohne Reinigung weiter umgesetzt wurde.
¹H-NMR (CDCl₃): δ [ppm] = 8.14 (ddd, 3H), 8.02 (ddd, 3 H), 7.85 (dd, 3 H), 7.76 (ddd, 3 H), 7.30 (ddd, 3 H), 6.94 (dd, 3 H), 6.87 (ddd, 3 H), 3.10 (s, 9 H, CH₃).
³¹P{¹H}-NMR (CDCl₃): δ [ppm] = 14.0 (s).

### Beispiel 3: Tris(6-(2-hydroxyphenyl)-2-pyridyl)phosphinoxid, (PPL-01)

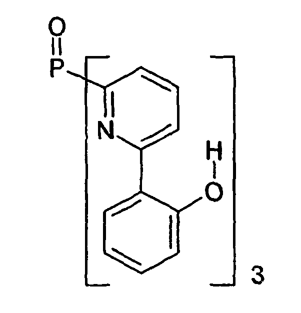

Eine Mischung aus 30.0 g (50 mmol) Tris(6-(2-methoxyphenyl)-2-pyridyl)phosphinoxid und 104.0 g (900 mmol) Pyridiniumhydrochlorid wurden 12h bei 130 °C gerührt. Nach Abkühlen der Schmelze auf 80 °C wurde mit 300 ml Wasser und dann mit einer Lösung von 44.9 g (800 mmol) Kaliumhydroxid in 100 ml Wasser versetzt. Die wäßrige Phase wurde dreimal mit 500 ml Dichlormethan extrahiert. Die vereinigten organischen Phasen wurden dreimal mit 500 ml Wasser gewaschen. Nach Trocknen der organischen Phase über Magnesiumsulfat und Abziehen des Dichlormethans wurde der ölige Rückstand in 100 ml Methyl-tert-butylether aufgenommen und mit 100 ml *n*-Heptan versetzt. Nach 12 h Stehen wurden die farblosen Kristalle abgesaugt und aus Methyl-*tert*-butylether / *n*-Heptan umkristallisiert. Die Ausbeute betrug 10.9 g (39.1 %) bei einer Reinheit von größer 99.0% nach ¹H-NMR.
¹H-NMR (CDCl₃): δ [ppm] = 14.33 (s, 3H, OH), 8.45 (m, 3H), 8.13 (m, 3H), 7.88 (m, 3H), 7,61 (m, 3H), 7.23 (m, 3H), 7.01 (m, 3H), 6.90 (m, 3H).
³¹P{¹H}-NMR (CDCl₃): δ [ppm] = 10.3 (s).

### Beispiel 4: Tris(2-brom-6-pyridyl)fluormethan

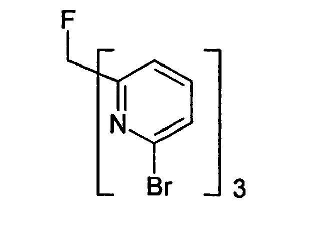

Eine Lösung von 50.0 g (100 mmol) Tris(2-brom-6-pyridyl)methanol in 750 ml Dichlormethan wurde unter gutem Rühren tropfenweise mit 47.3 ml (400 mmol) Diethylaminoschwefeltrifluorid versetzt. Anschließend wurde 30 min. unter Rückfluß erhitzt, dann wurde die Reaktionsmischung auf 5 °C abgekühlt und unter gutem Rühren (stark exotherm!!!) mit 300 ml Wasser und anschließend mit einer Lösung von 64.0 g (1600 mmol) Natriumhydroxid in 600 ml Wasser (stark exotherm!!!) versetzt. Die organische Phase wurde abgetrennt, die wäßrige wurde zweimal mit 200 ml Dichlormethan gewaschen, die vereinigten organischen Phasen wurden über Calciumchlorid getrocknet und anschließend vom Dichlormethan befreit. Der verbliebene rotbraune Kristallbrei wurde in 100 ml Methanol aufgenommen und abfiltriert. Nach waschen mit Methanol wurden die farblosen bis beigen Kristalle im Vakuum getrocknet. Die Ausbeute betrug 47.4 g (91.3 %) bei einer Reinheit von größer 99.0% nach ¹H-NMR.
¹H-NMR (CDCl₃): δ [ppm] = 7.58 (ddd, ³J_{HH} = 7.7 Hz, ³J_{HH} = 7.7 Hz, ⁵J_{FH} = 0.7 Hz, 1 H, H-4,), 7.53 (dd, ³J_{HH} = 7.7 Hz, ⁴J_{HH} = 0.7 Hz, 1 H, H-3), 7.45 (ddd, ³J_{HH} = 7.7 Hz , ⁴J_{HH} = 0.7 Hz, ⁴J_{FH} = 0.9 Hz, 1 H, H-5).
¹⁹F{¹H}-NMR (CDCl₃): δ [ppm] = -146.2 (s).

### Beispiel 5: 2-Brom-4-fluor-1-(tetrahydropyran-2-yloxy)-benzol

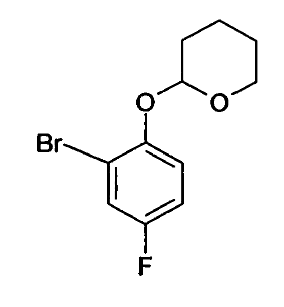

Ein Gemisch von 478.0 ml (5.24 mol) 3,4-Dihydropyran und 750 ml Dichlormethan wurde mit 65.4 g (260 mmol) Pyridinium-p-toluolsulfonat versetzt. Anschließend wurde eine Lösung von 500.0 g (2.62 mmol) 2-Brom-4-fluorphenol in 500 ml Dichlormethan zugetropft. Nach 24 h Rühren wurde die Reaktionsmischung mit einer Lösung von 50 g Kaliumcarbonat in 500 ml Wasser, und dann mit 500 ml gesättigter Kochsalzlösung versetzt. Die organische Phase wurde abgetrennt, über Kaliumcarbonat getrocknet und nach Befreien vom Lösungsmittel von Kaliumcarbonat mittels einer Vigreux-Kolonne (40 cm) fraktioniert destilliert (ca.
1 mbar, Kopftemperatur 79 bis 82 °C). Das Produkt wurde als farbloses niederviskoses Öl erhalten. Die Ausbeute betrug 520.5 g (72.2 %) bei einer Reinheit von größer 98.0% nach ¹H-NMR.
¹H-NMR (CDCl₃): δ [ppm] = 7.27 (dd, ³J_{FH} = 8.0 Hz, ⁴J_{HH} = 3.0 Hz, 1 H, H-3), 7.10 (dd, ³J_{HH} = 9.7 Hz, ⁴J_{FH} = 5.0 Hz, 1 H, H-6), 6.93 (ddd, ³J_{HH} = 9.7 Hz , ³J_{FH} = 8.0 Hz, ⁴J_{HH} = 3.2 Hz, 1 H, H-5), 5.39 (m, 1 H, CH), 3,88 (m, 1 H, CH₂O), 3.59 (m, 1 H, CH₂O), 2.12 - 1.53 (m, 6 H, CH₂).
¹⁹F{¹H}-NMR (CDCl₃): δ [ppm] = -121.1 (s).

### Beispiel 6: 5-Fluor-2-(tetrahydropyran-2-yloxy)-benzolboronsäure

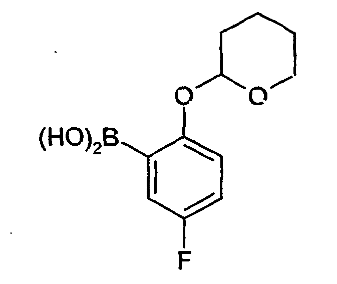

Aus 48.6 g (2.00 mol) Magnesium und 510 g (1.85 mol) 2-Brom-4-fluor-1-(tetrahydropyran-2-yloxy)-benzol in 1250 ml THF wurde ein Grignargreagenz dargestellt. Dieses Grignardreagenz wurde langsam zu einer Mischung von 241.6 ml (2.00 mol) Trimethylborat in 500 ml THF bei - 78 °C zugetropft. Nach vollendeter Zugabe ließ man die Reaktionsmischung auf Raumtemperatur erwärmen und hydrolysierte durch Zugabe von 100 ml gesättigter Kaliumcarbonatlösung und 1000 ml Wasser. Die organische Phase wurde mit gesättigter Kochsalzlösung gewaschen (1 x 500 ml) und anschließend zur Trockene eingeengt. Die Ausbeute betrug 428.2 g (1.78 mol), wobei das Produkt als wachsartiger Feststoff anfiel und wechselnde Anteile an Boronsäureanhydrid und Borinsäuren enthielt und in der folgenden Stufe ohne weitere Reinigung eingesetzt wurde.

### Beispiel 7: Tris(6-(5-fluor-2-hydroxyphenyl)-2-pyridyl)-fluormethan, (PPL-02)

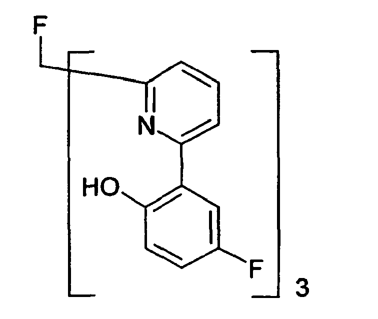

Durchführung der Suzuki-Kupplung analog Beispiel 2, wobei 51.9 g (100 mmol) Tris(2-brom-6-pyridyl)fluormethan (Beispiel 4), 108.0 g (450 mmol) 5-Fluor-2-(tetrahydropyran-2-yloxy)-benzolboronsäure (Beispiel 6), 57.5 g (990 mmol) Kaliumfluorid, 1.35 g (6 mmol) Palladium(II)acetat und 1.8 ml (8 mmol) Tri-tert-butylphosphin in 1000 ml THF zum Einsatz kamen.
Nach 6 h unter Rückfluß wurde die Reaktionsmischung am Rotationsverdampfer vom THF befreit, der breiige Rückstand wurde in 1000 ml Methanol aufgenommen, mit einem Gemisch von 300 ml Wasser und 55 ml 5 N HCl versetzt und anschließend 3 h bei 50°C nachgerührt. Der entstandene Kristallbrei wurde abgesaugt (P3), mit Methanol gewaschen und getrocknet. Umkristallisation aus wenig Chloroform unter Zugabe von Methanol ergab 53.2 g (89.0 %) des Produkts in Form von farblosen Kristallen mit einer Reinheit von größer 99.0 % nach ¹H-NMR.
¹H-NMR (CDCl₃): δ [ppm] = 12.34 (s, 3 H, OH), 7.99 (dd, ³ J_{HH} = 8.4 Hz, ³J_{HH} = 8.4 Hz, 3 H, H-4-Py), 7.86 (d, ³J_{HH} = 8.4 Hz, 3 H, H-5-Py), 7.79 (d, ³J_{HH} = 8.4 Hz, 3 H, H-3-Py), 7.45 (dd, ³J_{HH} = 9.4 Hz, ⁴J_{FH} = 3.0 Hz, 3 H, H-3), 6.95 (ddd, ³J_{HH} = 9.4 Hz , ³J_{FH} = 8.0 Hz, ⁴J _{HH} = 3.0 Hz, 3 H, H-4), 6.76 (dd, ³J_{FH} = 9.0 Hz, ⁴J_{HH} = 3.0 Hz, 3 H, H-6).
¹⁹F{¹H}-NMR (CDCl₃): δ [ppm] = -144.9 (s, 1 F), -125.9 (s, 3 F).

### Beispiel 8: 2-Brom-5-fluor-1-(tetrahydropyran-2-yloxy)-benzol

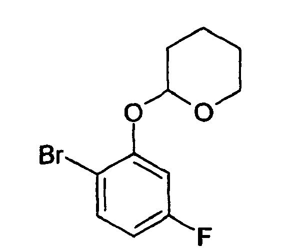

Durchführung analog Beispiel 5. Einsatz von 478.0 ml (5.24 mol) 3,4-Dihydropyran, 65.4 g (260 mmol) Pyridiniuim-p-toluolsulfonat und 500.0 g (2.62 mmol) 2-Brom-5-fluorphenol. Die Ausbeute betrug 562.2 g (78.0 %) bei einer Reinheit von größer 98.0% nach ¹H-NMR.
¹H-NMR (CDCl₃): δ [ppm] = 7.45 (dd, ³J_{HH} = 9.1 Hz, ⁴J_{FH} = 6.4 Hz, 1 H, H-3), 6.92 (dd, ³J_{FH} = 10.7 Hz, ⁴J_{FH} = 2.7 Hz, 1 H, H-6), 6.60 (ddd, ³J_{HH} = 9.1 Hz, ³J_{FH} = 8.7 Hz, ⁴J_{HH} = 2.7 Hz, 1 H, H-4), 5.46 (m, 1 H, CH), 3,84 (m, 1 H, CH₂O), 3.62 (m, 1 H, CH₂O), 2.14 - 1.56 (m, 6 H, CH₂).
¹⁹F{¹H}-NMR (CDCl₃): δ [ppm] = -112.7 (s).

### Beispiel 9: 4-Fluor-2-(tetrahydropyran-2-yloxy)-benzolboronsäure

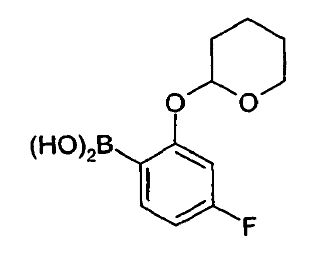

Durchführung analog Beispiel 6. Einsatz von 48.6 g (2.00 mol) Magnesium, 510 g (1.85 mol) 2-Brom-5-fluor-1-(tetrahydropyran-2-yloxy)-benzol und 241.6 ml (2.00 mol) Trimethylborat. Die Ausbeute betrug 434.5 g (1.81 mol), wobei das Produkt als wachsartiger Feststoff anfiel und wechselnde Anteile an Boronsäureanhydriden und Borinsäuren enthielt und in der folgenden Stufe ohne weitere Reinigung eingesetzt wurde.

### Beispiel 10: Tris(6-(4fluor-2-hydroxyphenyl)-2-pyridyl)-fluormethan, (PPL-03)

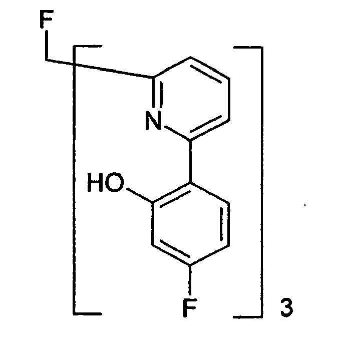

Durchführung analog Beispiel 7. Einsatz von 51.9 g (100 mmol) Tris(2-brom-6-pyridyl)fluormethan (Beispiel 4), 108.0 g (450 mmol) 4-Fluor-2-(tetrahydropyran-2-yloxy)-benzolboronsäure (Beispiel 9), 57.5 g (990 mmol) Kaliumfluorid, 1.35 g (6 mmol) Palladium(II)acetat und 1.8 ml (8 mmol) Tri-tert-butylphosphin. Die Ausbeute betrug 56.9 g (95.5 %) des Produkts in Form von farblosen Kristallen mit einer Reinheit von größer 99.0 % nach ¹H-NMR.
¹H-NMR (CDCl₃): δ [ppm] = 13.01 (s, 3 H, OH), 7.96 (dd, ³J_{HH} = 8.2 Hz, ³J_{HH} = 8.2 Hz, 3 H, H-4-Py), 7.86 (d, ³J_{HH} = 8.2 Hz, 3 H, H-5-Py), 7.75 (dd, ³J_{HH} = 9.4 Hz, ⁴J_{FH} = 6.4 Hz, 3 H, H-6), 7.52 (d, ³J_{HH} = 8.2 Hz, 3 H, H-3-Py), 6.59 (ddd, ³J_{HH} = 9.4 Hz, ³J_{FH} = 8.0 Hz, ⁴J_{HH} = 2.7 Hz, 3 H, H-5), 6.51 (dd, ³J_{FH} = 10.7 Hz, ⁴J_{HH} = 2.7 Hz, 3 H, H-3).
¹⁹F{¹H}-NMR (CDCl₃): δ [ppm] = -144.7 (s, 1 F), -108.6 (s, 3 F).

### Beispiel 11: Mono[tris(6-(2-oxyphenyl)-2-pyridyl)phosphinoxido]aluminium(III); Al-PPL-01

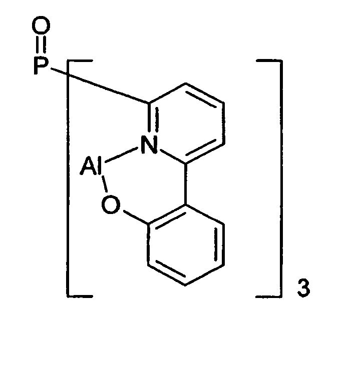

Eine Lösung von 5.58 g (10 mmol) Tris(6-(2-hydroxyphenyl)-2-pyridyl)phosphinoxid (Beispiel 3) in 100 ml Toluol wurde bei 80 °C während 30 min. mit einer Lösung von 2.04 g (10 mmol) Tris(*iso*-propanolato)aluminium(III) in 50 ml Toluol versetzt. Die Reaktionsmischung wurde weitere 3 h unter Rückfluß erhitzt. Nach Erkalten auf Raumtemperatur wurde der farblose Niederschlag abgesaugt, mit Toluol (1 x 25 ml) gewaschen und getrocknet. Wiederholte Umkristallisation aus DMSO ergab 5.03 g (86.5 %) des Komplexes bei einer Reinheit von 99.8 % nach HPLC.
MS (FAB): m/e = 582.

### Beispiel 12: Mono[tris(6-(2-oxyphenyl)-2-pyridyl)phosphinoxido]lanthan(III); La-PPL-01

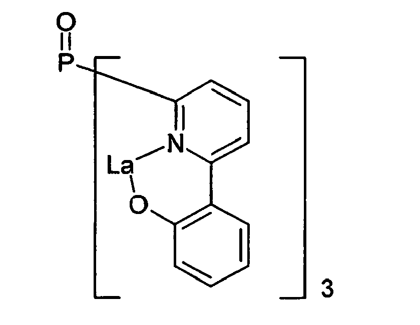

Eine Lösung von 5.58 g (10 mmol) Tris(6-(2-hydroxyphenyl)-2-pyridyl)phosphinoxid (Beispiel 3) in 100 ml Toluol wurde bei 80 °C während 30 min. mit 36.4 ml (10 mmol) einer 10 Gew.-%igen Lösung von Tris(2-methoxy-ethanolato)lanthan(III) in 2-Methoxyethanol versetzt. Die Reaktionsmischung wurde weitere 3 h unter Rückfluß erhitzt. Nach Erkalten auf Raumtemperatur wurde der farblose Niederschlag abgesaugt, mit Toluol (1 x 25 ml) gewaschen und getrocknet. Wiederholte Umkristallisation aus DMSO ergab 5.68 g (81.7 %) des Komplexes bei einer Reinheit von 99.8 % nach HPLC.
MS (FAB): m/e = 693.

### Beispiel 13: Mono[tris(6-(5-fluor-2-oxyphenyl)-2-pyridyl)fluormethanato] aluminium(III); Al-PPL-02

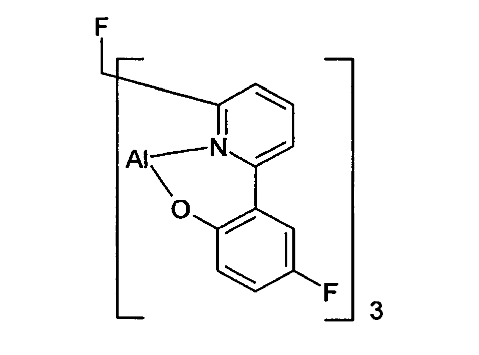

Eine Lösung von 5.96 g (10 mmol) Tris(6-(5-fluor-2-hydroxyphenyl)-2-pyridyl)-fluormethan (Beispiel 7) in 200 ml THF wurde zunächst mit 19.4 ml (240 mmol) Pyridin und dann tropfenweise bei Raumtemperatur während 30 min. mit einer Lösung von 20 ml einer 0.5 N Aluminiumchlorid-Lösung in Ethanol versetzt. Die Reaktionsmischung wurde weitere 3 h unter Rückfluß erhitzt. Nach Erkalten auf Raumtemperatur wurde der farblose Niederschlag abgesaugt, mit THF (3 x 50 ml) und Ethanol (3 x 50 ml) gewaschen und dann getrocknet. Wiederholte Umkristallisation aus DMSO (200 ml) ergab 5.59 g (90.3 %) des fahlgelben Komplexes bei einer Reinheit von 99.9 % nach ¹H-NMR.
¹H-NMR (DMSO-d6): δ [ppm] = 8.23 (dd, ³J_{HH} = 8.0 Hz, ³J_{HH} = 8.0 Hz, 3 H, H-4-Py), 8.17 (d, ³J_{HH} = 8.0 Hz, 3 H, H-5-Py), 7.99 (dd, ³J_{HH} = 8.0 Hz, ⁴J_{FH} = 3.4 Hz, 3 H, H-3-Py), 7.69 (dd, ³J_{HH} = 9.0 Hz, ⁴J_{FH} = 3.0 Hz, 3 H, H-3), 7.03 (ddd, ³J_{HH} = 9.0 Hz , ³J_{FH} = 9.0 Hz, ⁴J_{HH} = 3.4 Hz, 3 H, H-4), 6.19 (dd, ³J_{FH} = 9.0 Hz, ⁴J_{HH} = 3.4 Hz, 3 H, H-6).
¹⁹F{¹H}-NMR (DMSO-d6): δ [ppm] = -177.5 (s, 1 F), -128.6 (s, 3 F).
T_{g}: 178 °C.

### Beispiel 14: Mono[tris(6-(5-fluor-2-oxyphenyl)-2-pyridyl)fluormethanato] eisen(III); Fe-PPL2

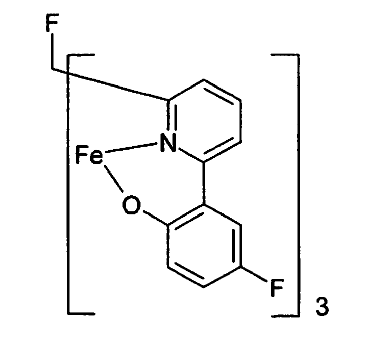

Durchführung analog Beisiel 13. Einsatz von 5.96 g (10 mmol) Tris(6-(5-fluor-2-hydroxyphenyl)-2-pyridyl)-fluormethan (Beispiel 10) und 20 ml einer 0.5 N Eisen(III)chlorid x 6 H₂O-Lösung in Ethanol. Wiederholte Umkristallisation aus DMSO (200 ml) unter Zugabe von 100 ml Ethanol nach Abkühlen der Lösung auf 120 °C ergab 5.39 g (83.1 %) des schwarzen Komplexes.
MS (FAB): m/e = 648.

### Beispiel 15: Mono[tris(6-(4-fluor-2-oxyphenyl)-2-pyridyl)fluormethanato] aluminium(III); Al-PPL3

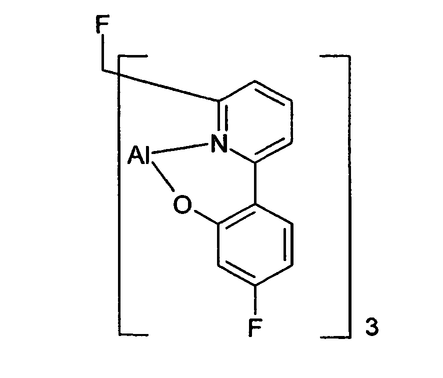

Durchführung analog Beispiel 13. Einsatz von 5.96 g (10 mmol) Tris(6-(4-fluor-2-hydroxyphenyl)-2-pyridyl)-fluormethan (Beispiel 10) und 20 ml einer 0.5 N Aluminiumchlorid-Lösung in Ethanol. Wiederholte Umkristallisation aus DMSO (200 ml) ergab 5.32 g (86.0 %) des fahlgelben Komplexes bei einer Reinheit von 99.9 % nach ¹H-NMR.
¹H-NMR (DMSO-d6): δ [ppm] = 8.24 (dd, ³J_{HH} = 8.0 Hz, ³J_{HH} = 8.0 Hz, 3 H, H-4-Py), 8.15 (d, ³J_{HH} = 8.0 Hz, 3 H, H-5-Py), 7.97 (dd, ³J_{HH} = 8.0 Hz, ⁴J_{FH} = 3.4 Hz 3 H, H-3-Py), 7.92 (dd, ³J_{HH} = 9.0 Hz, ⁴J_{FH} = 7.0 Hz, 3 H, H-6), 6.53 (ddd, ³J_{HH} = 8.7 Hz , ³J_{FH} = 8.7 Hz, ⁴J_{HH} = 2.7 Hz, 3 H, H-5), 5.92 (dd, ³J_{FH} = 11.4 Hz, ⁴J_{HH} = 2.7 Hz, 3 H, H-3).
¹⁹F{¹H}-NMR (DMSO-d6): δ [ppm] = -178.4 (s, 1 F), -109.5 (s, 3 F).
T_{g}: 197 °C.

### Vergleichsexperimente zur Hydrolysestabilität

### Beispiel 16:

In einem Vergleichsexperiment wurde die Hydolysestabilität des polypodalen Aluminiumkomplexes Mono[tris(6-(5-fluor-2-oxyphenyl)-2-pyridyl)fluormethanato] aluminium(III) (Al-PPL-2), gemäß Beispiel 13, mit der des strukturell analogen, aber nicht polypodalen Variante, Tris[5-fluor-2-oxyphenyl)2-pyridylato]aluminium(III), das in der Anmeldung JP 09176629 A2 als OLED-Material beschrieben wird, verglichen. Dazu wurde von beiden Komplexen eine 10 mmolare Lösung in trockenem DMSO-d6 uner Inertgasatmosphäre hergestellt. Diese Lösung wurde mit Hilfe der ¹H- und der ¹⁹F-NMR-Spektroskopie charakterisiert. Anschließend wurden diese Lösungen mit der 1000 molaren Menge Wasser bei Raumtemperatur versetzt und nach 10 min. stehen erneut mit Hilfe der ¹H- und der ¹⁹F-NMR-Spektroskopie charakterisiert. Im Fall des polypodalen Aluminiumkomplexes Mono[tris(6-(5-fluor-2-oxyphenyl)-2-pyridyl)fluormethanato]aluminium(III) (Al-PPL-2), gemäß Beispiel 13, konnte keinerlei Veränderung der NMR festgestellt werden. Im Gegensatz dazu, war im Falle des nicht polypodalen Tris(5-fluor-2-oxyphenyl)2-pyridylato)aluminium(III), eine vollständige Zersetzung des Komplexes zu beobachten, erkennbar am Auftreten der Protonen- und Fluorsignale des nicht koordinierten Liganden. Selbst nach fünfstündigem Erhitzen der oben beschriebenen Hydrolysemischung auf 180 °C war kein Anzeichen einer Zersetzung des polypodalen Aluminiumkomplexes Al-PPL-2, gemäß Beispiel 13, nachzuweisen. Dieses Vergleichsexperiment demonstriert eindringlich die exzellente Hydrolysestabilität der erfindungsgemäßen polypodalen Komplexe.

### Herstellung und Charakterisierung von organischen Elektrolumineszenzvorrichtungen, die erfindungsgemäße Verbindungen enthalten.

Die Herstellung der OLEDs erfolgte nach einem allgemeinen Verfahren, das im Einzelfall auf die jeweiligen Gegebenheiten (z. B. Schichtdickenvariation zur Optimierung der Effizienz bzw. der Farbe) angepaßt wurde. Erfindungsgemäße Elektrolumineszenzvorrichtungen können, wie beispielsweise in DE10330761.3 oder auch DE 10261545.4 beschrieben, dargestellt werden.

### Beispiel 17: Deviceaufbau

Die folgenden Beispiele zeigen die Ergebnisse verschiedener OLEDs, sowohl mit Phosphoreszenzemittern als auch Fluoreszenzemittern, wobei erfindungsgemäße Verbindungen im ersten Fall als Lochblockiermaterialien eingesetzt wurden und BCP und BAlq als Vergleichsmaterialien (siehe Tabelle 1) verwendet wurden. Im zweiten Fall wurde eine erfindungsgemäße Verbindung als Elektronentransport-material und AIQ₃ als entsprechendes Vergleichsmaterial (siehe Tabelle 2) verwendet. Der grundlegende Aufbau; die verwendeten Materialien und Schichtdicken (außer der HBLs) waren zur besseren Vergleichbarkeit identisch.
Gemäß dem o. g. allgemeinen Verfahren wurden phosphoreszierende OLEDs mit folgendem Aufbau erzeugt:

| | |
|---|---|
| PEDOT (HIL) | 60 nm (aus Wasser aufgeschleudert; bezogen als Baytron P von H. C. Starck; Poly-(3,4-ethylendioxy-2,5-thiophen)) |
| NaphDATA (HTL) | 20 nm (aufgedampft; bezogen von SynTec; 4,4',4"-Tris(N-1-naphthyl-N-phenylamino)-triphenylamin) |
| S-TAD (HTL) | 20 nm (aufgedampft; hergestellt nach WO 99/12888; 2,2',7,7'-Tetrakis(diphenylamino)-spirobifluoren) |
| (EML) | Materialien und Schichtdicken: siehe Tabelle 1 oder 2 |
| (HBL) | wenn vorhanden, Materialien und Schichtdicken: siehe Tabelle 1 |
| (ETL) | Materialien und Schichtdicken: siehe Tabelle 1 oder 2 |
| Ba-Al (Kathode) | 3 nm Ba, darauf 150 nm Al. |

Diese noch nicht optimierten OLEDs wurden standardmäßig charakterisiert; hierfür wurden die Elektrolumineszenzspektren, die Effizienz (gemessen in cd/A), die Leistungseffizienz (gemessen in lm/W) in Abhängigkeit der Helligkeit und die Lebensdauer bestimmt. Als Lebensdauer wird die Zeit definiert, nach der die Anfangshelligkeit der OLED bei einer konstanten Stromdichte von 10 mA/cm² auf die Hälfte abgesunken ist.

In Tabelle 1 sind die Ergebnisse der erfindungsgemäßen OLEDs bei Verwendung des Phosphoreszenzemitters Ir(PPy)₃ dotiert zu 10% in CBP (4,4'-Bis(carbazol-9-yl)-biphenyl) und Verwendung des Komplexes Al-PPL2, gemäß Beispiel 13, als Lochblockiermaterial und einem Vergleichsbeispiel (mit BAlq) zusammengestellt. In Tabelle 1 sind lediglich die Lochblockierschicht und die Elektronentransportschicht (Zusammensetzung und Schichtdicke) aufgeführt. Als Elektronentransportmaterial wurde AlQ₃ (Tris(8-hydroxychinolinato)aluminium(III)), bezogen von SynTec, eingesetzt. Die anderen Schichten entsprechen dem oben genannten Aufbau.

In Tabelle 2 sind die Ergebnisse der erfindungsgemäßen OLEDs bei Verwendung eines Fluoreszenzemitters und Verwendung des Komplexes Al-PPL2, gemäß Beispiel 13, als Elektronentransportmaterial und einiger Vergleichsbeispiele (mit dem Elektronentransportmaterial AIQ₃) zusammengefasst. In Tabelle 2 sind lediglich die Emitterschicht und die Elektronentransportschicht (Zusammensetzung und Schichtdicke) aufgeführt. Die anderen Schichten entsprechen dem oben genannten Aufbau.

Die oben bzw. in den Tabellen 1 und 2 verwendeten Abkürzungen entsprechen den folgenden Verbindungen:

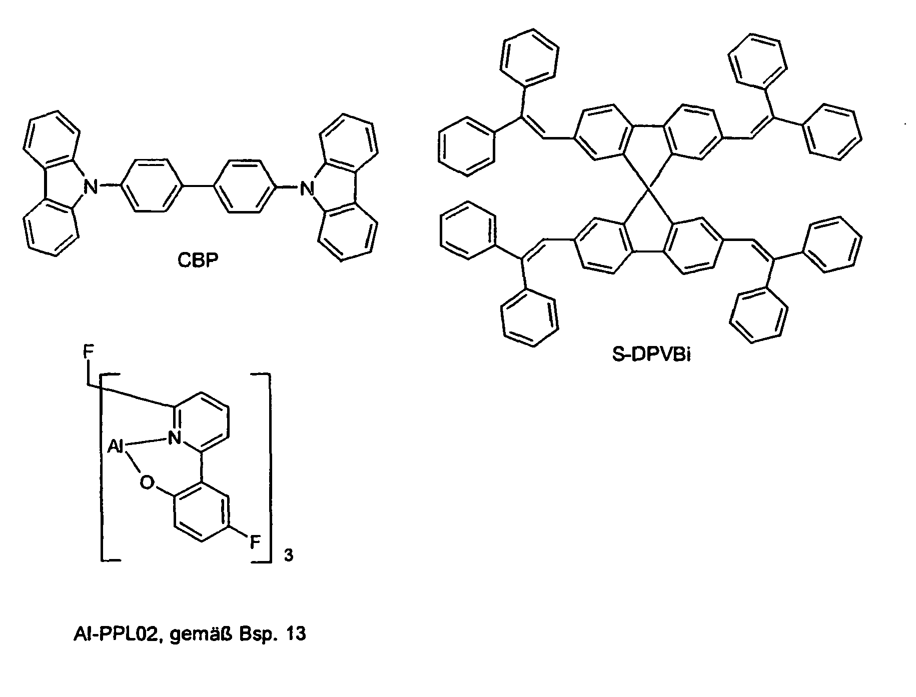

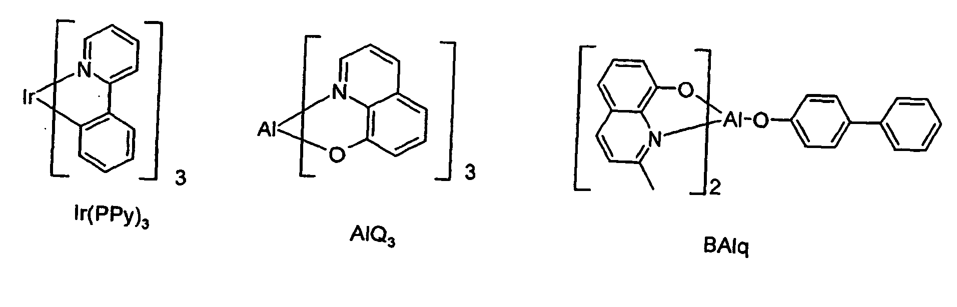

**Tabelle 1:**

| | | | Max. | | Leistungseffizienz | | |
|---|---|---|---|---|---|---|---|
| Beispiel | HBL | ETL | Effizienz | Spannung (V) bei | (Im/W) bei | CIE (x, y) | Lebensdauer (h) |
| | | | (cd/A) | 100 cd/m² | max. Effizienz | | bei 10 mA/cm² |
| **Beispiel T1** | AI-PPL2 | AlQ₃ | 30.0 | 5.1 | 16.5 | 0.32 / 0.62 | 600 |
| | (10 nm) | (20 nm) | | | | | |
| **Beispiel T2** | BAlq | AIQ₃ | 18.3 | 5.1 | 8.5 | 0.32 / 0.62 | 250 |
| (Vergleich) | (10 nm) | (20 nm) | | | | | |
| **Beispiel T3** | Al-PPL2 | --- | 22.9 | 3.2 | 23.2 | 0.32/0.62 | 290 |
| | (20 nm) | | | | | | |
| **Beispiel T4** | BAlq | --- | 16.5 | 5.3 | 8.8 | 0.32/0.62 | 180 |
| (Vergleich) | (20 nm) | | | | | | |

**Tabelle 2:**

| | | | Max. | | Leistungseffizienz | | |
|---|---|---|---|---|---|---|---|
| Beispiel | EML | ETL | Effizienz | Spannung (V) bei | (lm/W bei | CIE (x, y) | Lebensdauer (h) |
| | | | (cd/A) | 100 cd/m² | max. Effizienz | | bei 10 mA/cm² |
| **Beispiel S1** | S-DPVBi | Al-PPL2 | 4.7 | 3.6 | 3.8 | 0.16 / 0.20 | 800 |
| | (30 nm) | (10 nm) | | | | | |
| **Beispiel S2** | S-DPVBi | AlQ₃ | 3.9 | 4.9 | 2.4 | 0.16/0.20 | 640 |
| (Vergleich) | (30 nm) | (10 nm) | | | | | |

Tabelle 1 zeigt, daß die Verwendung von AI-PPL2 in phosphoreszierenden OLEDs als Lochblockiermaterial die Effizienz, insbesondere die Leistungseffizienz der OLED, im Vergleich zu BAlq, deutlich steigert, wobei typischerweise eine Verdopplung der Leistungseffzienz beobachtet wurde. Dabei wurde auch die Lebensdauer deutlich verbessert. Die Beispiele zeigen, daß sogar die Elektronentransportschicht weggelassen werden kann, was eine deutliche Vereinfachung des Deviceaufbaus darstellt.

Bei Verwendung von AI-PPL2 als Elektronentransportmaterial in fluoreszierenden OLEDs wird ebenfalls die Effizienz, Leistungseffizienz und Lebensdauer deutlich verbessert, wie Tabelle 2 zu entnehmen ist.

Zusammenfassend kann gesagt werden, daß phosphoreszierende und fluoreszierende OLEDs, die erfindungesgemäße Verbindungen wie AI-PPL2 als Lochblockiermaterialien oder Elektronentransportmaterialien enthalten, hohe Effizienzen bei gleichzeitig langen Lebensdauern und niedrigen Betriebsspannungen aufweisen, wie man leicht den in den Tabellen 1 und 2 aufgeführten Beispielen entnehmen kann.

Bevorzugt sind ebenfalls erfindungsgemäße Verbindungen (1) bis (41), bei denen für das Symbol Q = O, S gilt.

Bevorzugt sind ebenfalls erfindungsgemäße Verbindungen (1) bis (41), bei denen für das Symbol T = N gilt.

Bevorzugt sind ebenfalls erfindungsgemäße Verbindungen (1) bis (41), bei denen für das Symbol X = CR, N gilt.

Bevorzugt sind ebenfalls erfindungsgemäße Verbindungen (1) bis (41), bei denen für das Symbol Z = B, CH, CR¹, COR¹, CF, CCI, CBr, SiR, N, P, PO, RC(CR₂)₃, RC(CR₂CR₂)₃, cis,cis-1,3,5-Cyclohexyl, RSi(CR₂)₃, RSi(CR₂CR₂)₃, N(CR₂)₃, N(C=O)₃, N(CR₂CR₂)₃ gilt.

Bevorzugt sind ebenfalls erfindungsgemäße Verbindungen (1) bis (41), bei denen für das Symbol Y = O, S gilt.

Bevorzugt sind ebenfalls erfindungsgemäße Verbindungen (1) bis (41), bei denen das Symbol R für H, F, Cl, Br, l, CN, eine geradkettige oder verzweigte oder cyclische Alkyl- oder Alkoxygruppe mit 1 bis 6 C-Atomen oder eine Aryl- oder Heteroarylgruppe mit 3 bis 8 C-Atomen steht, die durch einen oder mehrere, nicht aromatische Reste R substituiert sein kann, wobei mehrere Substituenten R, sowohl am selben Ring als auch an den beiden unterschiedlichen Ringen zusammen wiederum ein weiteres mono- oder polycyclisches, aliphatisches oder aromatisches Ringsystem aufspannen können.

Sofern in den Verbindungen (1) bis (41) durch die Reste R Ringsysteme aufgespannt werden, sind diese bevorzugt Benzol, 1- bzw. 2-Naphthalin, 1-, 2- bzw. 9-Anthracen, 2-, 3- bzw. 4-Pyridin, 2-, 4- bzw. 5-Pyrimidin, 2-Pyrazin, 3- bzw. 4-Pyridazin, 2-, 3-, 4-, 5-, 6-, 7- bzw. 8-Chinolin, 2- bzw. 3-Pyrrol, 3-, 4- bzw. 5-Pyrazol, 2-, 4- bzw. 5-Imidazol, 2- bzw. 3-Thiophen, 2- bzw. 3-Selenophen, 2- bzw. 3-Furan, 2-(1,3,4-Oxadiazol), Indol oder Carbazol.

Ebenfalls Gegenstand der vorliegenden Erfindung sind die polypodalen Liganden gemäß Verbindungen (42) bis (82), gemäß Schema 5:

| | | |
|---|---|---|
| 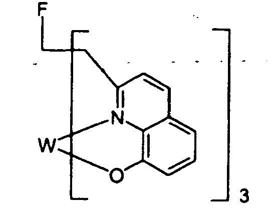 | 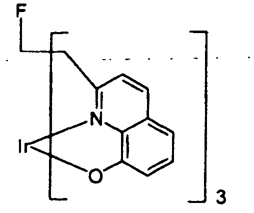 | 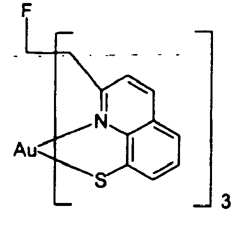 |
| Beispiel 100 | Beispiel 101 | Beispiel 102 |

Die oben beschriebenen erfindungsgemäßen Verbindungen - z.B. Verbindungen gemäß den Beispielen 7, 14, 26, 27, 37, 38, 39 und 41 - können beispielsweise als Co-Monomere zur Erzeugung entsprechender konjugierter, teilkonjugierter oder auch nicht-konjugierter Polymere oder auch als Kern von Dendrimeren - z.B. Verbindungen gemäß den Beispielen 14 und 26 - Verwendung finden. Die entsprechende Einpolymerisation erfolgt dabei bevorzugt über die Halogenfunktionalität.
So können sie u. a. in lösliche Polyfluorene (z. B. gemäß EP-A-842208 oder WO 00/22026), Poly-spirobifluorene (z. B. gemäß EP-A- 707020 oder EP-A-894107), Poly-para-phenylene (z. B. gemäß WO 92/18552), Poly-carbazole (z.B. gemäß den Anmeldungen DE 10304819.7 und DE 10328627.6), Polyvinylcarbazole oder auch Polythiophene (z. B. gemäß EP-A-1028136), oder auch Copolymere aus mehreren dieser Einheiten, einpolymerisiert werden.

Weiterer Gegenstand der Erfindung sind somit konjugierte, teilkonjugierte und nicht-konjugierte Polymere oder Dendrimere enthaltend eine oder mehrere Verbindungen der Formel (1) bis (41), wobei ein oder mehrere der oben definierten R eine Bindung zum Polymer oder Dendrimer darstellt.

Weiterhin können die erfindungsgemäßen Metallkomplexe natürlich auch weiter funktionalisiert werden und so zu *erweiterten Metallkomplexen* umgesetzt werden. Hier ist als Beispiel die Funktionalisierung mit Arylboronsäuren gem. SUZUKI oder mit Aminen gem. HARTWIG-BUCHWALD zu nennen.

Die oben beschriebenen erfindungsgemäßen Verbindungen, Polymere, Dendrimere oder wie oben beschrieben weiter funktionalisierten Verbindungen finden Verwendung als aktive Komponenten in elektronischen Bauteilen, wie z. B. Organischen Leuchtdioden (OLEDs), Organischen Integrierten Schaltungen (O-ICs), Organischen Feld-Effekt-Transistoren (OFETs), Organischen Dünnfilmtransistoren (OTFTs), Organischen Solarzellen (Q-SCs) oder auch Organische Laserdioden (O-Laser).

Aktive Komponenten sind beispielsweise Ladungsinjektions- oder Ladungstransportmaterialien, Ladungsblockiermaterialien und Emissionsmaterialien. Für diese Funktion zeigen die erfindungsgemäßen Verbindungen besonders gute Eigenschaften, wie einerseits vorne schon erläutert und andererseits im folgenden noch näher ausgeführt wird.

Gegenstand der Erfindung ist also weiterhin die Verwendung dieser Verbindungen in elektronischen Bauteilen.

Weiterhin Gegenstand der Erfindung sind elektronische Bauteile, wie z. B. Organische Integrierte Schaltungen (O-ICs), Organische Feld-Effekt-Transistoren (OFETs), Organische Dünnfilmtransistoren (OTFTs), Organische Solarzellen (O-SCs) oder auch Organische Laserdioden (O-Laser), insbesondere aber organische Leuchtdioden (OLEDs), die ein oder mehrere der erfindungsgemäßen Verbindungen, Polymere oder Dendrimere enthalten.

Die vorliegende Erfindung wird durch die folgenden Beispiele für Ladungstranport- bzw. Lochblockermaterialien näher erläutert, ohne sie darauf beschränken zu wollen. Der Fachmann kann aus den Schilderungen ohne erfinderisches Zutun weitere erfindungsgemäße Komplexe, wie beispielsweise Emissionsmaterialien, herstellen bzw. das erfindungsgemäße Verfahren anwenden.

### Beispiele:

### Synthese von homoleptischen Aluminium-, Eisen- und Lanthan-Chelatkomplexen mit hexapodalen Liganden:

Die nachfolgenden Synthesen wurden - sofern nicht anders angegeben - unter einer Schutzgasatmosphäre in getrockneten Lösungsmitteln durchgeführt. Die Edukte wurden von ALDRICH bzw. ABCR [2-Methoxybenzolboronsäure, 2-Brom-4-fluorphenol, 2-Brom-5-fluorphenol, Kaliumfluorid (sprühgetrocknet), Diethylaminoschwefeltrifluorid (DAST), Tri-*tert*-butylphosphin, Palladium(II)acetat, Pyridiniumhydrochlorid, Aluminium-tri-*iso*-propylat, 10 Gew.%ige Lösung von Tris(2-methoxyethanolato)lanthan(III) in 2-Methoxyethanol] bezogen. Tris(2-brom-6-benachbarte CH₂-Gruppen durch -R¹C=CR¹-, -C≡C-, Si(R¹)₂, Ge(R¹)₂, Sn(R¹)₂, C=O, C=S, C=Se, C=NR¹, -O-, -S-, -NR¹- oder -CONR¹-ersetzt sein können und wobei ein oder mehrere H-Atome durch F ersetzt sein können, oder eine Aryl- oder Heteroarylgruppe mit 1 bis 14 C-Atomen, die durch einen oder mehrere, nicht aromatische Reste R substituiert sein kann, wobei mehrere Substituenten R, sowohl am selben Ring als auch an den beiden unterschiedlichen Ringen zusammen wiederum ein weiteres mono- oder polycyclisches, aliphatisches oder aromatisches Ringsystem aufspannen können;
- R¹: sind gleich oder verschieden bei jedem Auftreten H oder ein aliphatischer oder aromatischer Kohlenwasserstoffrest mit 1 bis 20 C-Atomen;
- c: ist bei jedem Auftreten gleich oder verschieden 0 oder 1.
8. Metallkomplexe gemäß einem oder mehreren der Ansprüche 1 bis 6, ausgewählt aus den Verbindungen (9) bis (12), 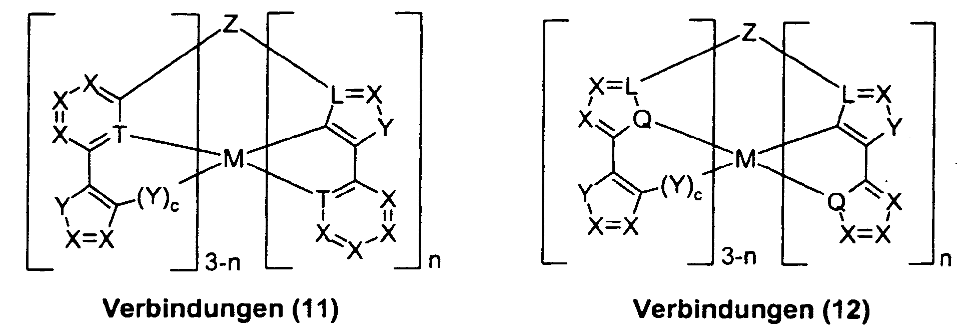 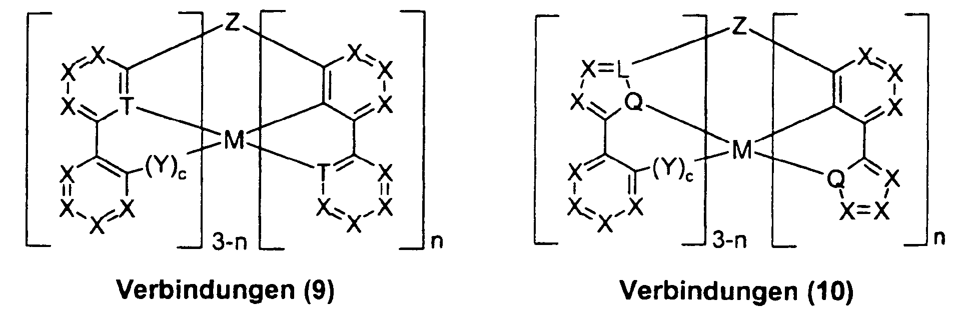 wobei die Symbole und Indizes M, L, Q, T, X, Y, Z, R, R¹, und c die Bedeutungen wie in Anspruch 7 haben, und wobei:
- n: 1 oder 2 ist.

## Patentansprüche

1. Verbindungen gemäß der Struktur 1, 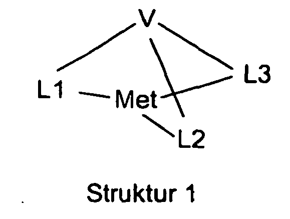 enthaltend mindestens ein Metall Met, koordiniert an einen polypodalen Liganden Lig gemäß Struktur 2, 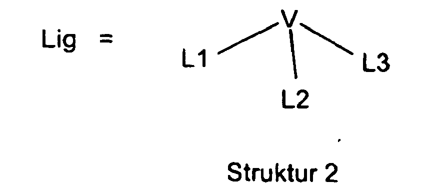 wobei V eine Verbrückungseinheit ist, **dadurch gekennzeichnet, daß** sie 1 bis 80 Atome enthält und die drei Teilliganden L1, L2 und L3, die gleich oder verschieden bei jedem Auftreten sein können, kovalent miteinander verbindet, und wobei die drei Teilliganden L1, L2 und L3 der Struktur 3 genügen, 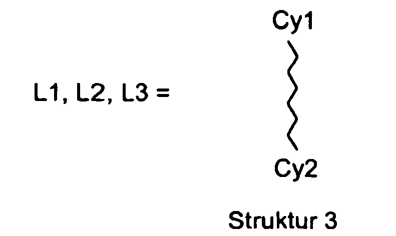 wobei Cy1 und Cy2, gleich oder verschieden bei jedem Auftreten, substituierten oder unsubstituierten, gesättigten, ungesättigten oder aromatischen Homo- oder Heterocyclen bzw. Homo- oder Heteroteilcyclen eines kondensierten Systems entsprechen, die jeweils über ein Ringatom oder über ein exocyclisch an den Homo- oder Heterocyclus gebundenes Atom ionisch, kovalent oder koordinativ an das Metall gebunden sind,
wobei die Verbindungen der Struktur 1 nicht geladen, d. h. nach außen elektrisch neutral, sind.

2. Verbindungen gemäß Anspruch 1, **dadurch gekennzeichnet, daß** L1 = L2 = L3 ist.

3. Verbindungen gemäß Anspruch 1, **dadurch gekennzeichnet, daß** L1 # L2 ist.

4. Verbindungen gemäß einem oder mehreren der Ansprüche 1 bis 3, **dadurch gekennzeichnet, daß** die Verknüpfungseinheit V als verknüpfendes Atom ein Element der 3., 4. oder 5. Hauptgruppe oder einen 3- bis 6-gliedrigen Homo- oder Heterocyclus enthält.

5. Verbindungen gemäß einem oder mehreren der Ansprüche 1 bis 4, **dadurch gekennzeichnet, daß** der polypodale Ligand Lig gemäß Struktur 4 eine *faciale* Koordinationsgeometrie am Metall Met erzeugt.

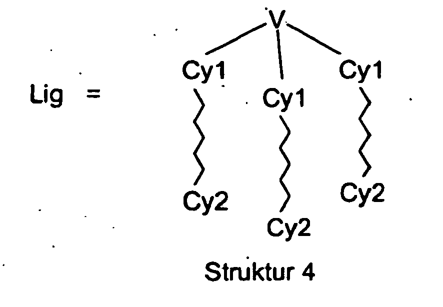

6. Verbindungen gemäß einem oder mehreren der Ansprüche 1 bis 4, **dadurch gekennzeichnet, daß** der polypodale Ligand Lig gemäß Struktur 5 eine *meridionale* Koordinationsgeometrie am Metall Met erzeugt.

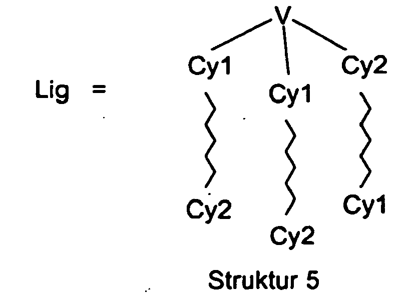

7. Metallkomplexe gemäß einem oder mehreren der Ansprüche 1 bis 6, ausgewählt aus den Verbindungen (1) bis (8),
benachbarte CH₂-Gruppen durch -R¹C=CR¹-, -C≡C-, Si(R¹)₂, Ge(R¹)₂, Sn(R¹)₂, C=O, C=S, C=Se, C=NR¹, -O-, -S-, -NR¹- oder-CONR¹-ersetzt sein können und wobei ein oder mehrere H-Atome durch F ersetzt sein können, oder eine Aryl- oder Heteroarylgruppe mit 1 bis 14 C-Atomen, die durch einen oder mehrere, nicht aromatische Reste R substituiert sein kann, wobei mehrere Substituenten R, sowohl am selben Ring als auch an den beiden unterschiedlichen Ringen zusammen wiederum ein weiteres mono- oder polycyclisches, aliphatisches oder aromatisches Ringsystem aufspannen können;
R¹ sind gleich oder verschieden bei jedem Auftreten H oder ein aliphatischer oder aromatischer Kohlenwasserstoffrest mit 1 bis 20 C-Atomen;
c ist bei jedem Auftreten gleich oder verschieden 0 oder 1.

8. Metallkomplexe gemäß einem oder mehreren der Ansprüche 1 bis 6, ausgewählt aus den Verbindungen (9) bis (12), 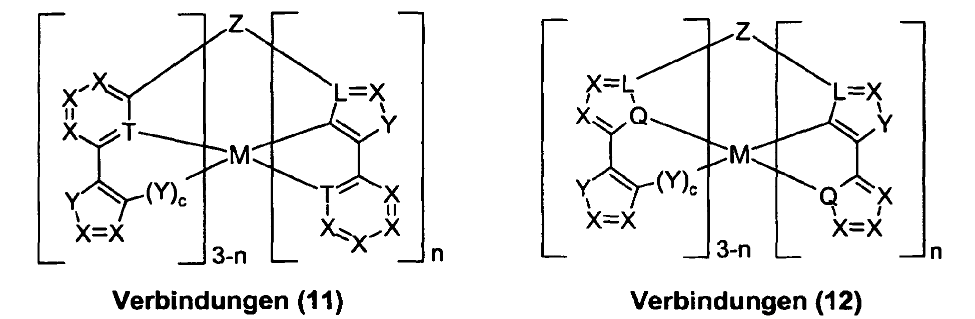 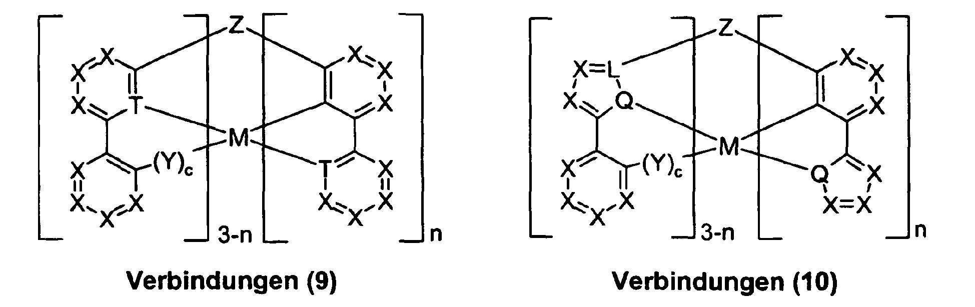 wobei die Symbole und Indizes M, L, Q, T, X, Y, Z, R, R¹, und c die Bedeutungen wie in Anspruch 7 haben, und wobei:
n 1 oder 2 ist.

9. Metallkomplexe gemäß einem oder meheren der Ansprüche 1 bis 6, ausgewählt aus den Verbindungen (13) bis (30), 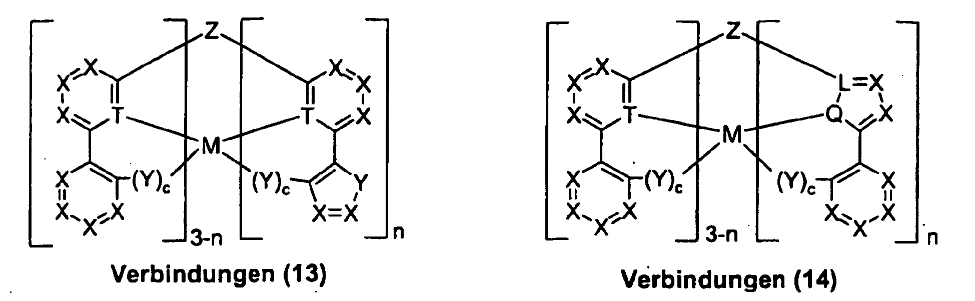 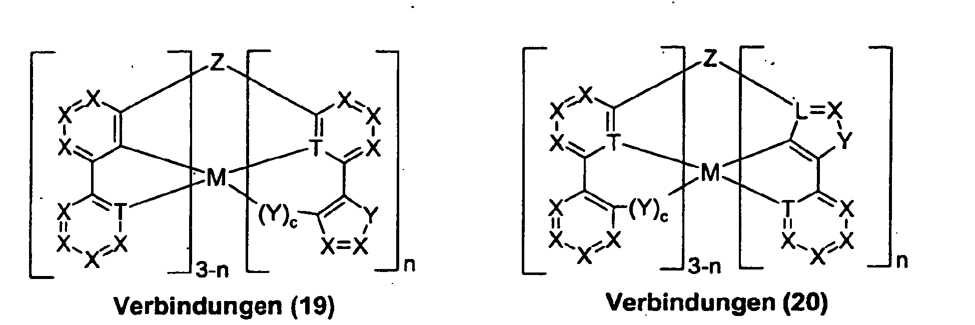 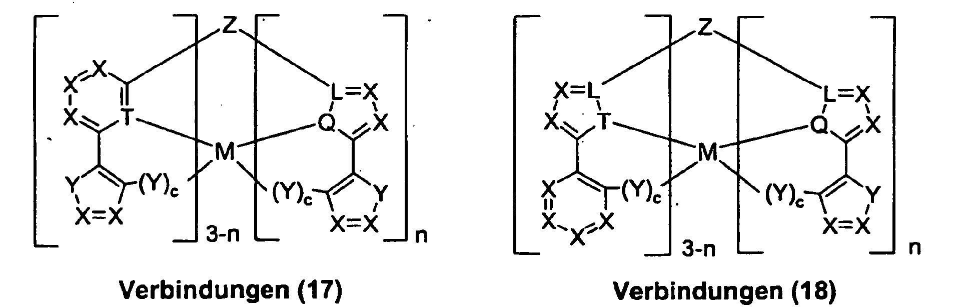 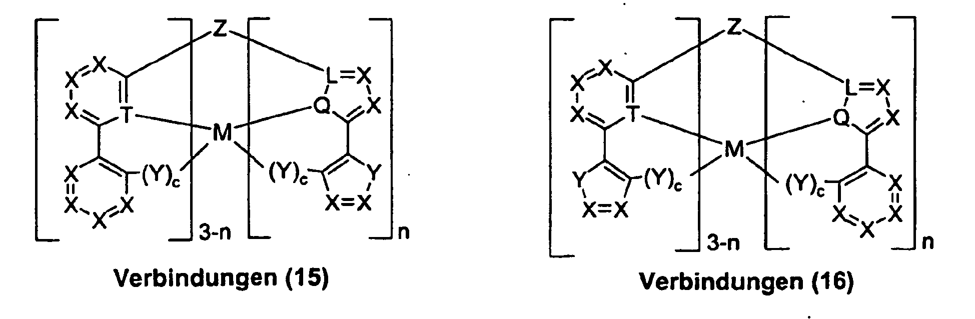 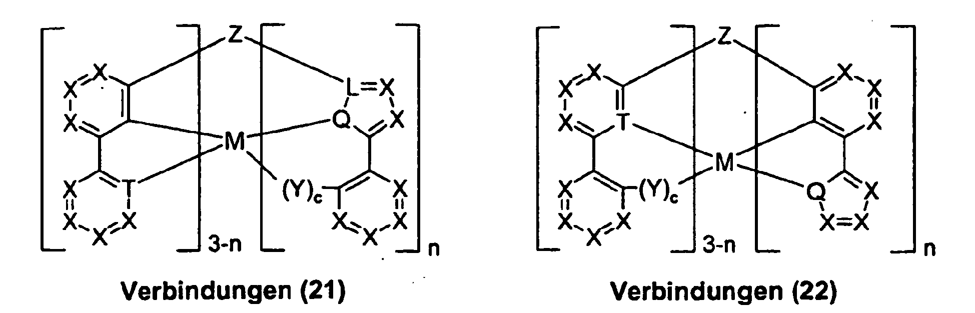 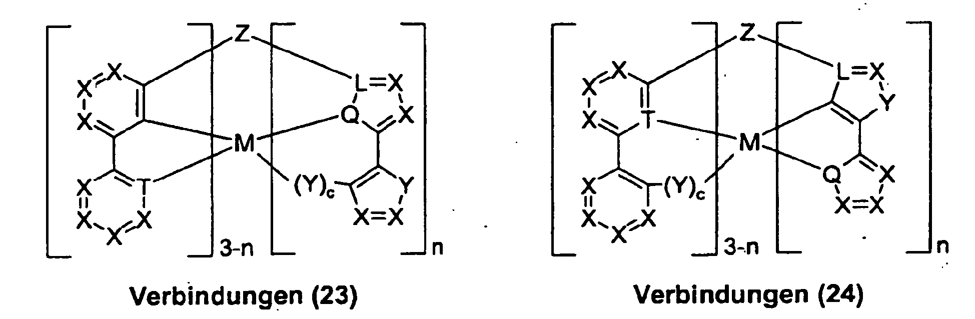 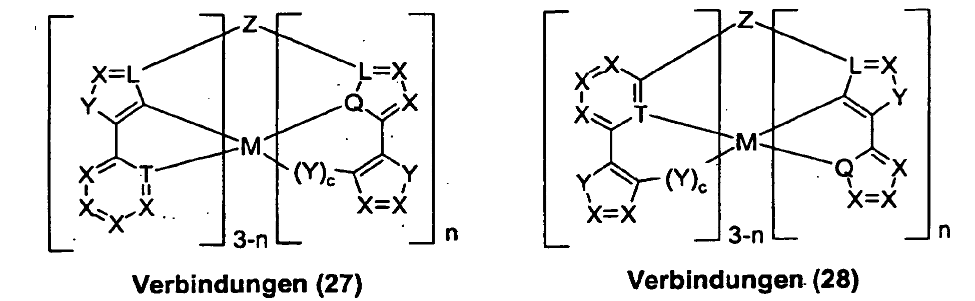 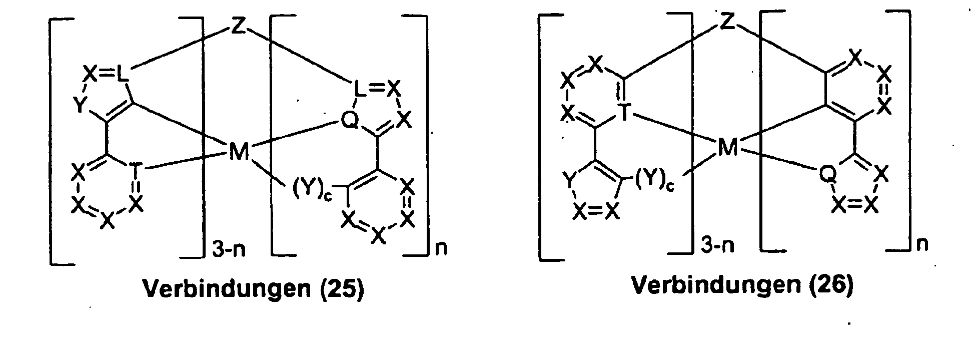 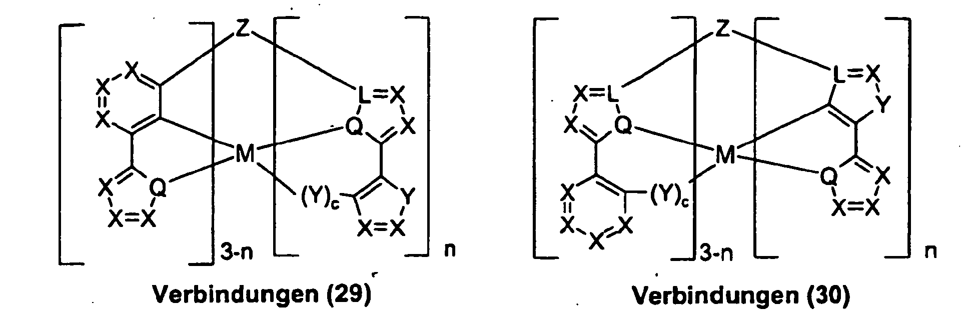 wobei die Symbole und Indizes M, L, Q, T, X, Y, Z, R, R¹, c und n die Bedeutungen wie in Anspruch 7 und 8 haben.

10. Metallkomplexe gemäß einem oder mehreren der Ansprüche 1 bis 6, ausgewählt aus den Verbindungen (31) bis (41), 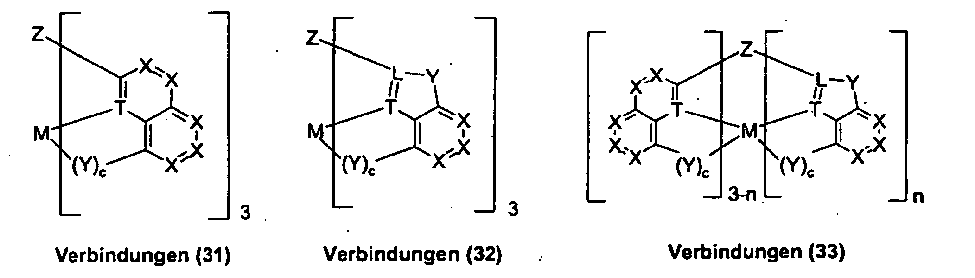 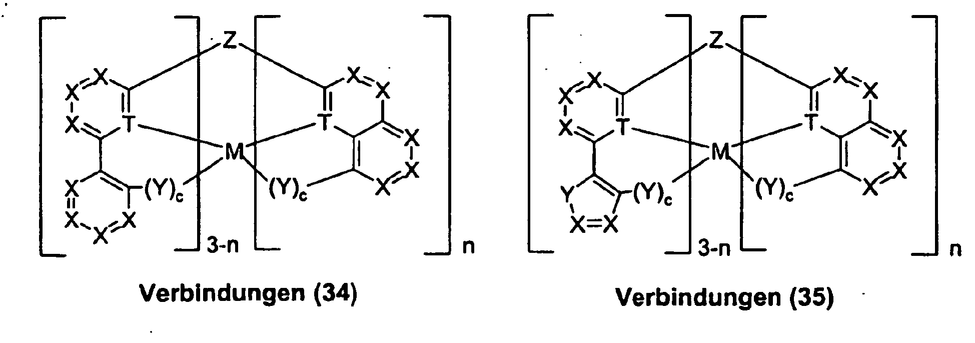 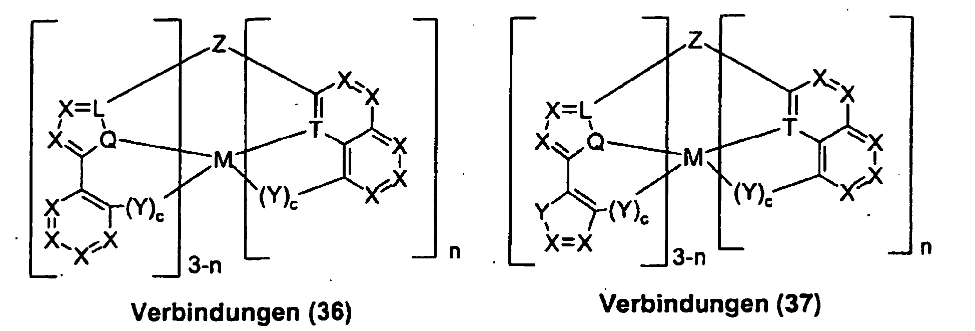 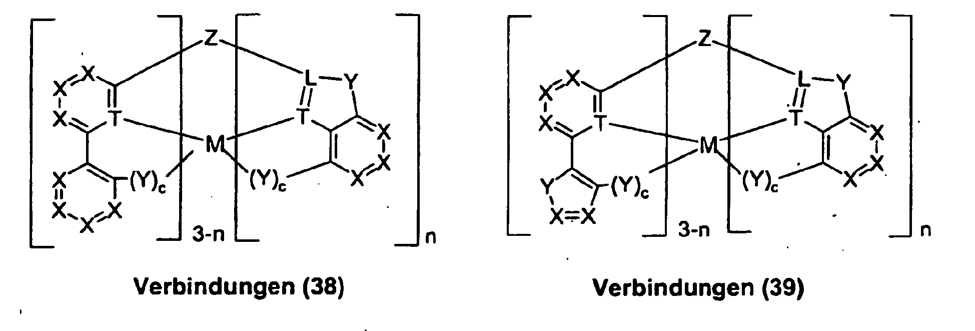 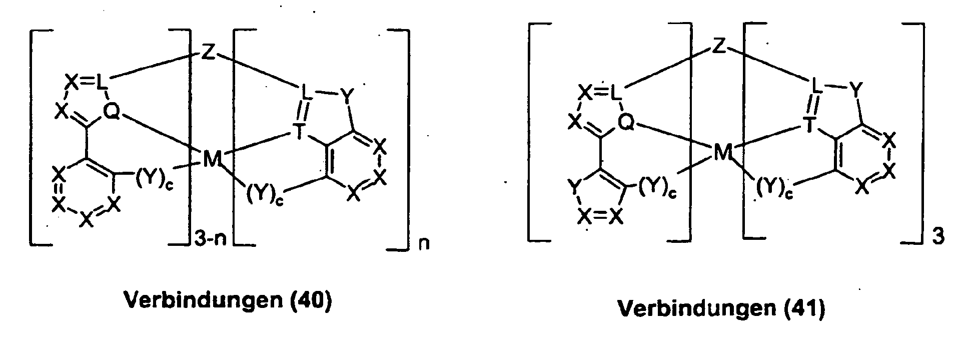 wobei die Symbole und Indizes M, L, Q, T, X, Y, Z, R, R¹, c und n die Bedeutungen wie in Anspruch 7 und 8 haben.

11. Verbindungen (42) bis (82), 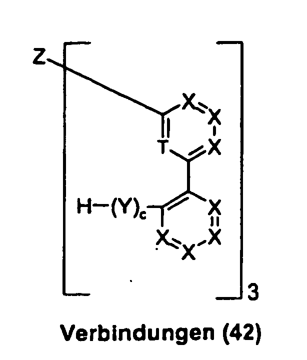 wobei die Symbole und Indizes T, Y, Z, R, R¹, c und n die Bedeutungen wie in Anspruch 7 und 8 haben,
X ist gleich oder verschieden bei jedem Auftreten CR oder P; 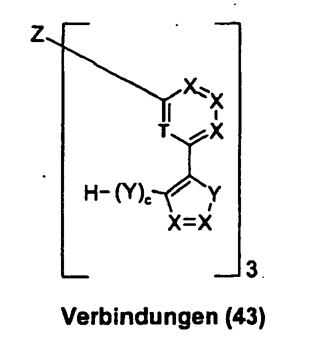 wobei die Symbole und Indizes T, X, Y, Z, R, R¹, c und n die Bedeutungen wie in Anspruch 7 und 8 haben. 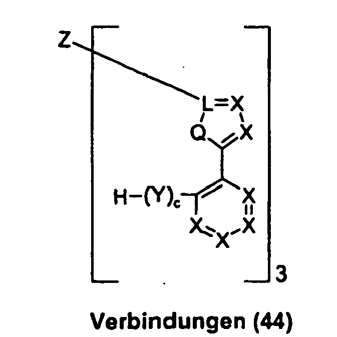 wobei die Symbole und Indizes Q, T, X, Y, Z, R, R¹, c und n die Bedeutungen wie in Anspruch 7 und 8 haben,
L ist gleich oder verschieden bei jedem Auftreten C oder P; 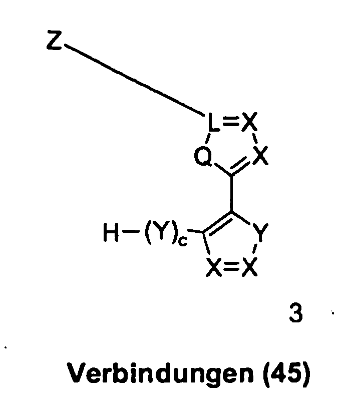 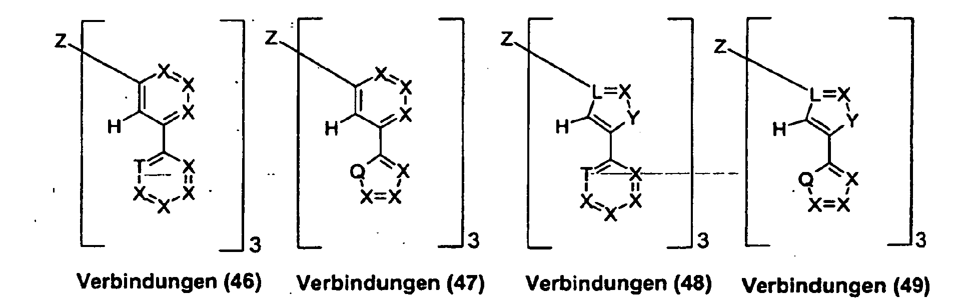 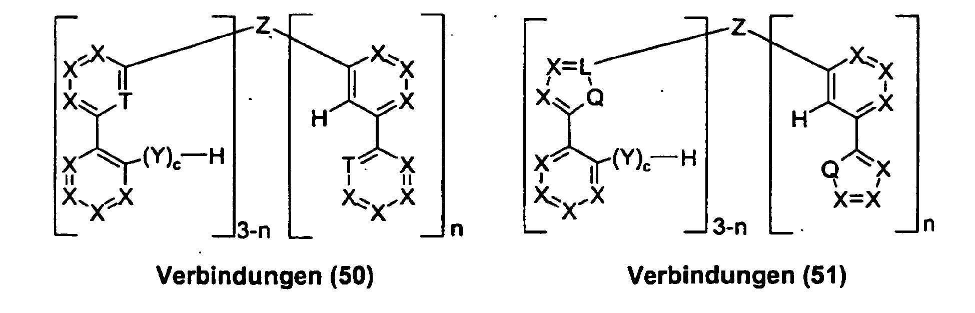 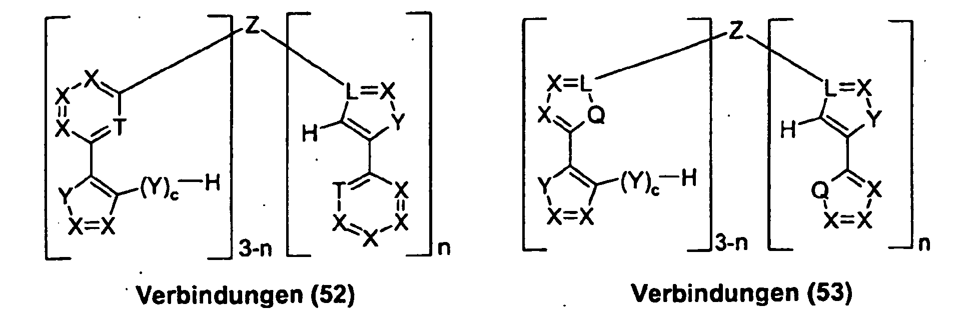 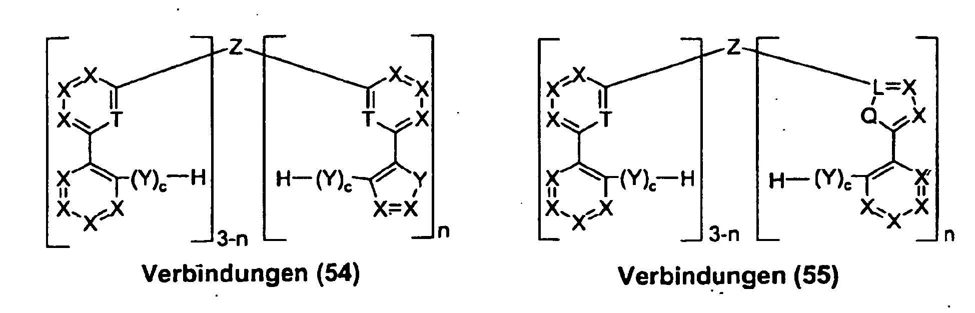 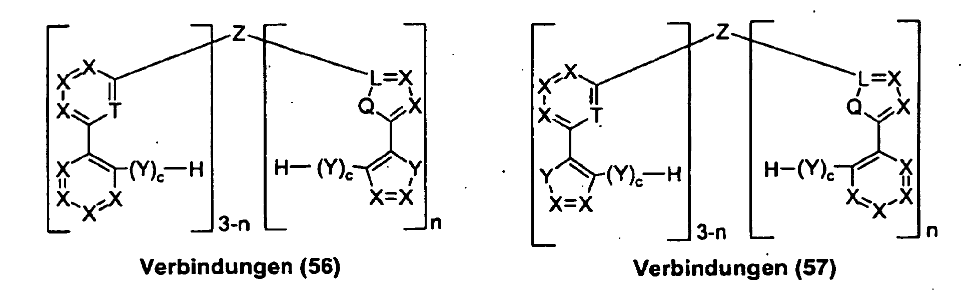 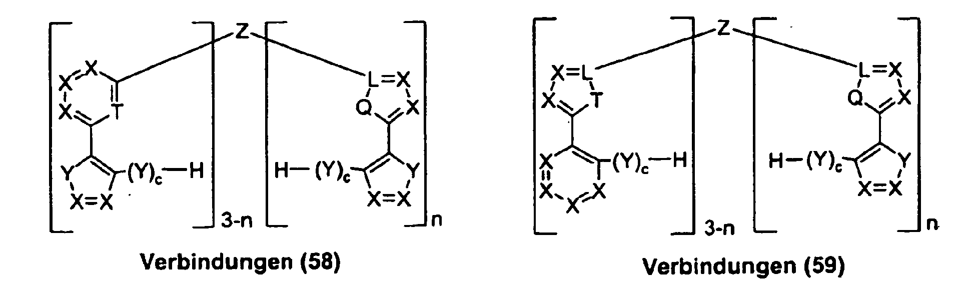 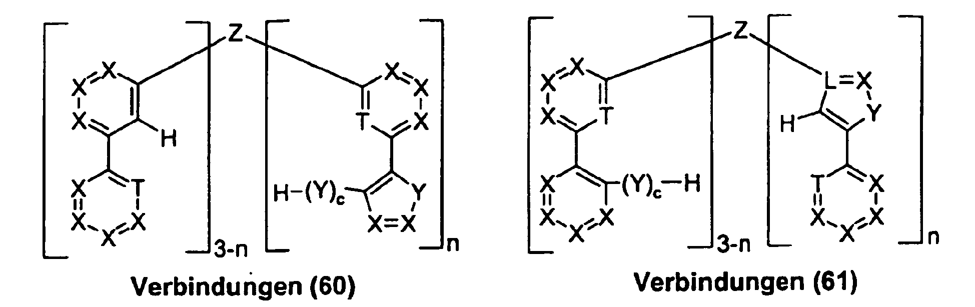 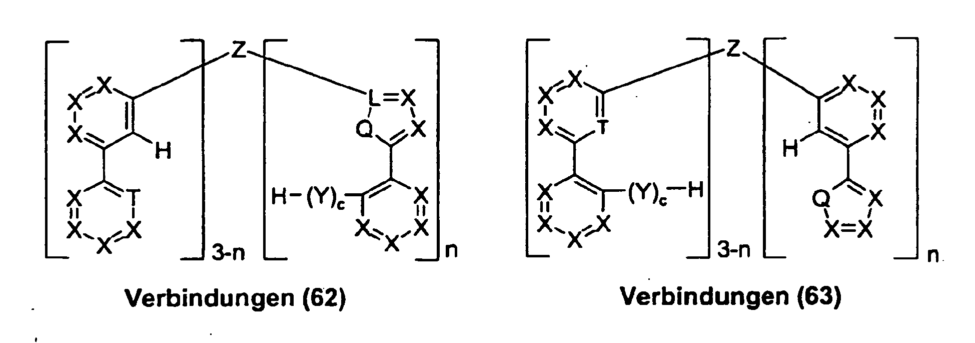 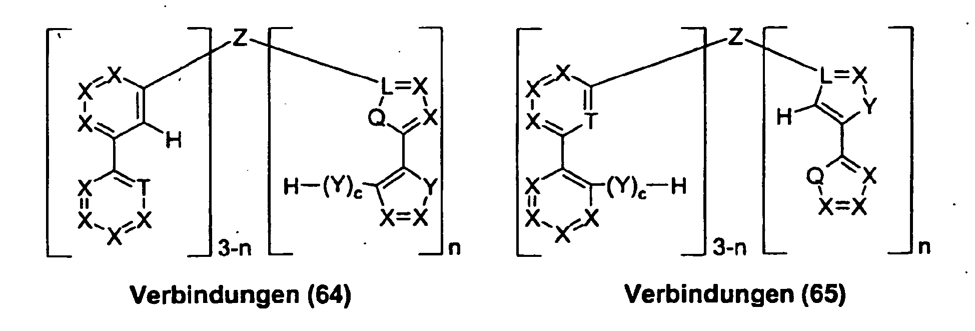 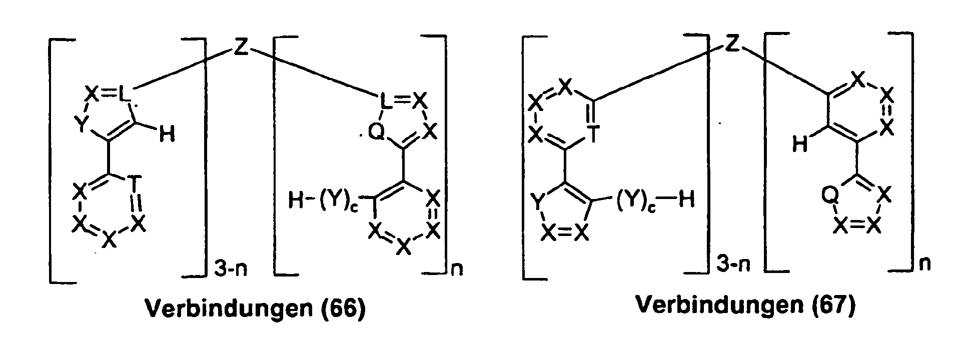 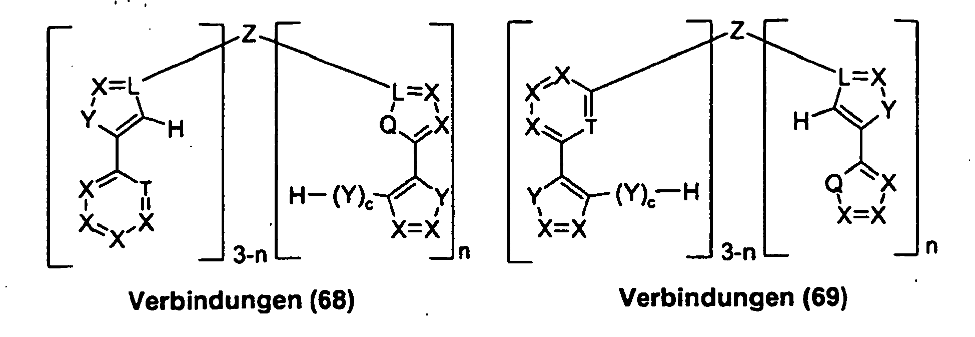 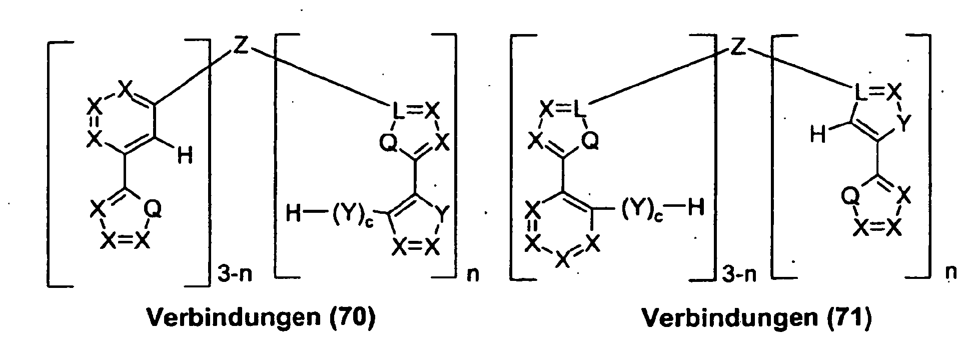 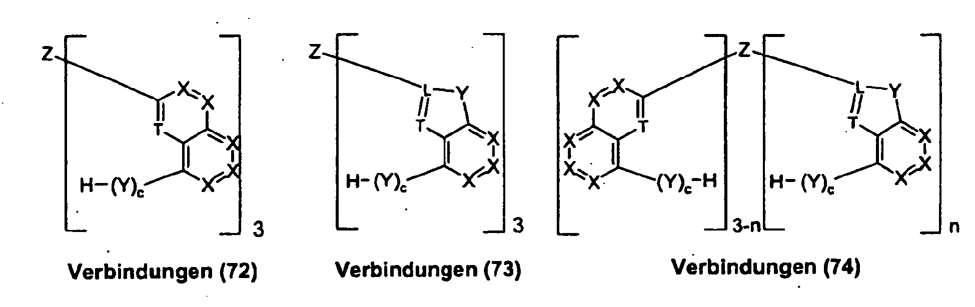 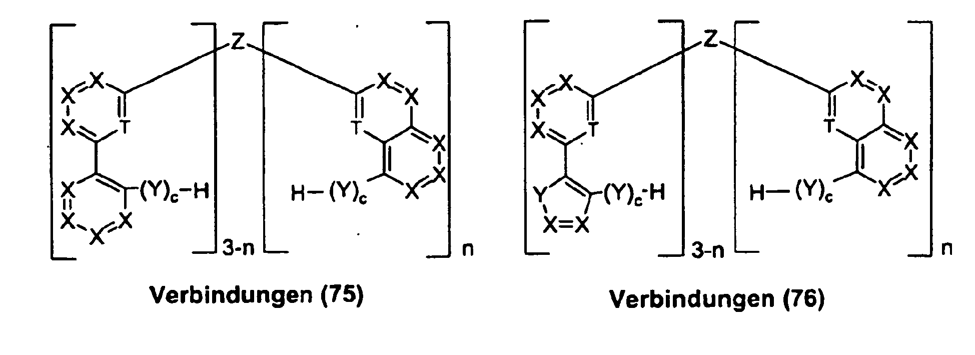 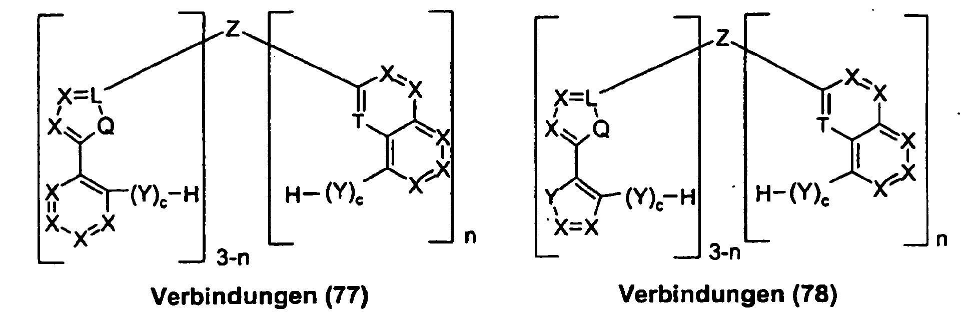 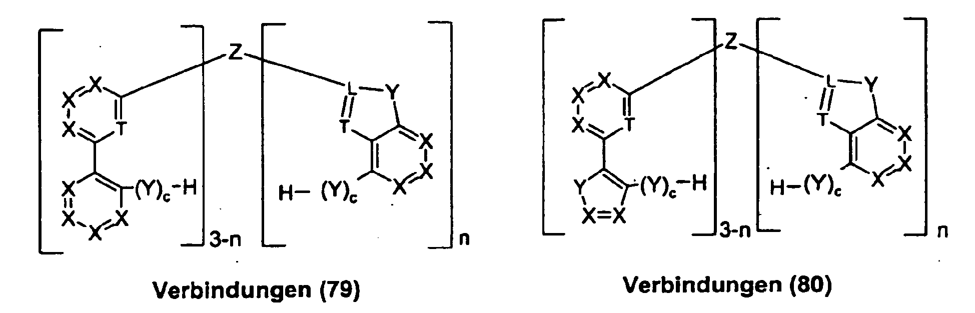 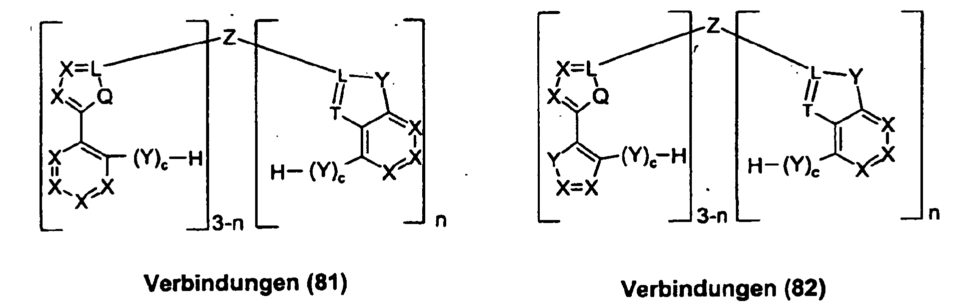
wobei die Symbole und Indizes L, Q, T, X, Y, Z, R, R¹, c und n die Bedeutungen wie in Anspruch 7 und 8 haben.

12. Verbindungen gemäß einem oder mehreren der Ansprüche 7 bis 10, **dadurch gekennzeichnet, daß** das Symbol M = AI, Ga, In, Sc, Y, La, Ru, Os, Rh, Ir oder Au bedeutet.

13. Verbindungen gemäß einem oder mehreren der Ansprüche 7 bis 11, **dadurch gekennzeichnet, daß** das Symbol L für die Verbindungen (44) in Anspruch 11 = C bedeutet und daß das Symbol L für die weiteren Verbindungen = C oder N bedeutet.

14. Verbindungen gemäß einem oder mehreren der Ansprüche 7 bis 11, **dadurch gekennzeichnet, daß** das Symbol Q = O oder S bedeutet.

15. Verbindungen gemäß einem oder mehreren der Ansprüche 7 bis 11, **dadurch gekennzeichnet, daß** das Symbol T = N bedeutet.

16. Verbindungen gemäß einem oder mehreren der Ansprüche 7 bis 11, **dadurch gekennzeichnet, daß** das Symbol X für die Verbindungen-(42) in Anspruch 11 = CR bedeutet und daß das Symbol X für die weiteren Verbindungen = CR oder N bedeutet.

17. Verbindungen gemäß einem oder mehreren der Ansprüche 7 bis 11, **dadurch gekennzeichnet, daß** das Symbol Z = B, CH, CR¹, COR¹, CF, CCI, CBr, SiR, N, P, PO, RC(CR₂)₃, RC(CR₂CR₂)₃, cis,cis-1,3,5-Cyclohexyl, RSi(CR₂)₃, RSi(CR₂CR₂)₃, N(CR₂)₃, N(C=O)₃, N(CR₂CR₂)₃ bedeutet.

18. Verbindungen gemäß einem oder mehreren der Ansprüche 7 bis 11, **dadurch gekennzeichnet, daß** das Symbol Y = O oder S bedeutet.

19. Verbindungen gemäß einem oder mehreren der Ansprüche 7 bis 11, **dadurch gekennzeichnet, daß** das Symbol R = H, F, Cl, Br, I, CN eine geradkettige oder verzweigte oder cyclische Alkyl- oder Alkoxygruppe mit 1 bis 6 C-Atomen oder eine Aryl- oder Heteroarylgruppe mit 3 bis 8 C-Atomen bedeutet, die durch einen oder mehrere, nicht aromatische Reste R substituiert sein kann, wobei mehrere Substituenten R, sowohl am selben Ring als auch an den beiden unterschiedlichen Ringen zusammen wiederum ein weiteres mono- oder polycyclisches, aliphatisches oder aromatisches Ringsystem aufspannen können.

20. Verbindungen gemäß einem oder mehreren der Ansprüche 7 bis 11, **dadurch gekennzeichnet, daß** das gegebenenfalls durch den oder die Rest(e) R aufgespannte polycycliche Ringsystem Benzol, 1- bzw. 2-Naphthalin, 1-, 2- bzw. 9-Anthracen, 2-, 3- bzw. 4-Pyridin, 2-, 4- bzw. 5-Pyrimidin, 2-Pyrazin, 3- bzw. 4-Pyridazin, 2-, 3-, 4-, 5-, 6-, 7- bzw. 8-Chinolin, 2- bzw. 3-Pyrrol, 3-, 4- bzw. 5-Pyrazol, 2-, 4- bzw. 5-Imidazol, 2- bzw. 3-Thiophen, 2- bzw. 3-Selenophen, 2- bzw. 3-Furan, 2-(1,3,4-Oxadiazol), Indol oder Carbazol entspricht.

21. Verfahren zur Herstellung der Verbindungen gemäß Anspruch 7 bis 10, durch Umsetzung der Verbindungen (42) bis (82), gemäß Anspruch 11, mit Metallalkoholaten der Formel (83), mit Metallketoketonaten der Formel (84), oder Metallhalogeniden der Formel (85), 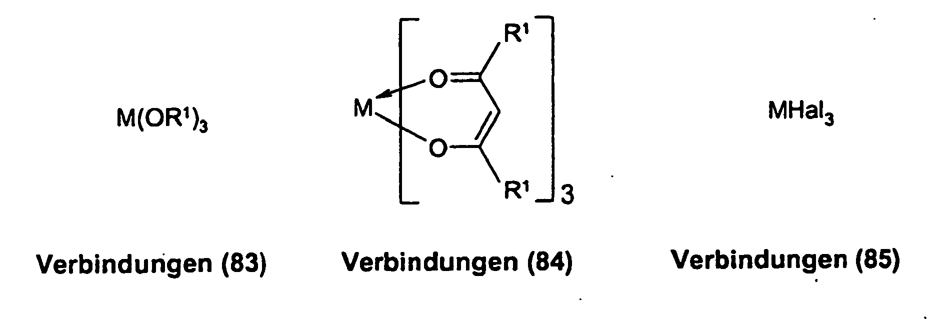 wobei das Symbol R¹ die in Anspruch 7 angegebene Bedeutung hat und Hal = F, Cl, Br, l ist.

22. Verbindungen gemäß einem oder mehreren der Ansprüche 1 bis 10 und 12 bis 20, **dadurch gekennzeichnet, daß** ihre Reinheit (mittels ¹H-NMR und/oder HPLC bestimmt) mehr als 99% beträgt.

23. Konjugierte, teilkonjugierte oder nicht-konjugierte Polymere oder Dendrimere enthaltend eine oder mehrere Verbindungen der Struktur (1) bzw. der Formel (1) bis (41) gemäß einem oder mehreren der Ansprüche 1 bis 10 und 12 bis 20.

24. Konjugierte, teilkonjugierte oder nicht-konjugierte Polymere oder Dendrimere gemäß Anspruch 23, wobei ein oder mehrere der in Anspruch 7 definierten R eine Bindung zum Polymer oder Dendrimer darstellt.

25. Polymere gemäß Anspruch 23 und / oder 24, **dadurch gekennzeichnet, daß** das Polymer aus der Gruppe Polyfluorene, Poly-spirobifluorene, Poly-para-phenylene, Poly-carbazole, Poly-vinylcarbazole, Polythiophene oder auch aus Copolymeren, die mehrere der hier genannten Einheiten aufweisen, ausgewählt ist.

26. Polymere gemäß einem oder mehreren der Ansprüche 23 bis 25, **dadurch gekennzeichnet, daß** das Polymer in organischen Lösemitteln löslich ist.

27. Verwendung einer. Verbindung gemäß einem oder mehreren der Ansprüche 1 bis 10 und 12 bis 20 oder eines Polymers oder Dendrimers gemäß einem oder mehreren der Ansprüche 23 bis 26 in elektronischen Bauteilen.

28. Elektronisches Bauteil, enthaltend mindestens eine Verbindung gemäß einem oder mehreren der Ansprüche 1 bis 10 und 12 bis 20 oder ein Polymer oder Dendrimer gemäß einem oder mehreren der Ansprüche 23 bis 26.

29. Elektronisches Bauteil gemäß Anspruch 28, **dadurch gekennzeichnet, daß** es sich um eine Organische Leuchtdiode (OLED), Organische Integrierte Schaltung (O-IC), Organischen Feld-Effekt-Transistor (OFET), Organischen Dünnfilmtransistor (OTFT), Organische Solarzelle (O-SC) oder Organische Laserdiode (O-Laser) handelt.

## Claims

1. Compounds of structure 1 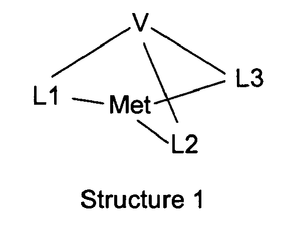 containing at least one metal Met coordinated to a polypodal ligand Lig of structure 2 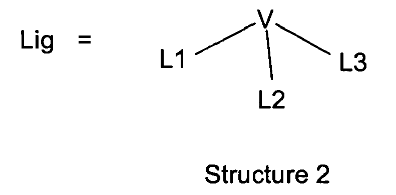 where V is a bridging unit, **characterised in that** it contains 1 to 80 atoms and covalently bonds the three ligand moieties L1, L2 and L3, which may be identical or different on each occurrence, to one another, and where the three ligand moieties L1, L2 and L3 satisfy structure 3 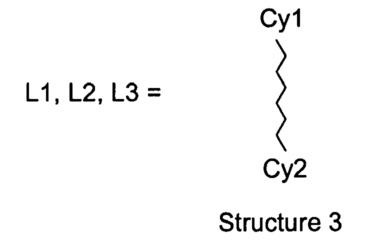 where Cy1 and Cy2, identically or differently on each occurrence, correspond to substituted or unsubstituted, saturated, unsaturated or aromatic homo- or heterocycles or homo- or heterocycle moiety of a condensed system, which are in each case bonded covalently or coordinatively to the metal via a ring atom or via an atom bonded exocyclically to the homo- or heterocycle,
where the compounds of structure 1 are uncharged, i.e. are electrically neutral to the outside.

2. Compounds according to Claim 1, **characterised in that** L1 = L2 = L3.

3. Compounds according to Claim 1, **characterised in that** L1 # L2.

4. Compounds according to one or more of Claims 1 to 3, **characterised in that** the linking unit V contains, as linking atom, an element from main group 3, 4 or 5 or a 3-to 6-membered homo- or heterocycle.

5. Compounds according to one or more of Claims 1 to 4, **characterised in that** the polypodal ligand Lig of structure 4 produces a *facial* coordination geometry at the metal Met.

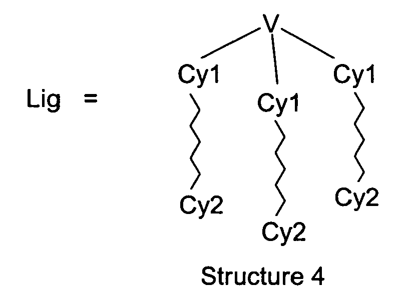

6. Compounds according to one or more of Claims 1 to 4, **characterised in that** the polypodal ligand Lig of structure 5 produces a *meridional* coordination geometry at the metal Met.

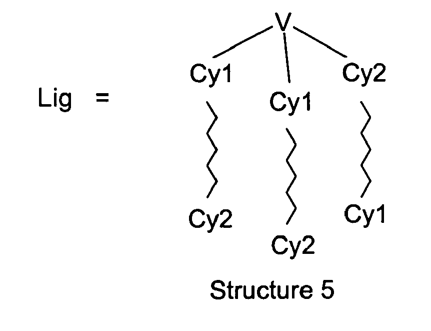

7. Metal complexes according to one or more of Claims 1 to 6, selected from compounds (1) to (8) 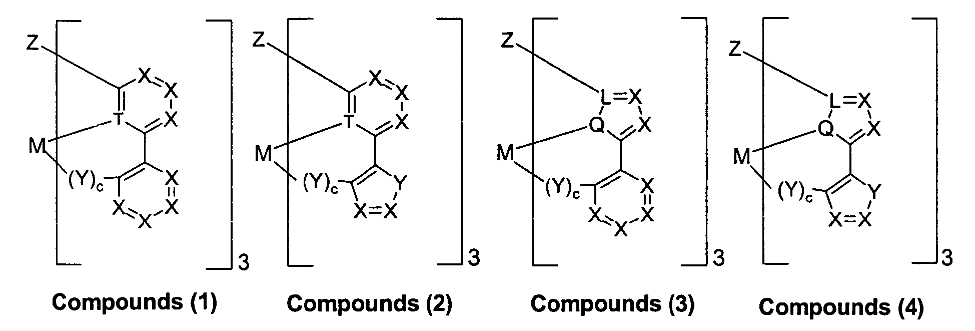 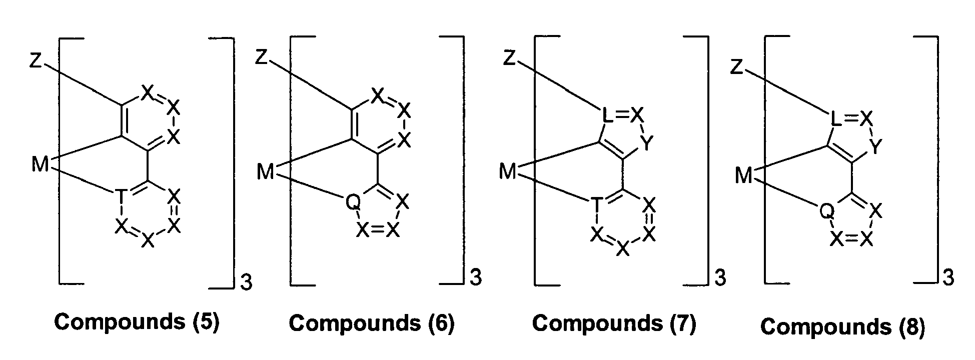 where the symbols and indices have the following meaning:
M Al, Ga, In, Tl, P, As, Sb, Bi, Sc, Y, La, V, Nb, Ta, Cr, Mo, W, Fe, Ru, Os, Co, Rh, Ir, Cu, Au, Pr, Nd, Sm, Eu, Gd, Tb, Dy, Ho, Er, Tm, Yb, Lu;
L is, identically or differently on each occurrence, C, N, P;
Q is, identically or differently on each occurrence, O, S, Se, Te, N;
T is, identically or differently on each occurrence, N, P, C;
X is, identically or differently on each occurrence, CR, N, P;
Y is, identically or differently on each occurrence, NR¹, O, S, Se, Te, SO, SeO, TeO, SO₂, SeO₂, TeO₂;
Z B, BR, B(CR₂)₃, B(CR₂CR₂)₃, CR, COH, COR¹, CF, CCI, CBr, C-I, CNR¹₂, RC(CR₂)₃, RC(CR₂CR₂)₃, RC(SiR₂)₃, RC(SiR₂CR₂)₃, RC(CR₂SiR₂)₃, RC(SiR₂SiR₂)₃, cis,cis-1,3,5-cyclohexyl, 1,3,5-(CR₂)₃C₆H₃, SiR, SiOH, SiOR¹, RSi(CR₂)₃, RSi(CR₂CR₂)₃, RSi(SiR₂)₃, RSi(SiR₂CR₂)₃, RSi(CR₂SiR₂)₃, RSi(SiR₂SiR₂)₃, N, N(CR₂)₃, N(C=O)₃, N(CR₂CR₂)₃, NO, P, As, Sb, Bi, PO, AsO, SbO, BiO, PSe, AsSe, SbSe, BiSe, PTe, AsTe, SbTe, BiTe;
R is, identically or differently on each occurrence, H, F, Cl, Br, l, NO₂, CN, a straight-chain or branched or cyclic alkyl or alkoxy group having 1 to 20 C atoms, in which one or more non-adjacent CH₂ groups may be replaced by -R¹C=CR¹-, -C≡C-, Si(R¹)₂, Ge(R¹)₂, Sn(R¹)₂, C=O, C=S, C=Se, C=NR¹, -O-, -S-, -NR¹- or -CONR¹- and in which one or more H atoms may be replaced by F, or an aryl or heteroaryl group having 1 to 14 C atoms, which may be substituted by one or more non-aromatic radicals R, where a plurality of substituents R, both on the same ring and also on the two different rings, may together in turn form a further mono- or polycyclic, aliphatic or aromatic ring system;
R¹ is, identically or differently on each occurrence, H or an aliphatic or aromatic hydrocarbon radical having 1 to 20 C atoms;
c is on each occurrence, identically or differently, 0 or 1.

8. Metal complexes according to one or more of Claims 1 to 6, selected from compounds (9) to (12) 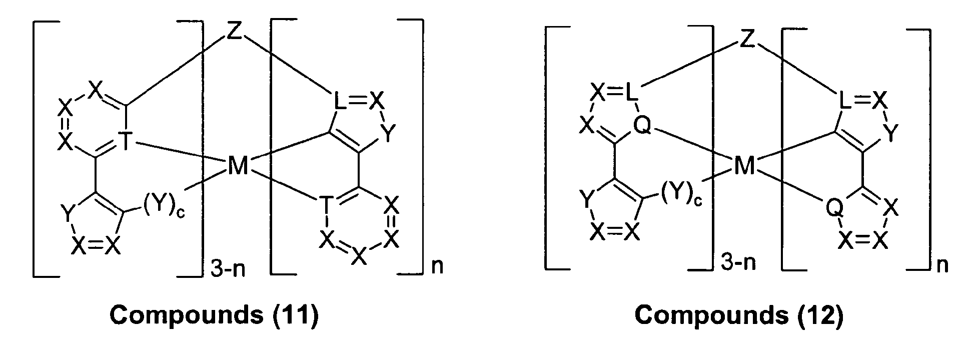 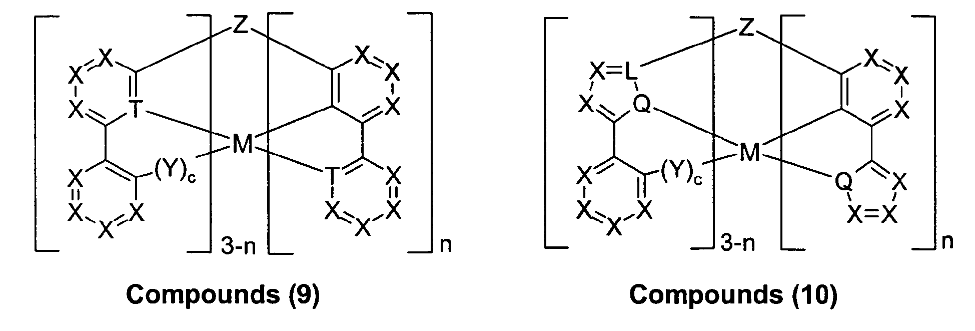 where the symbols and indices M, L, Q, T, X, Y, Z, R, R¹ and c have the meanings as in Claim 7, and where:
n is 1 or 2.

9. Metal complexes according to one or more of Claims 1 to 6, selected from compounds (13) to (30) 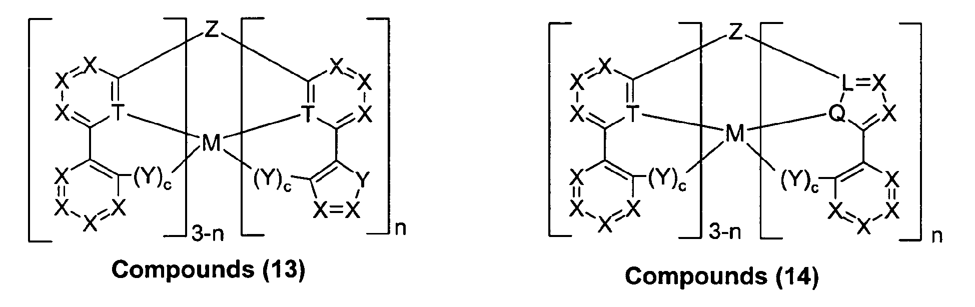 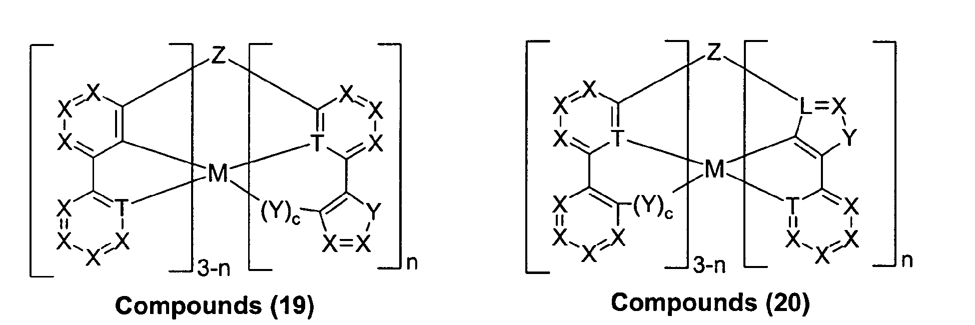 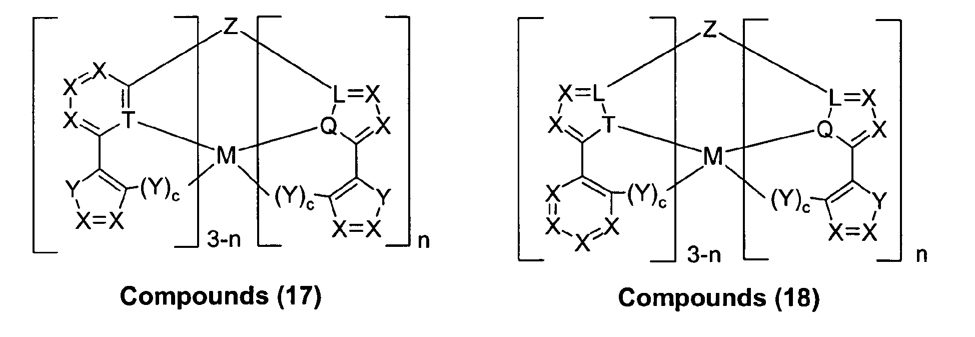 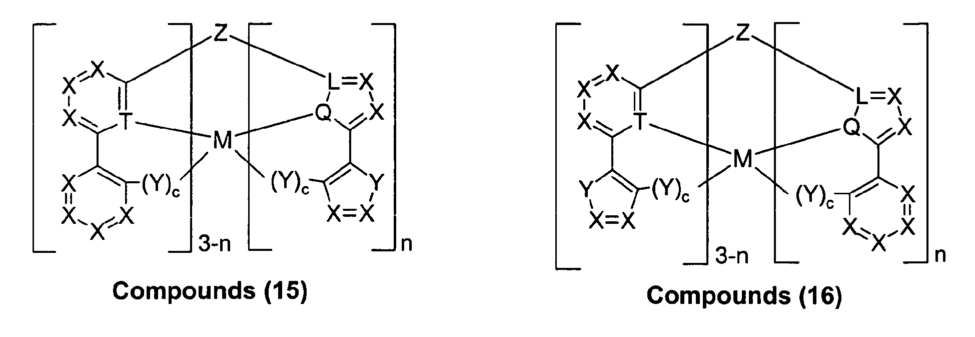 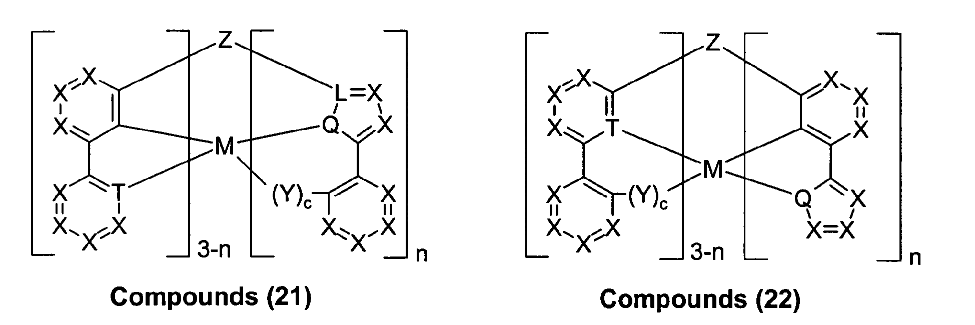 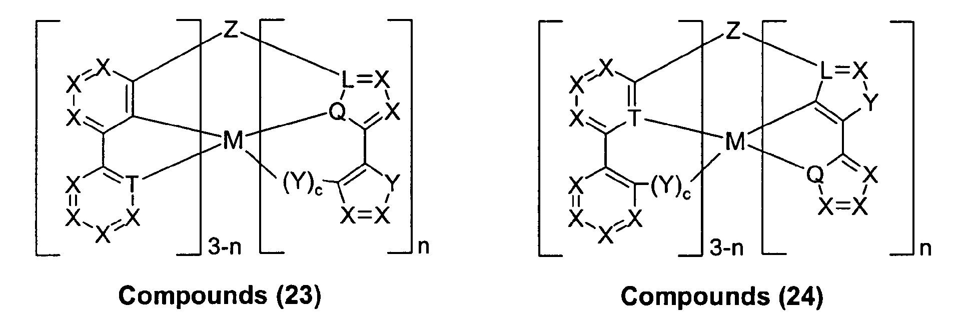 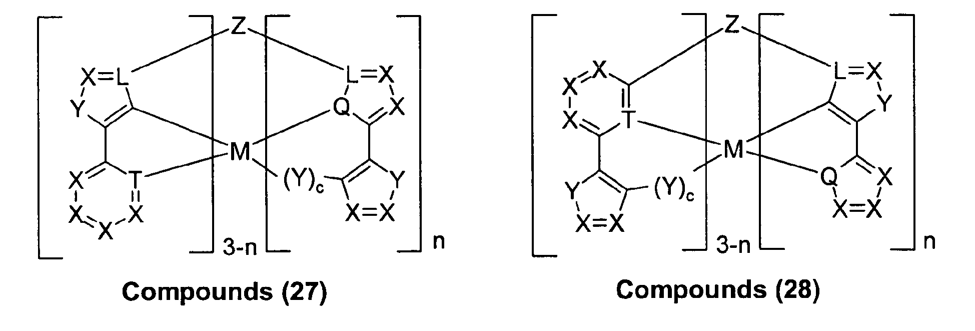 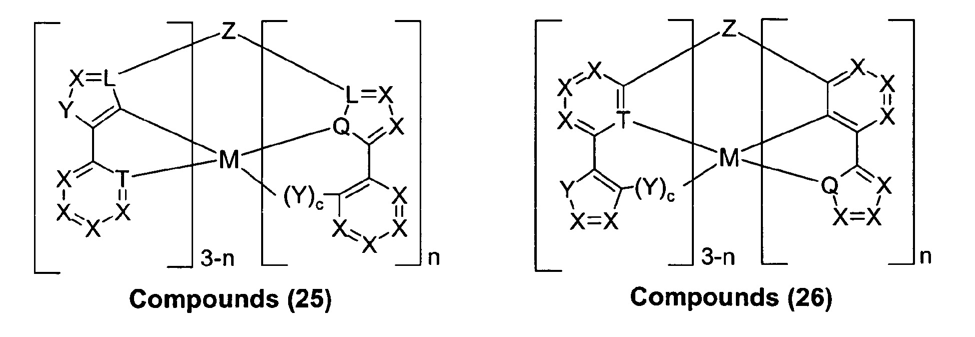 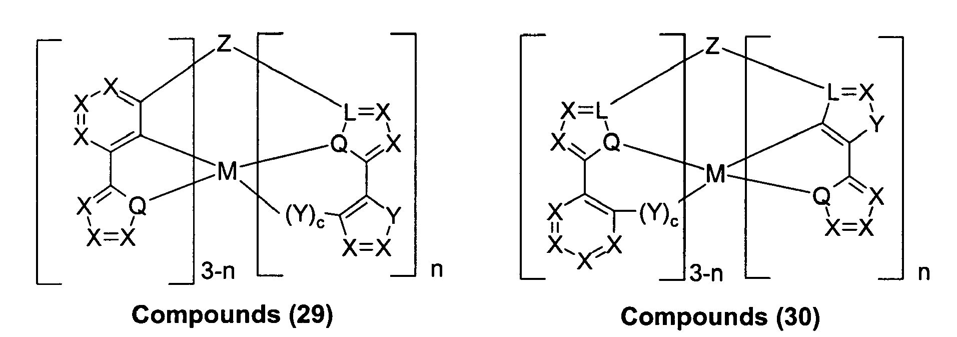 where the symbols and indices M, L, Q, T, X, Y, Z, R, R¹, c and n have the meanings as in Claims 7 and 8.

10. Metal complexes according to one or more of Claims 1 to 6, selected from compounds (31) to (41) 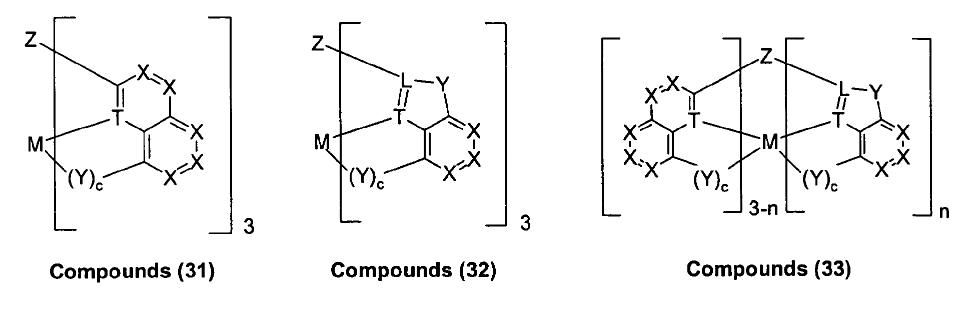 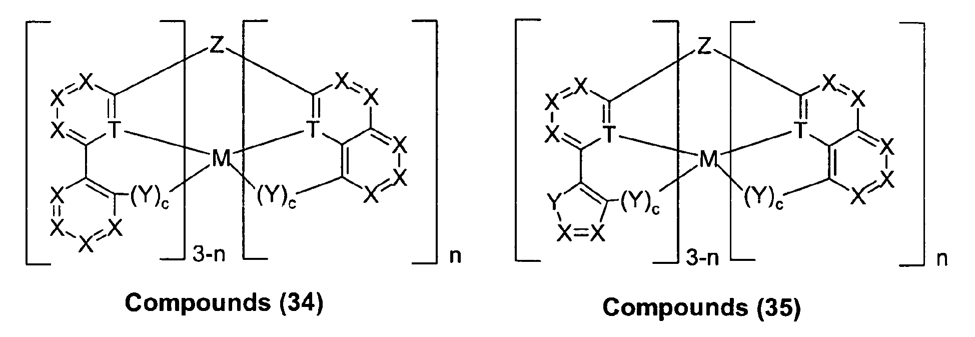 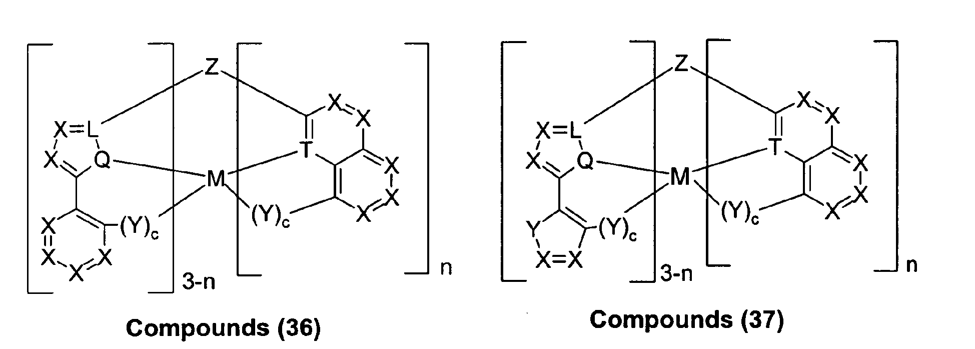 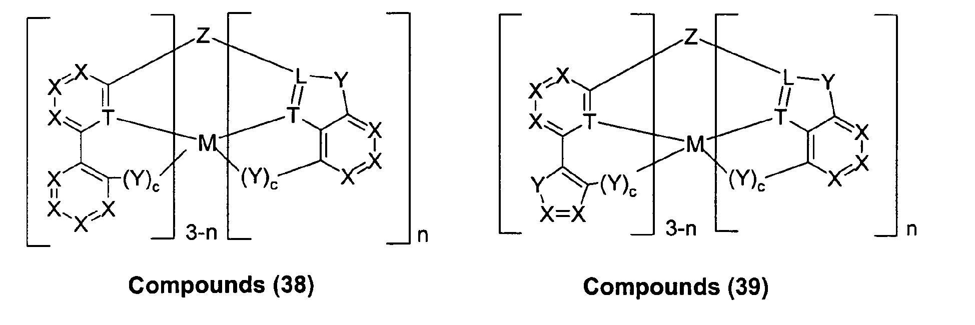 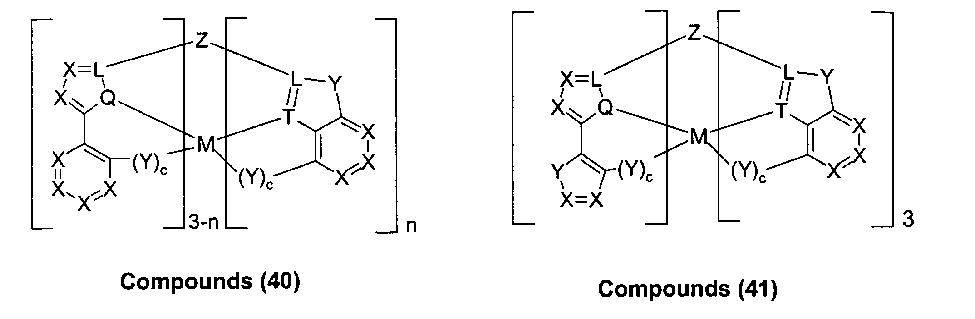 where the symbols and indices M, L, Q, T, X, Y, Z, R, R¹, c and n have the meanings as in Claims 7 and 8.

11. Compounds (42) to (82) 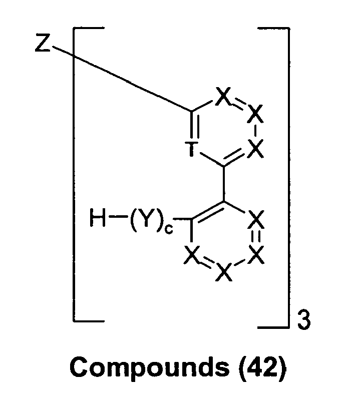 where the symbols and indices T, Y, Z, R, R¹, c and n have the meanings as in Claims 7 and 8,
X is, identically or differently on each occurrence, CR or P; 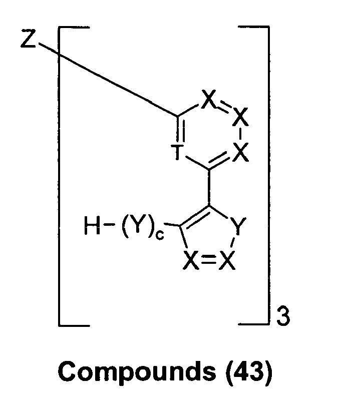 where the symbols and indices T, X, Y, Z, R, R¹, c and n have the meanings as in Claims 7 and 8, 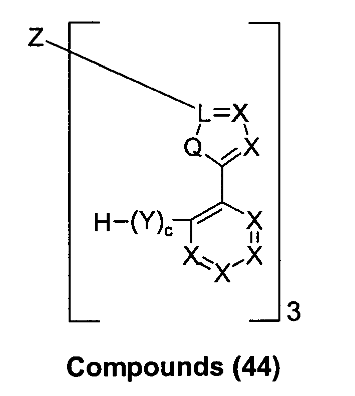 where the symbols and indices Q, T, X, Y, Z, R, R¹, c and n have the meanings as in Claims 7 and 8,
L is, identically or differently on each occurrence, C or P; 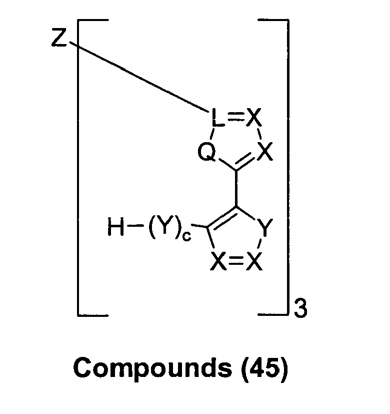 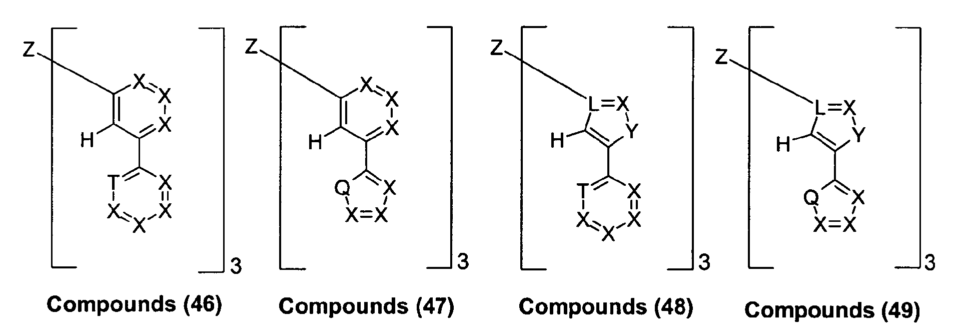 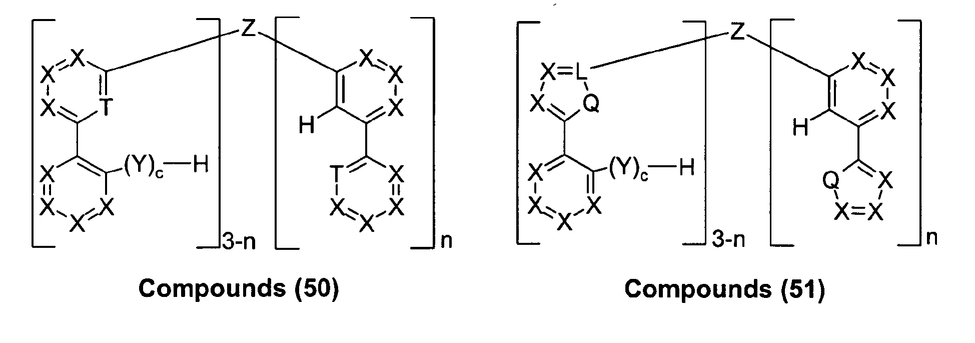 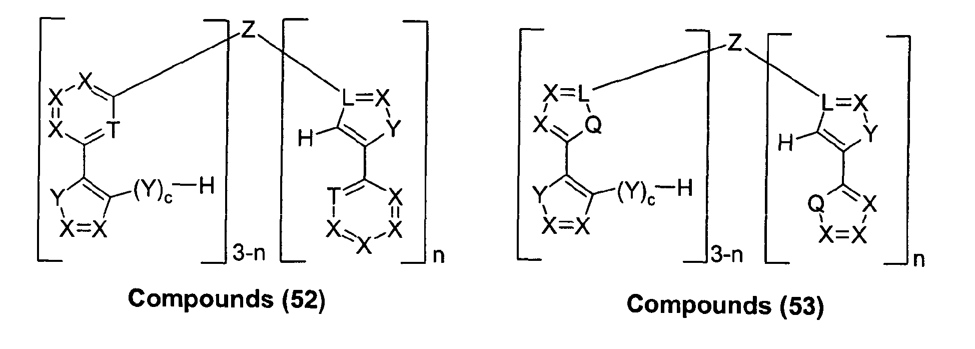 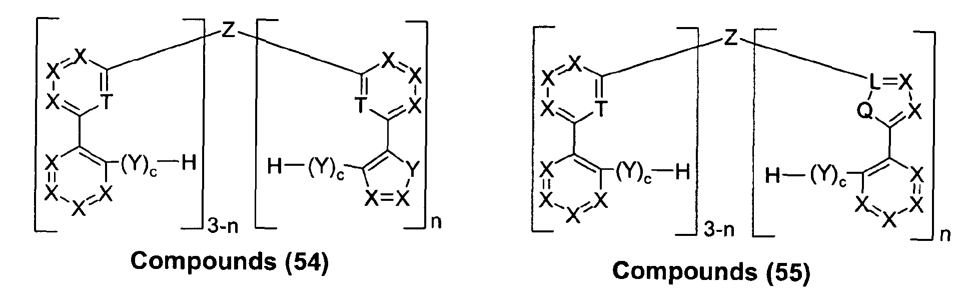 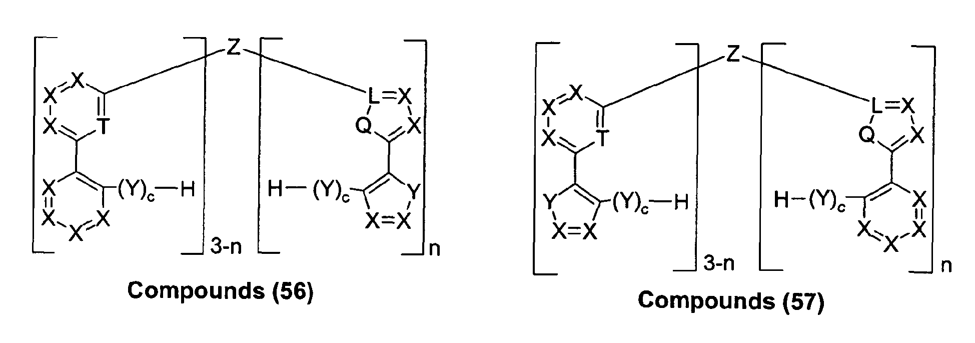 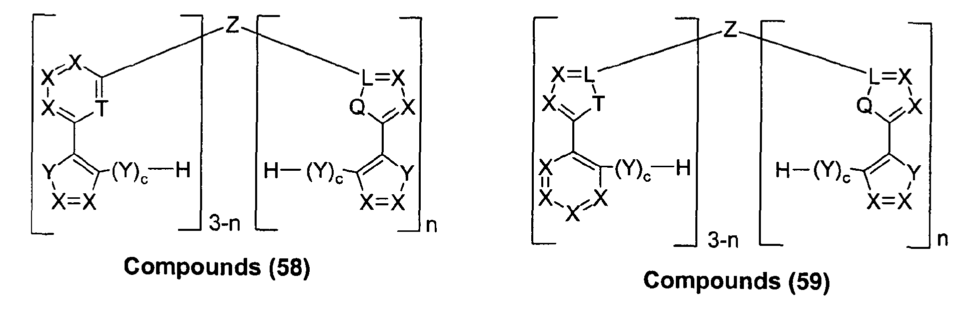 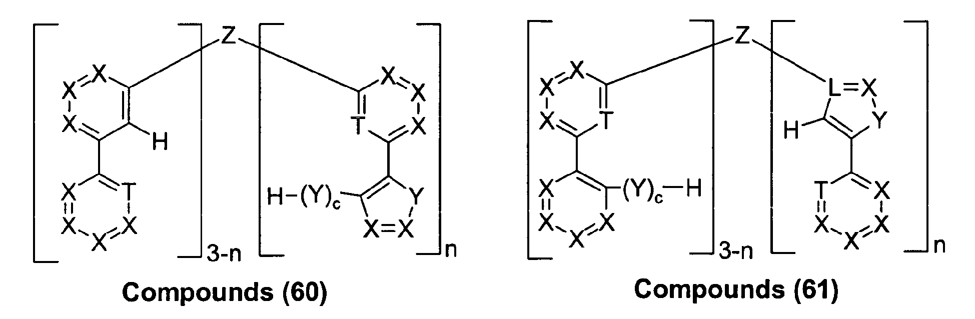 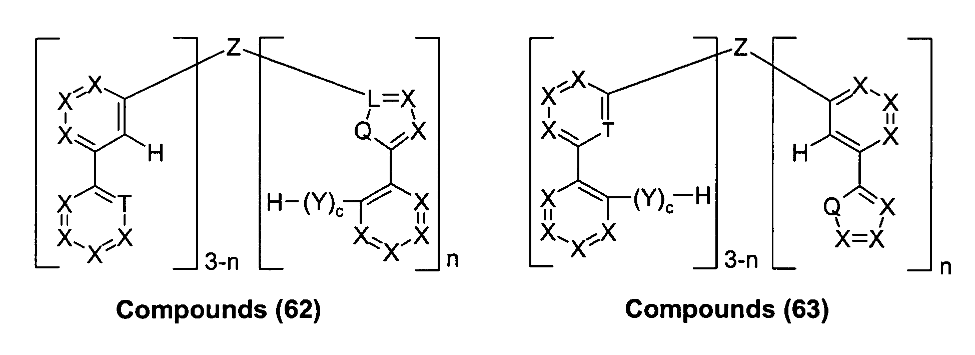 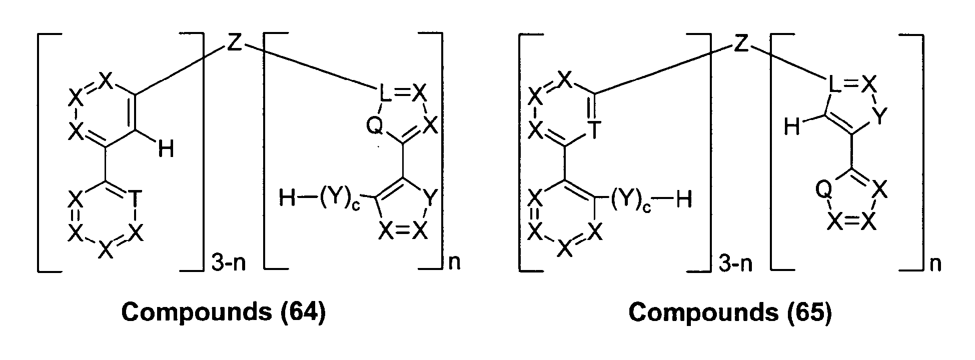 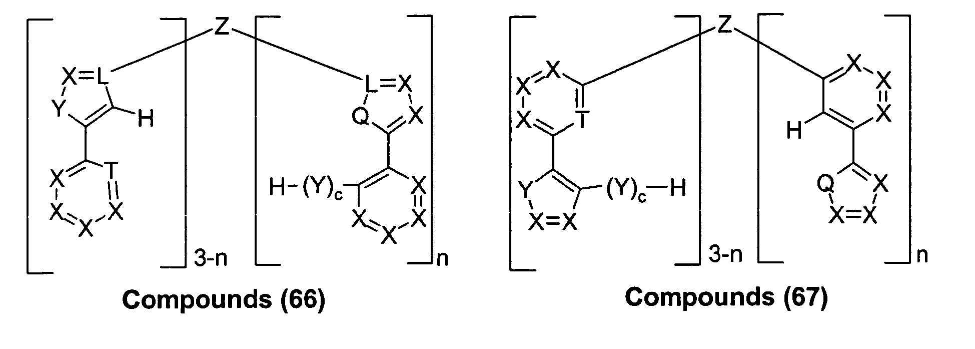 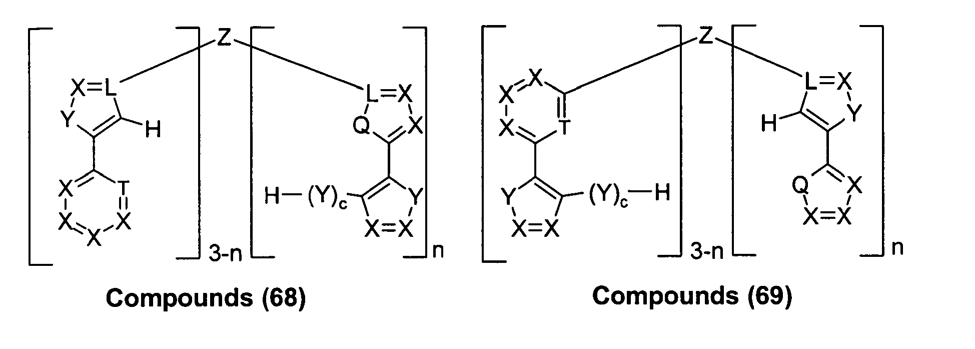 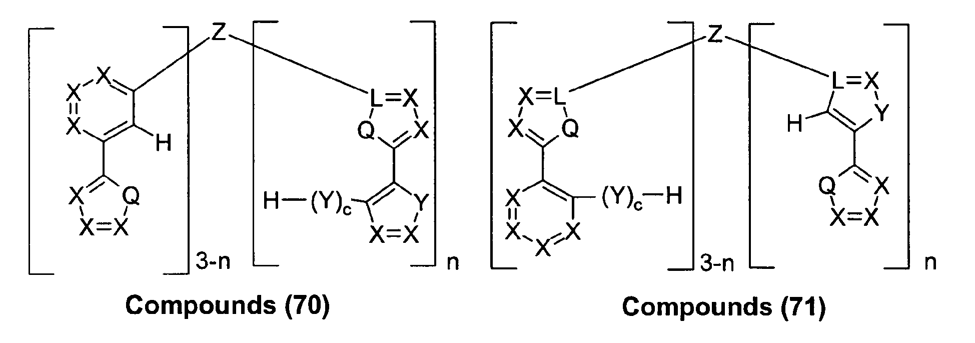 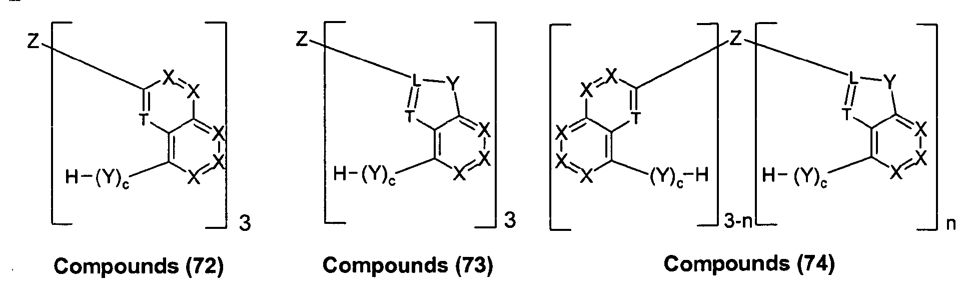 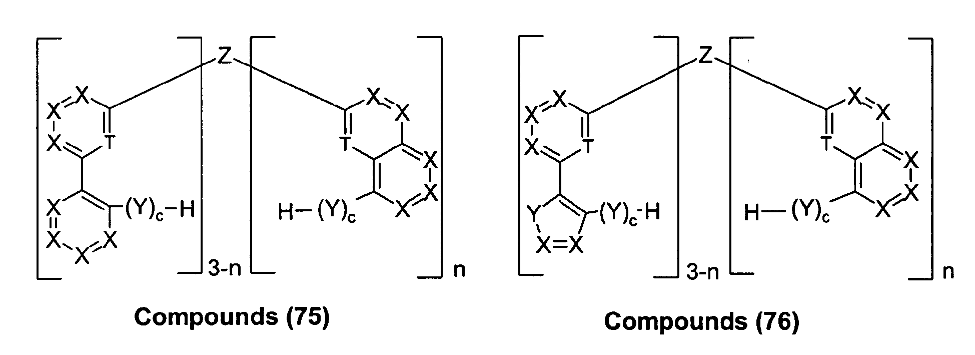 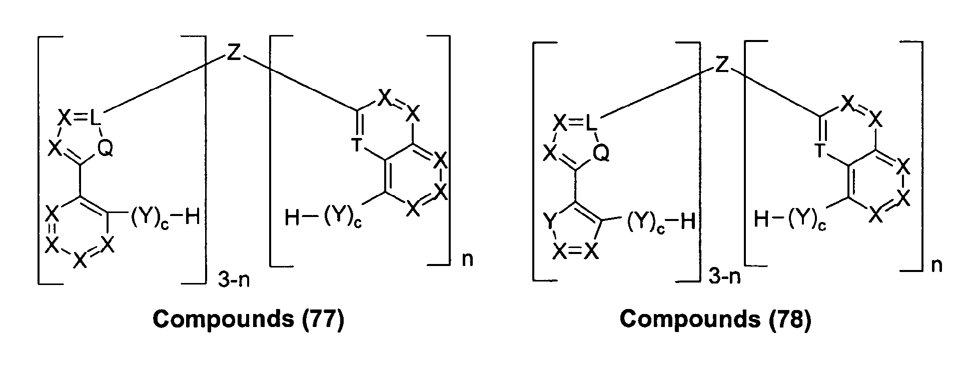 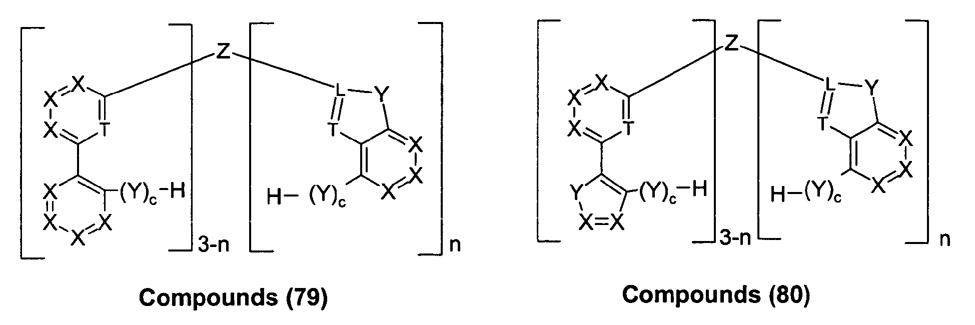 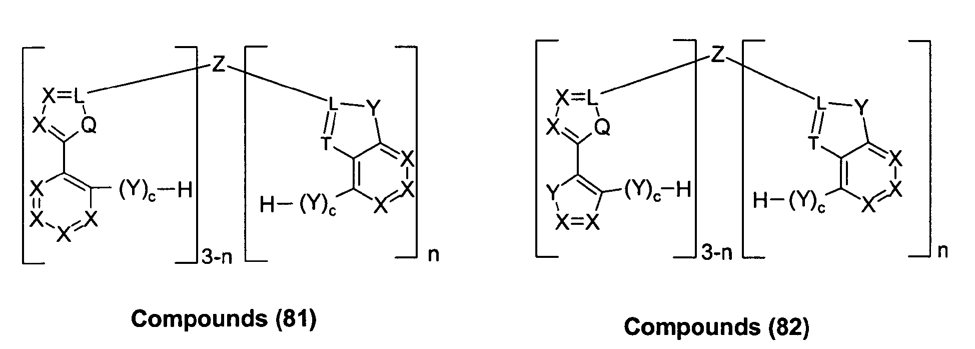
where the symbols and indices L, Q, T, X, Y, Z, R, R¹, c and n have the meanings as in Claims 7 and 8.

12. Compounds according to one or more of Claims 7 to 10, **characterised in that** the symbol M = Al, Ga, In, Sc, Y, La, Ru, Os, Rh, Ir or Au.

13. Compounds according to one or more of Claims 7 to 11, **characterised in that** the symbol L = C for compounds (44) in Claim 11 and **in that** the symbol L = C or N for the other compounds.

14. Compounds according to one or more of Claims 7 to 11, **characterised in that** the symbol Q = O or S.

15. Compounds according to one or more of Claims 7 to 11, **characterised in that** the symbol T = N.

16. Compounds according to one or more of Claims 7 to 11, **characterised in that** the symbol X = CR for compounds (42) in Claim 11 and **in that** the symbol X = CR or N for the other compounds.

17. Compounds according to one or more of Claims 7 to 11, **characterised in that** the symbol Z = B, CH, CR¹, COR¹, CF, CCI, CBr, SiR, N, P, PO, RC(CR₂)₃, RC(CR₂CR₂)₃, cis,cis-1,3,5-cyclohexyl, RSi(CR₂)₃, RSi(CR₂CR₂)₃, N(CR₂)₃, N(C=O)₃, N(CR₂CR₂)₃.

18. Compounds according to one or more of Claims 7 to 11, **characterised in that** the symbol Y = O or S.

19. Compounds according to one or more of Claims 7 to 11, **characterised in that** the symbol R = H, F, Cl, Br, I, CN, a straight-chain or branched or cyclic alkyl or alkoxy group having 1 to 6 C atoms or an aryl or heteroaryl group having 3 to 8 C atoms, which may be substituted by one or more non-aromatic radicals R, where a plurality of substituents R, both on the same ring and also on the two different rings, may together in turn form a further mono- or polycyclic, aliphatic or aromatic ring system.

20. Compounds according to one or more of Claims 7 to 11, **characterised in that** the polycyclic ring system optionally formed by the radical(s) R corresponds to benzene, 1- or 2-naphthalene, 1-, 2- or 9-anthracene, 2-, 3- or 4-pyridine, 2-, 4- or 5-pyrimidine, 2-pyrazine, 3- or 4-pyridazine, 2-, 3-, 4-, 5-, 6-, 7- or 8-quinoline, 2- or 3-pyrrole, 3-, 4- or 5-pyrazole, 2-, 4- or 5-imidazole, 2- or 3-thiophene, 2- or 3-selenophene, 2- or 3-furan, 2-(1,3,4-oxadiazole), indole or carbazole.

21. Process for the preparation of the compounds according to Claims 7 to 10, by reaction of compounds (42) to (82) according to Claim 11 with metal alkoxides of the formula (83), with metal ketoketonates of the formula (84) or metal halides of the formula (85) 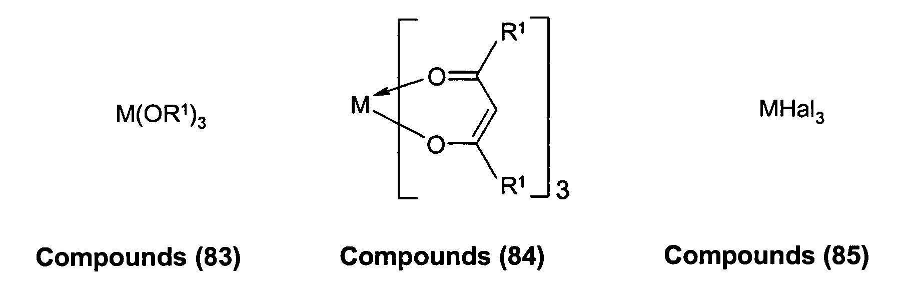 where the symbol R¹ has the meaning indicated in Claim 7, and Hal = F, Cl, Br, I.

22. Compounds according to one or more of Claims 1 to 10 and 12 to 20, **characterised in that** their purity (determined by means of ¹H-NMR and/or HPLC) is greater than 99%.

23. Conjugated, partially conjugated or non-conjugated polymers or dendrimers containing one or more compounds of structure (1) or of the formulae (1) to (41) according to one or more of Claims 1 to 10 and 12 to 20.

24. Conjugated, partially conjugated or non-conjugated polymers or dendrimers according to Claim 23, where one or more of the R defined in Claim 7 represent a bond to the polymer or dendrimer.

25. Polymers according to Claim 23 and/or 24, **characterised in that** the polymer is selected from the group polyfluorenes, polyspirobifluorenes, poly-para-phenylenes, polycarbazoles, polyvinylcarbazoles, polythiophenes or also from copolymers which contain a plurality of the units mentioned here.

26. Polymers according to one or more of Claims 23 to 25, **characterised in that** the polymer is soluble in organic solvents.

27. Use of a compound according to one or more of Claims 1 to 10 and 12 to 20 or of a polymer or dendrimer according to one or more of Claims 23 to 26 in electronic components.

28. Electronic component comprising at least one compound according to one or more of Claims 1 to 10 and 12 to 20 or a polymer or dendrimer according to one or more of Claims 23 to 26.

29. Electronic component according to Claim 28, **characterised in that** it is an organic light-emitting diode (OLED), organic integrated circuit (O-IC), organic field-effect transistor (OFET), organic thin-film transistor (OTFT), organic solar cell (O-SC) or organic laser diode (O-laser).

## Revendications

1. Composés de la structure 1 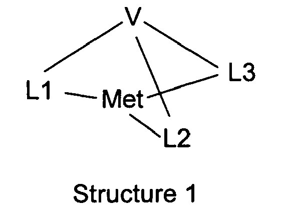 contenant au moins un métal Met coordonné à un ligand polypodal Lig de la structure 2 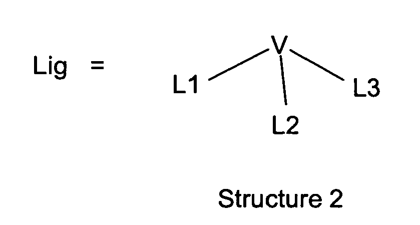 dans laquelle V est une unité de pontage, **caractérisée en ce qu'**elle contient de 1 à 80 atomes et elle lie de façon covalente les ligands en trois parties L1, L2 et L3, qui peuvent être identiques ou différentes lors de chaque occurrence, les uns aux autres et dans laquelle les ligands en trois parties L1, L2 et L3 satisfont la structure 3 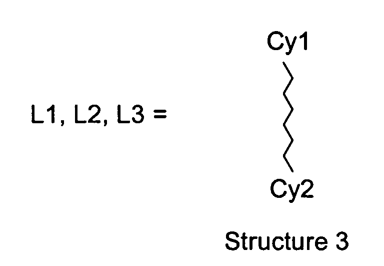 dans laquelle Cy1 et Cy2, de manière identique ou différente lors de chaque occurrence, correspondent à des homocycles ou hétérocycles substitués ou non substitués, saturés, non saturés ou aromatiques ou à des homocycles ou hétérocycles partiels d'un système condensé, lesquels sont dans chaque cas liés de manière covalente ou de façon coordonnée au métal via un atome de cycle ou via un atome lié exocycliquement à l'homocycle ou à l'hétérocycle,
dans laquelle les composés de la structure 1 sont non chargés, c'est-à-dire qu'ils sont électriquement neutres vis-à-vis de l'extérieur.

2. Composés selon la revendication 1, **caractérisés en ce que** L1 = L2 = L3.

3. Composés selon la revendication 1, **caractérisés en ce que** L1 # L2.

4. Composés selon une ou plusieurs des revendications 1 à 3, **caractérisés en ce que** l'unité de liaison V contient, en tant qu'atome de liaison, un élément pris parmi un groupe principal 3, 4 ou 5 ou un homocycle ou hétérocycle de 3 à 6 éléments.

5. Composés selon une ou plusieurs des revendications 1 à 4, **caractérisés en ce que** le ligand polypodal Lig de structure 4 produit une géométrie de coordination *faciale* au niveau du métal Met

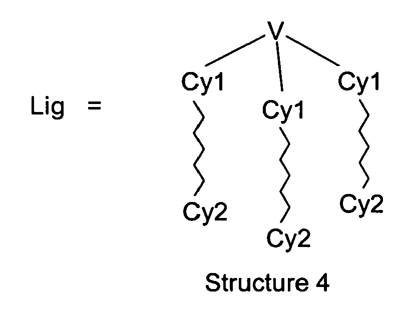

6. Composés selon une ou plusieurs des revendications 1 à 4, **caractérisés en ce que** le ligand polypodal Lig de structure 5 produit une géométrie de coordination *méridionale* au niveau du métal Met

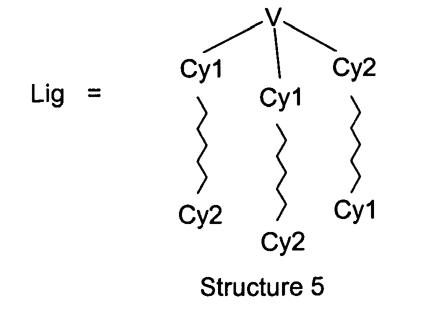

7. Complexes métalliques selon une ou plusieurs des revendications 1 à 6, choisis parmi les composés (1) à (8) 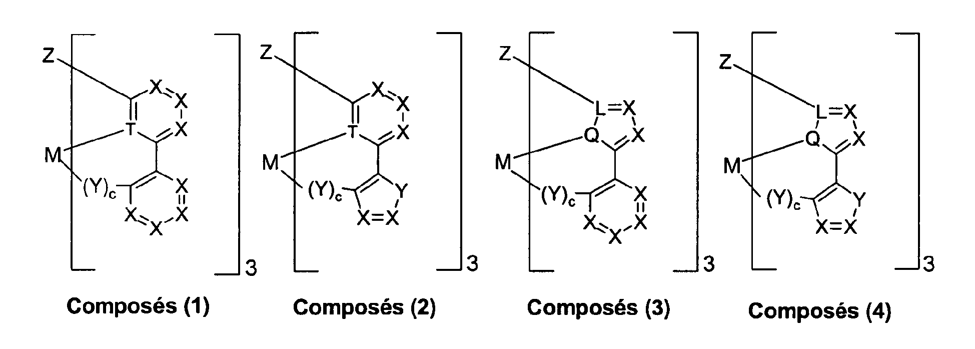 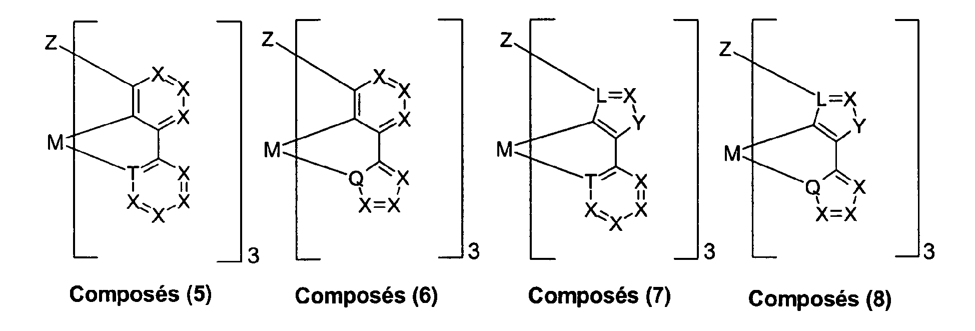 dans lesquels les symboles et indices présentent les significations qui suivent :
M Al, Ga, In, TI, P, As, Sb, Bi, Sc, Y, La, V, Nb, Ta, Cr, Mo, W, Fe, Ru, Os, Co, Rh, Ir, Cu, Au, Pr, Nd, Sm, Eu, Gd, Tb, Dy, Ho, Er, Tm, Yb, Lu ;
L est, de manière identique ou différente lors de chaque occurrence, C, N, P ;
Q est, de manière identique ou différente lors de chaque occurrence, O, S, Se, Te, N ;
T est, de manière identique ou différente lors de chaque occurrence, N, P, C ;
X est, de manière identique ou différente lors de chaque occurrence, CR, N, P ;
Y est, de manière identique ou différente lors de chaque occurrence, NR¹, O, S, Se, Te, SO, SeO, TeO, SO₂, SeO₂, TeO₂;
Z B, BR, B(CR₂)₃, B(CR₂CR₂)₃, CR, COH, COR¹, CF, CCI, CBr, C-l, CNR¹₂, RC(CR₂)₃, RC(CR₂CR₂)₃, RC(SiR₂)₃, RC(SiR₂CR₂)₃, RC(CR₂SiR₂)₃, RC(SiR₂SiR₂)₃, cis,cis-1,3,5-cyclohexyl, 1,3,5-(CR₂)₃C₆H₃, SiR, SiOH, SiOR¹, RSi(CR₂)₃, RSi(CR₂CR₂)₃, RSi(SiR₂)₃, RSi(SiR₂CR₂)₃, RSi(CR₂SiR₂)₃, RSi(SiR₂SiR₂)₃, N, N(CR₂)₃, N(C=O)₃, N(CR₂CR₂)₃, NO, P, As, Sb, Bi, PO, AsO, SbO, BiO, PSe, AsSe, SbSe, BiSe, PTe, AsTe, SbTe, BiTe ;
R est, de manière identique ou différente lors de chaque occurrence, H, F, Cl, Br, l, NO₂, CN, un groupe alkyle ou alkoxy en chaîne droite ou ramifié ou cyclique comportant de 1 à 20 atomes de C où un ou plusieurs groupes CH₂ non adjacents peuvent être remplacés par -R¹C=CR¹-, -C≡C-, Si(R¹)₂, Ge(R¹)₂, Sn(R¹)₂, C=O, C=S, C=Se, C=NR¹, -O-, -S-, -NR¹- ou -CONR¹- et où un ou plusieurs atomes de H peuvent être remplacés par F, ou un groupe aryle ou hétéroaryle comportant de 1 à 14 atomes de C, qui peuvent être substitués par un ou plusieurs radicaux R non aromatiques, où une pluralité de substituants R, à la fois sur le même cycle et également sur les deux cycles différents, peuvent à leur tour former ensemble un autre système de cycle monocyclique ou polycyclique, aliphatique ou aromatique ;
R¹ est, de manière identique ou différente lors de chaque occurrence, H ou un radical hydrocarbone aliphatique ou aromatique comportant de 1 à 20 atomes deC;
c est, lors de chaque occurrence, de manière identique ou différente, 0 ou 1.

8. Complexes métalliques selon une ou plusieurs des revendications 1 à 6, choisis parmi les composés (9) à (12) 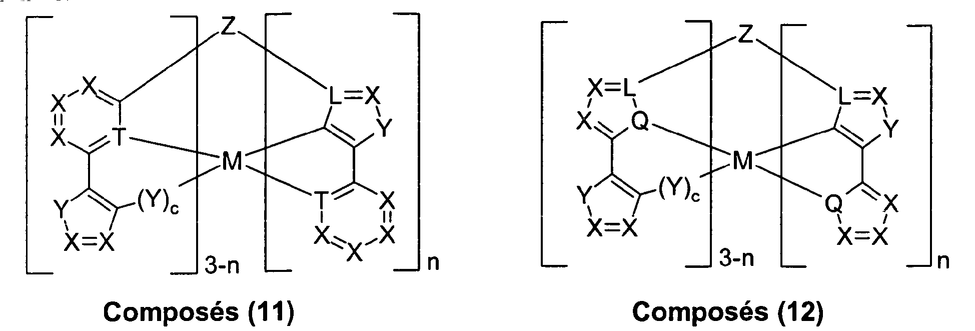 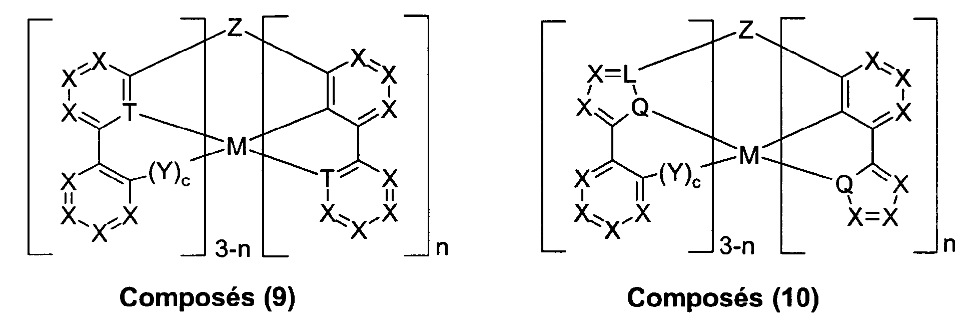 dans lesquels les symboles et indices M, L, Q, T, X, Y, Z, R, R¹ et c présentent les significations telles que selon la revendication 7 et où :
n est 1 ou 2.

9. Complexes métalliques selon une ou plusieurs des revendications 1 à 6, choisis parmi les composés (13) à (30) 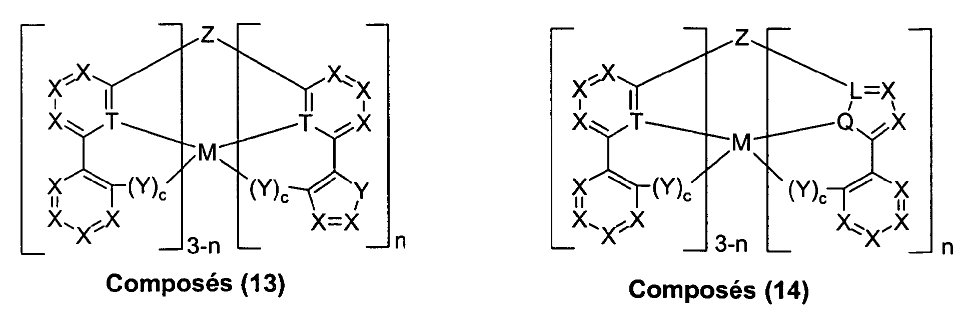 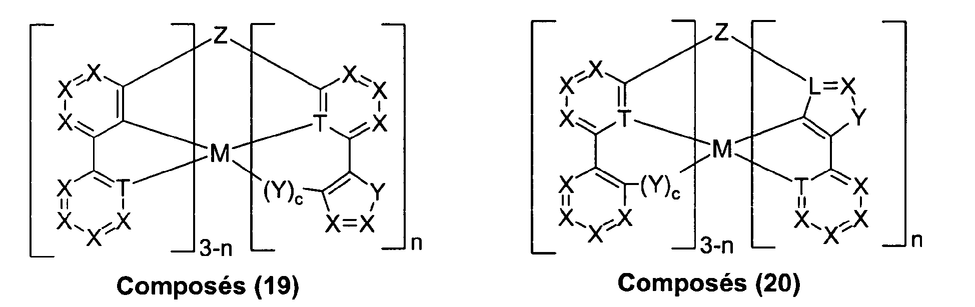 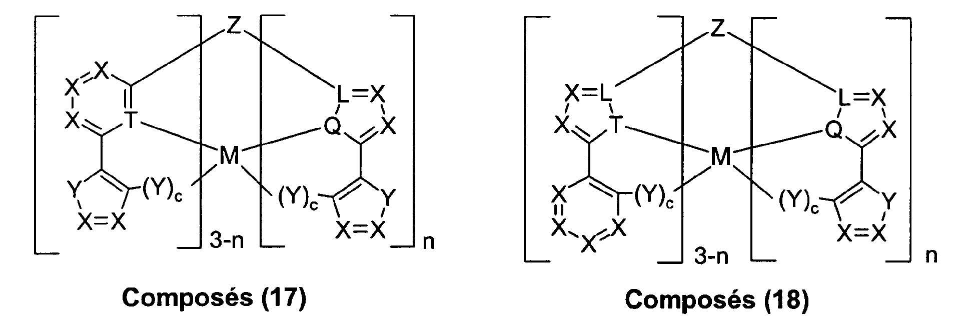 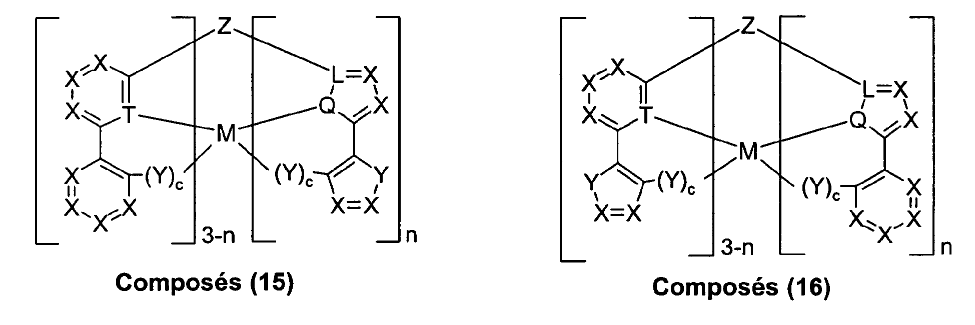 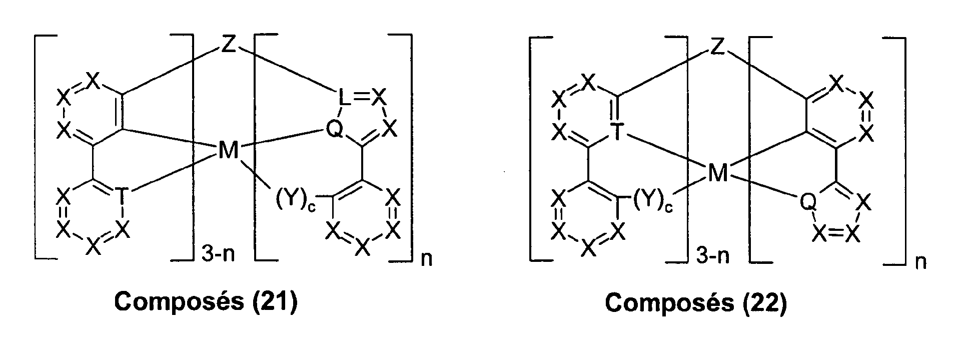 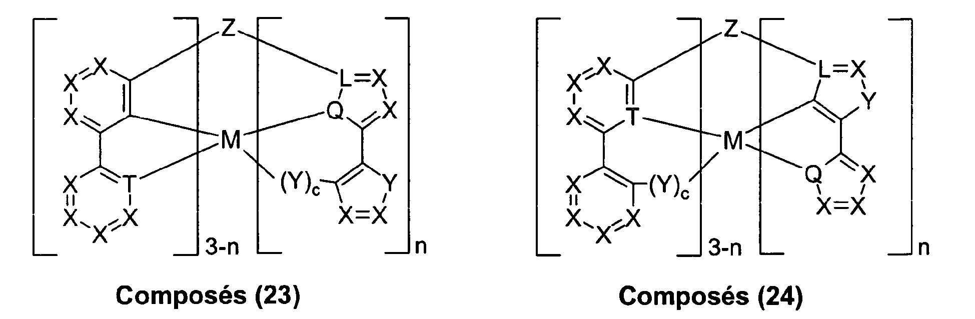 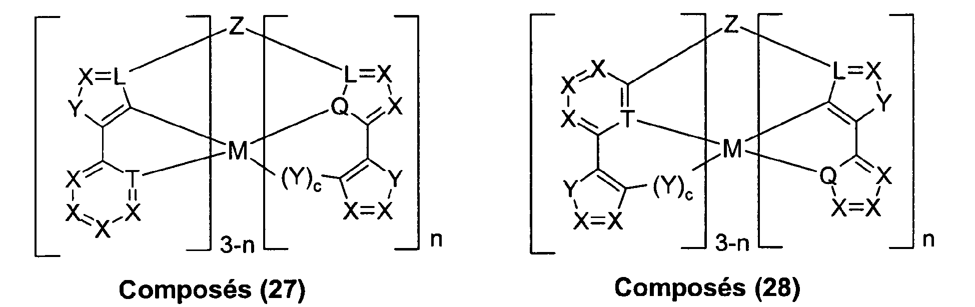 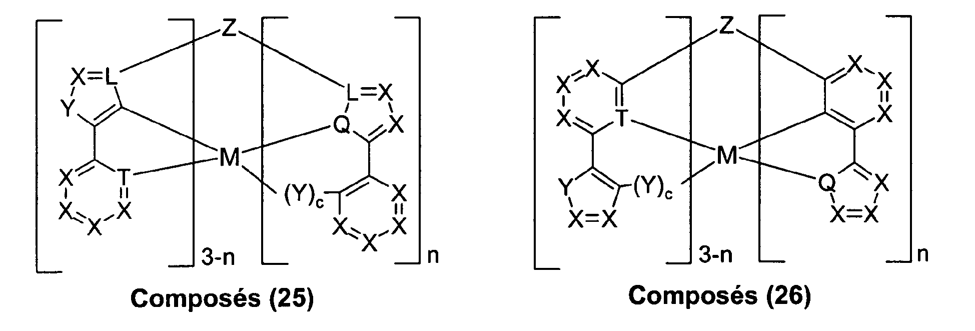 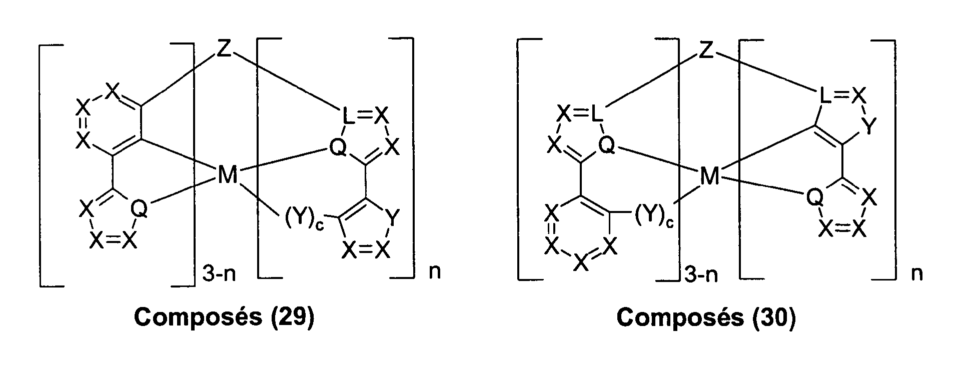 dans lesquels les symboles et indices M, L, Q, T, X, Y, Z, R, R¹, c et n présentent les significations telles que selon les revendications 7 et 8.

10. Complexes métalliques selon une ou plusieurs des revendications 1 à 6, choisis parmi les composés (31) à (41) 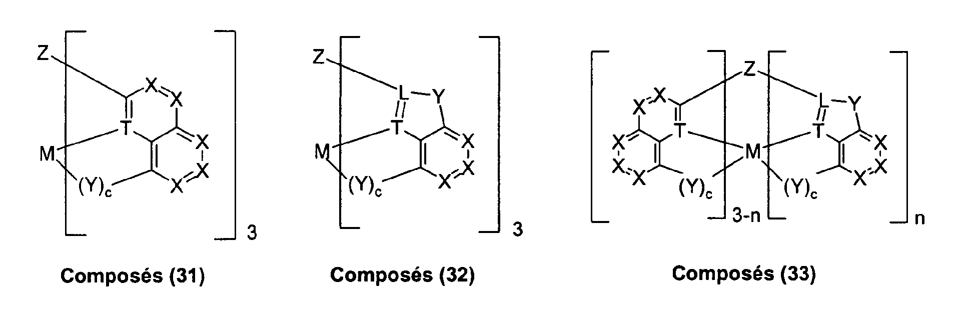 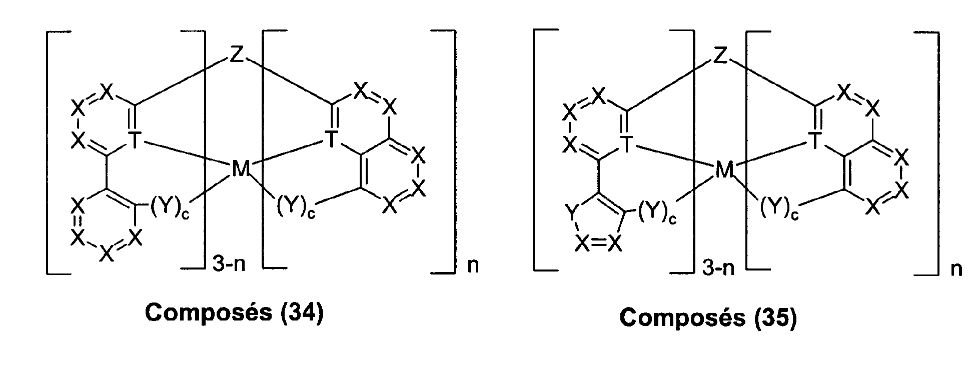 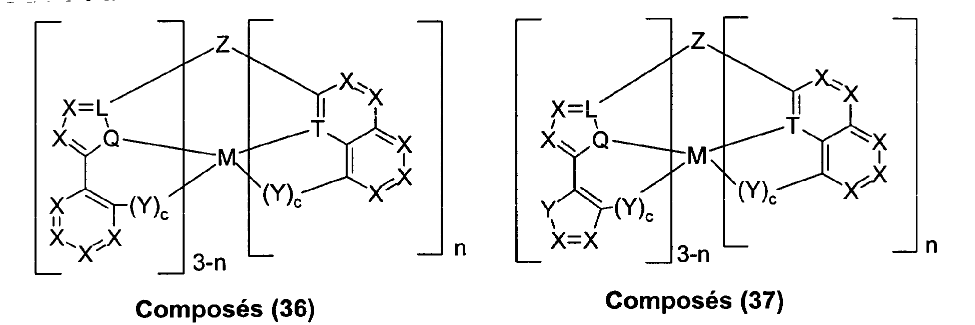 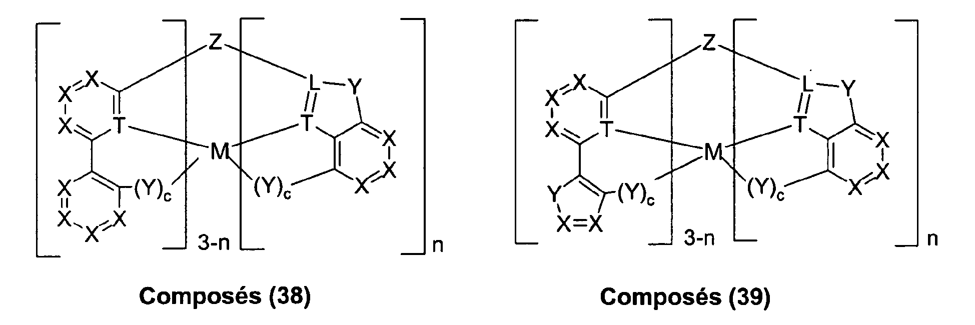 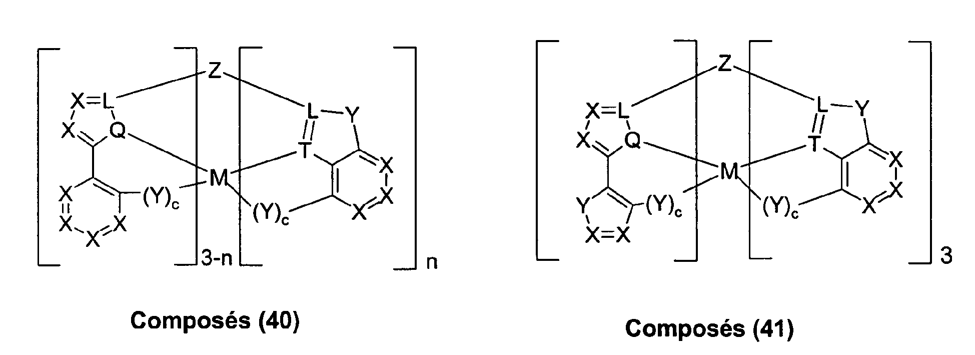 dans lesquels les symboles et indices M, L, Q, T, X, Y, Z, R, R¹, c et n présentent les significations telles que selon les revendications 7 et 8.

11. Composés (42) à (82) 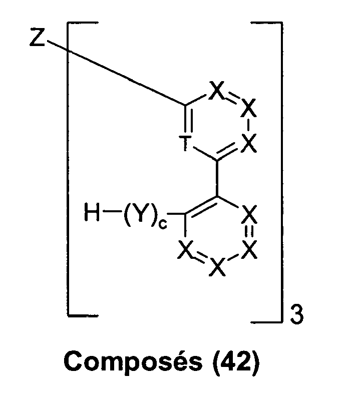 dans lesquels les symboles et indices T, Y, Z, R, R¹, c et n présentent les significations telles que selon les revendications 7 et 8,
X est, de manière identique ou différente lors de chaque occurrence, CR ou P ; 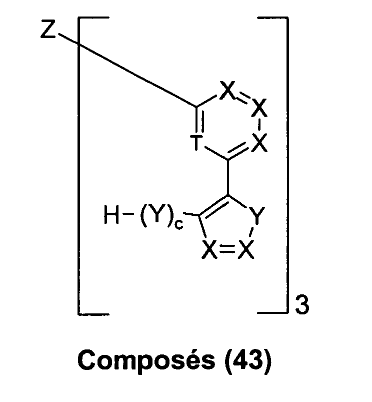 dans lesquels les symboles et indices T, Y, Z, R, R¹, c et n présentent les significations telles que selon les revendications 7 et 8, 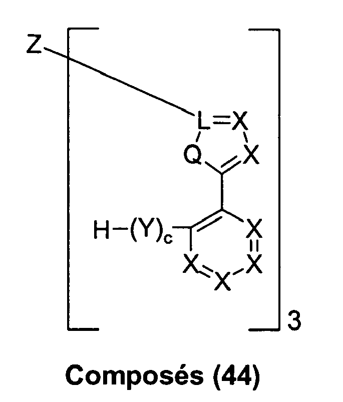 dans lesquels les symboles et indices Q, T, Y, Z, R, R¹, c et n présentent les significations telles que selon les revendications 7 et 8,
L est, de manière identique ou différente lors de chaque occurrence, C ou P ; 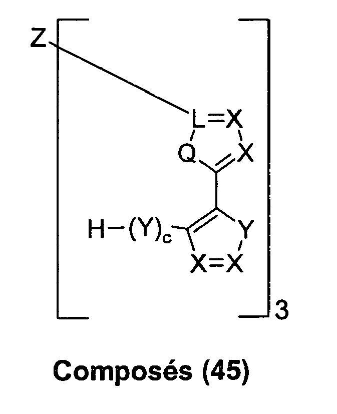 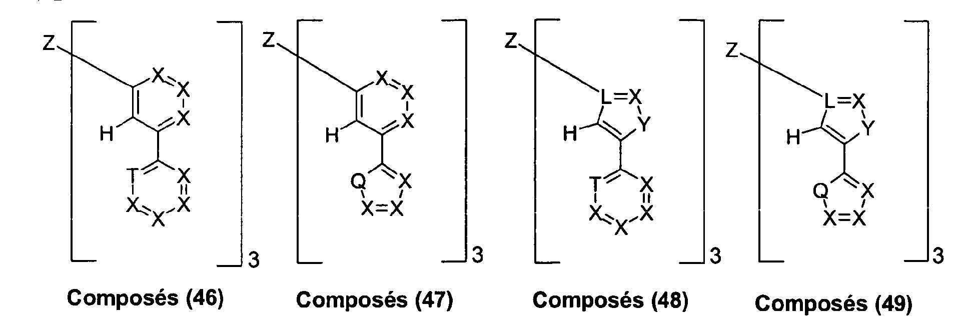 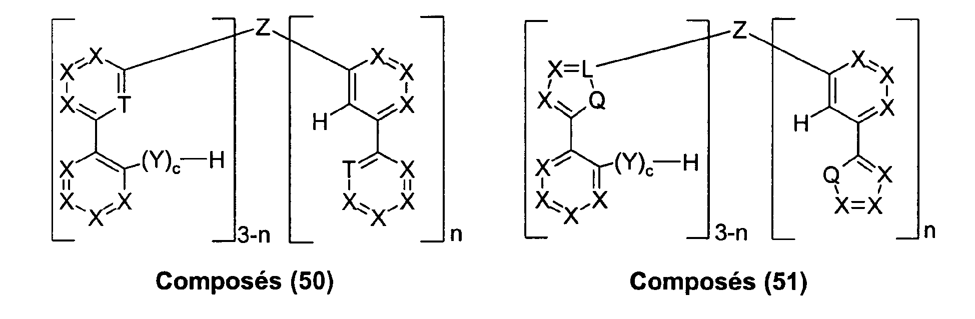 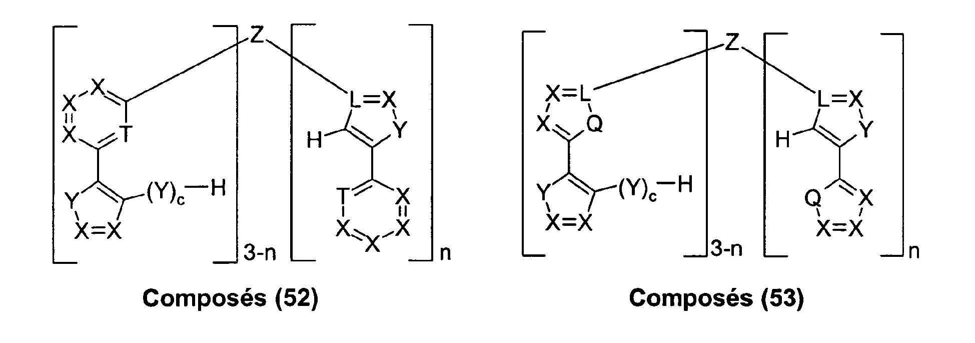 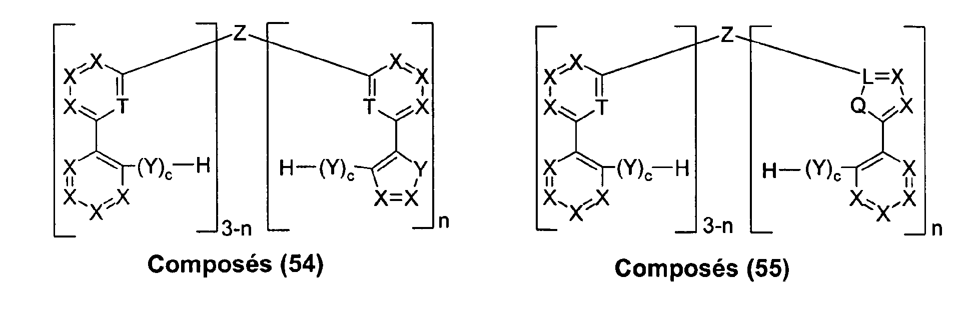 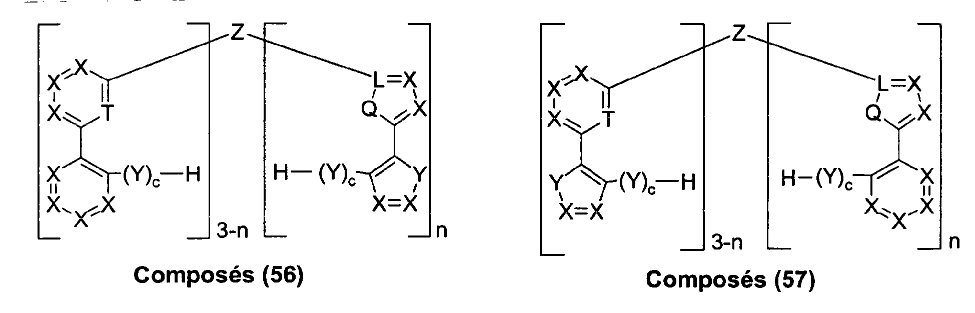 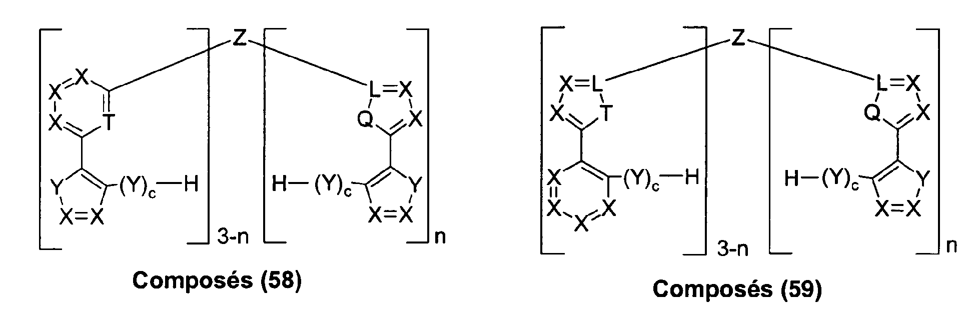 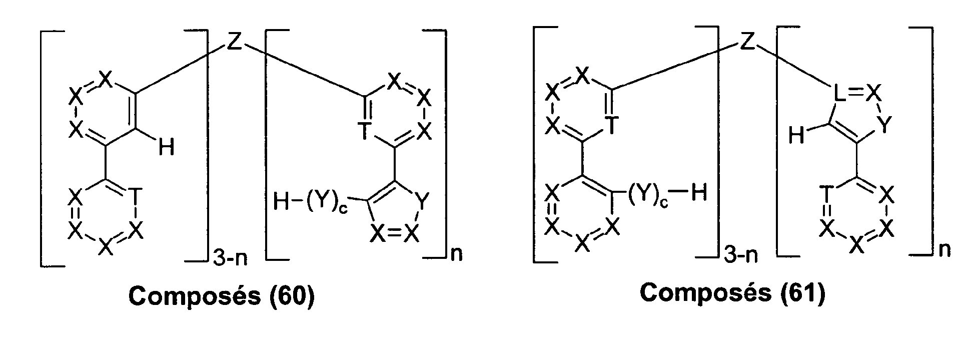 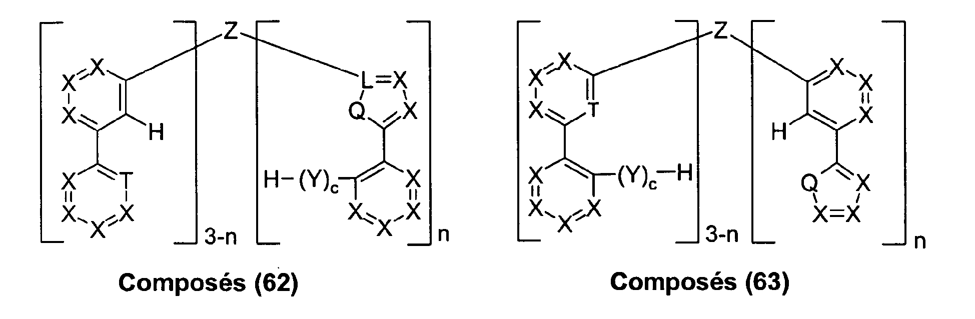 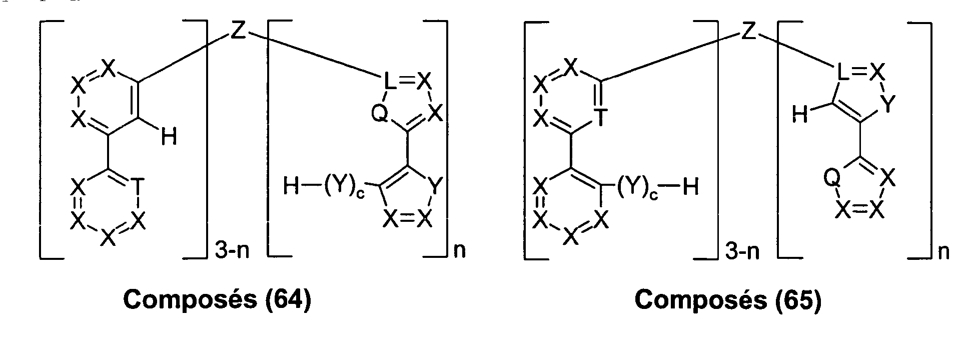 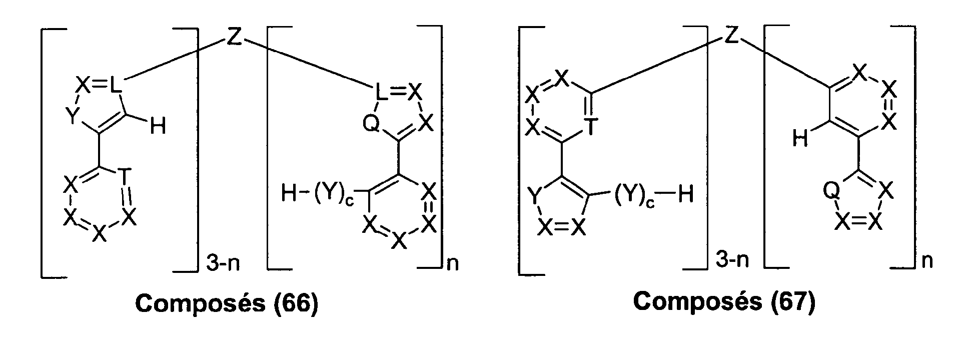 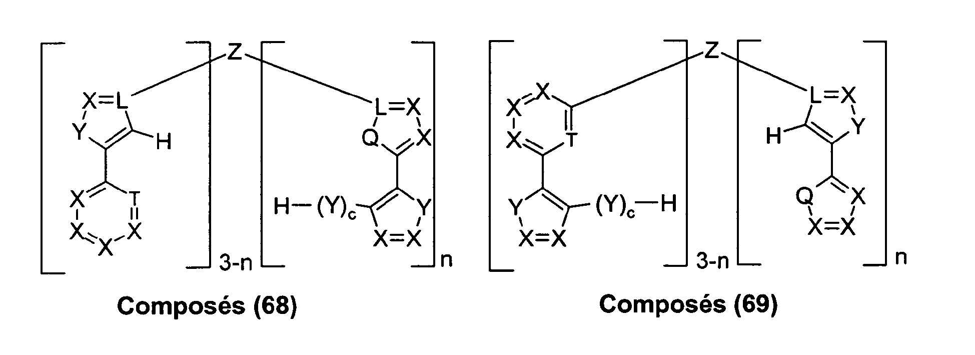 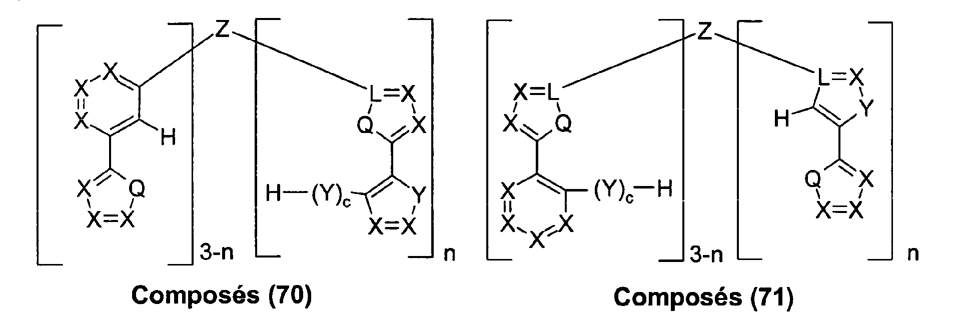 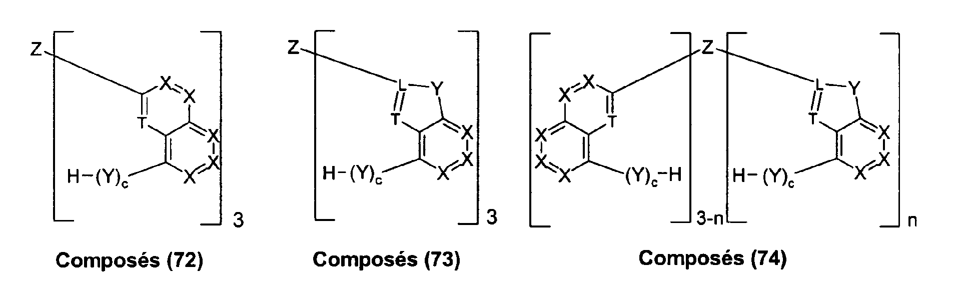 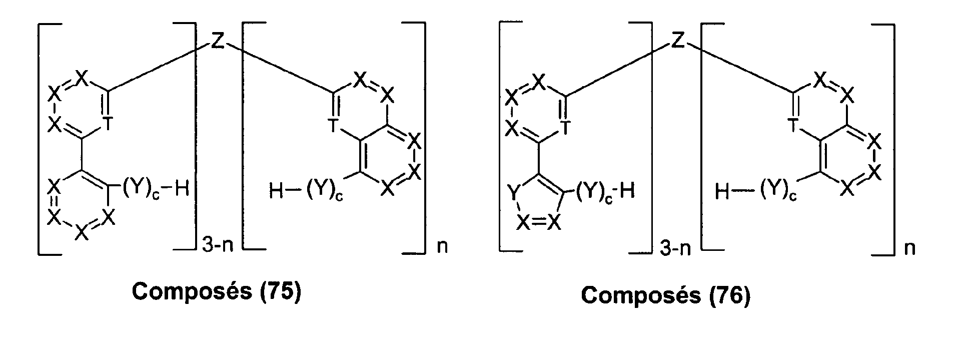 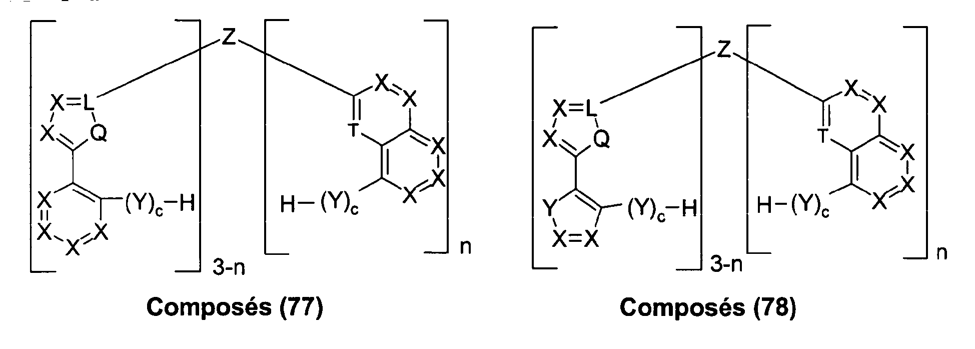 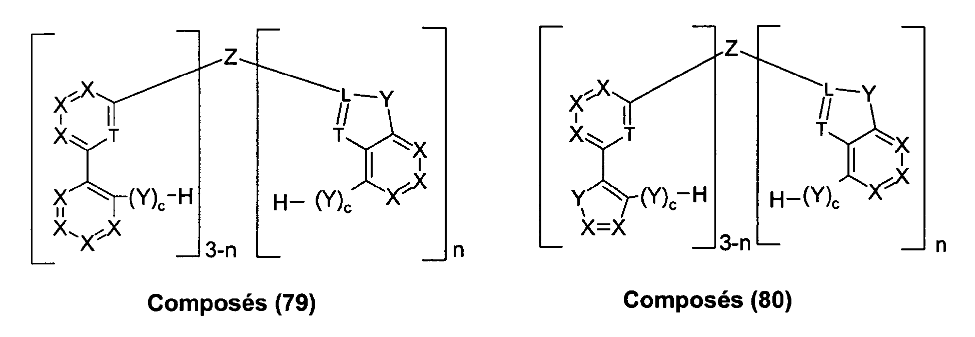 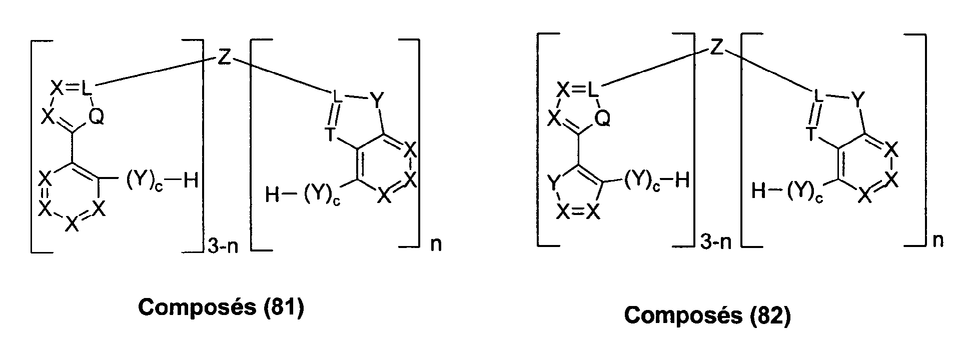
dans lesquels les symboles et indices L, Q, T, X, Y, Z, R, R¹, c et n présentent les significations selon les revendications 7 et 8.

12. Composés selon une ou plusieurs des revendications 7 à 10, **caractérisés en ce que** le symbole M = Al, Ga, In, Sc, Y, La, Ru, Os, Rh, Ir ou Au.

13. Composés selon une ou plusieurs des revendications 7 à 11, **caractérisés en ce que** le symbole L = C pour les composés (44) selon la revendication 11 et **en ce que** le symbole L = C ou N pour les autres composés.

14. Composés selon une ou plusieurs des revendications 7 à 11, **caractérisés en ce que** le symbole Q = O ou S.

15. Composés selon une ou plusieurs des revendications 7 à 11, **caractérisés en ce que** le symbole T = N.

16. Composés selon une ou plusieurs des revendications 7 à 11, **caractérisés en ce que** le symbole X = CR pour les composés (42) selon la revendication 11 et **en ce que** le symbole X = CR ou N pour les autres composés.

17. Composés selon une ou plusieurs des revendications 7 à 11, **caractérisés en ce que** le symbole Z = B, CH, CR¹, COR¹, CF, CCI, CBr, SiR, N, P, PO, RC(CR₂)₃, RC(CR₂CR₂)₃, cis,cis-1,3,5-cyclohexyl, RSi(CR₂)₃, RSi(CR₂CR₂)₃, N(CR₂)₃, N(C=O)₃, N(CR₂CR₂)₃.

18. Composés selon une ou plusieurs des revendications 7 à 11, **caractérisés en ce que** le symbole Y = O ou S.

19. Composés selon une ou plusieurs des revendications 7 à 11, **caractérisés en ce que** le symbole R = H, F, Cl, Br, I, CN, un groupe alkyle ou alkoxy en chaîne droite ou ramifié ou cyclique comportant de 1 à 6 atomes de C ou un groupe aryle ou hétéroaryle comportant de 3 à 8 atomes de C, qui peuvent être substitués par un ou plusieurs radicaux non aromatiques R, où une pluralité de substituants R, à la fois sur le même cycle et également sur les deux cycles différents, peuvent à leur tour former ensemble un autre système de cycle monocyclique ou polycyclique, aliphatique ou aromatique.

20. Composés selon une ou plusieurs des revendications 7 à 11, **caractérisés en ce que** le système de cycle polycyclique formé en option au moyen du radical ou des radicaux R correspond à benzène, 1- ou 2-naphthalène, 1-, 2- ou 9-anthracène, 2-, 3- ou 4-pyridine, 2-, 4- ou 5-pyrimidine, 2-pyrazine, 3- ou 4-pyridazine, 2-, 3-, 4-, 5-, 6-, 7- ou 8-quinoline, 2- ou 3-pyrrole, 3-, 4- ou 5-pyrazole, 2-, 4- ou 5-imidazole, 2-ou 3-thiophène, 2- ou 3-sélénophène, 2- ou 3-furane, 2-(1,3,4-oxadiazole), indole ou carbazole.

21. Procédé pour la préparation des composés selon les revendications 7 à 10, par réaction des composés (42) à (82) selon la revendication 1 avec des alkoxydes métalliques de la formule (83), avec des cétocétonates métalliques de la formule (84) ou avec des halogénures métalliques de la formule (85) 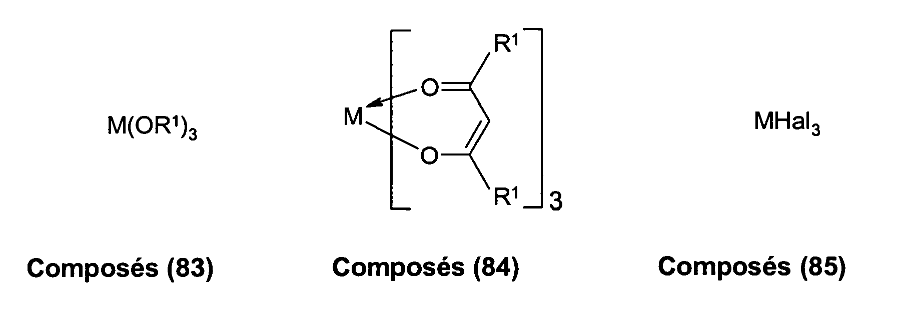 dans lesquels le symbole R¹ présente la signification indiquée selon la revendication 7, et Hal = F, Cl, Br, l.

22. Composés selon une ou plusieurs des revendications 1 à 10 et 12 à 20, **caractérisés en ce que** leur pureté (déterminée au moyen de ¹H-RMN et/ou HPLC) est supérieure à 99%.

23. Polymères ou dendrimères conjugués, partiellement conjugués ou non conjugués contenant un ou plusieurs composés de la structure (1) ou des formules (1) à (41) selon une ou plusieurs des revendications 1 à 10 et 12 à 20.

24. Polymères ou dendrimères conjugués, partiellement conjugués ou non conjugués selon la revendication 23, dans lesquels un ou plusieurs des R définis selon la revendication 7 représente(nt) une liaison sur le polymère ou dendrimère.

25. Polymères selon la revendication 23 et/ou 24, **caractérisés en ce que** le polymère est choisi parmi le groupe des polyfluorènes, des polyspirobifluorènes, des poly-para-phénylènes, des polycarbazoles, des polyvinylcarbazoles, des polythiophènes ou également à partir de copolymères qui contiennent une pluralité des unités mentionnées ici.

26. Polymères selon une ou plusieurs des revendications 23 à 25, **caractérisés en ce que** le polymère est soluble dans des solvants organiques.

27. Utilisation d'un composé selon une ou plusieurs des revendications 1 à 10 et 12 à 20 ou d'un polymère ou d'un dendrimère selon une ou plusieurs des revendications 23 à 26 dans des composants électroniques.

28. Composant électronique comprenant au moins un composé selon une ou plusieurs des revendications 1 à 10 et 12 à 20 ou un polymère ou un dendrimère selon une ou plusieurs des revendications 23 à 26.

29. Composant électronique selon la revendication 28, **caractérisé en ce qu'**il s'agit d'une diode émettrice de lumière organique (DELO), d'un circuit intégré organique (O-IC), d'un transistor à effet de champ organique (OFET), d'un transistor en film mince organique (OTFT), d'une cellule solaire organique (O-SC) ou d'une diode laser organique (O-laser).
